# EUROPEAN PATENT SPECIFICATION

(11) **EP 2 451 941 B1**
(45) Date of publication and mention of the grant of the patent: **13.06.2018**
(21) Application number: 10797829.8
(22) Date of filing: 08.07.2010
(51) Int. Cl.: C12N 5/071

(54) **CARDIAC TISSUE-DERIVED CELLS**
AUS HERZGEWEBE ABGELEITETE ZELLEN
CELLULES DÉRIVÉES DE TISSUS CARDIAQUES

(30) Priority: 09.07.2009 US 224446 P
(43) Date of publication of application: 16.05.2012
(73) Proprietor: Janssen Biotech, Inc., Horsham, PA 19044 (US)
(72) Inventor: WANG, Xiaozhen, Phoenixville PA 19460 (US); HARRIS, Ian, R., Berwyn PA 19312 (US); KENNEDY, Jeffrey, S., Aldan PA 19018 (US); YANG, Jing, Ambler PA 19002 (US); MUNGA, Susan, Norristown PA 19403 (US)
(74) Representative: Wise, Daniel Joseph
(86) International application number: PCT/US2010/041327
(87) International publication number: WO 2011/005930

(56) References cited:
- US-A1- 2001 034 439
- US-A1- 2002 072 117
- US-A1- 2002 137 204
- US-A1- 2003 148 514
- US-A1- 2004 014 206
- US-A1- 2005 255 588
- US-A1- 2005 281 788
- US-A1- 2005 283 844
- US-A1- 2006 093 586
- US-A1- 2007 093 748
- US-A1- 2007 116 684
- US-A1- 2008 070 303
- US-A1- 2008 233 090
- US-B1- 6 617 110
- R. R. SMITH ET AL: "Regenerative Potential of Cardiosphere-Derived Cells Expanded From Percutaneous Endomyocardial Biopsy Specimens", CIRCULATION, vol. 115, no. 7, 20 February 2007 (2007-02-20), pages 896-908, XP055061724, ISSN: 0009-7322, DOI: 10.1161/CIRCULATIONAHA.106.655209
- VAN TUYN J ET AL: "Epicardial cells of human adults can undergo an epithelial-to-mensenchymal transition and obtain characterictics of smooth muscle cells in vitro", STEM CELLS, ALPHAMED PRESS, DAYTON, OH, US, vol. 25, no. 2, 1 February 2007 (2007-02-01), pages 271-278, XP003024159, ISSN: 1066-5099, DOI: 10.1634/STEMCELLS.2006-0366
- CHRISTOPHER T. COTTAGE ET AL: "Increased Mitotic Rate Coincident with Transient Telomere Lengthening Resulting from Pim-1 Overexpression in Cardiac Progenitor Cells", STEM CELLS, vol. 30, no. 11, 22 October 2012 (2012-10-22), pages 2512-2522, XP55061653, ISSN: 1066-5099, DOI: 10.1002/stem.1211
- S ZIMMERMANN ET AL: "Lack of telomerase activity in human mesenchymal stem cells", LEUKEMIA, vol. 17, no. 6, 1 June 2003 (2003-06-01), pages 1146-1149, XP55061518, ISSN: 0887-6924, DOI: 10.1038/sj.leu.2402962
- HOOGDUIJN M J ET AL: "Donor-derived mesenchymal stem cells remain present and functional in the transplanted human heart.", AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS JAN 2009, vol. 9, no. 1, January 2009 (2009-01), pages 222-230, XP002696707, ISSN: 1600-6143
- E HIYAMA ET AL: "Telomere and telomerase in stem cells", BRITISH JOURNAL OF CANCER, vol. 96, no. 7, 13 March 2007 (2007-03-13) , pages 1020-1024, XP055061521, ISSN: 0007-0920, DOI: 10.1038/sj.bjc.6603671
- M. LUTZ ET AL: "Local injection of stem cell factor (SCF) improves myocardial homing of systemically delivered c-kit + bone marrow-derived stem cells", CARDIOVASCULAR RESEARCH, vol. 77, no. 1, 19 September 2007 (2007-09-19), pages 143-150, XP055062358, ISSN: 0008-6363, DOI: 10.1093/cvr/cvm027
- A. LERI: "Cardiac Stem Cells and Mechanisms of Myocardial Regeneration", PHYSIOLOGICAL REVIEWS, vol. 85, no. 4, 1 October 2005 (2005-10-01), pages 1373-1416, XP55061508, ISSN: 0031-9333, DOI: 10.1152/physrev.00013.2005
- NURZYNSKA DARIA ET AL: "Cardiac primitive cells become committed to a cardiac fate in adult human heart with chronic ischemic disease but fail to acquire mature phenotype: genetic and phenotypic study", BASIC RESEARCH IN CARDIOLOGY, vol. 108, no. 1, January 2013 (2013-01), XP002696708,
- MELO DE AGUIAR A., KULIGOWSKI C., BRENNER AFFONSO DA COSTA M., ET AL.: "Alkaline phosphatase-positive cells isolated from human hearts have mesenchymal stem cell characteristics", STEM CELL DISCOVERY, [Online] vol. 1, no. 3, 2011, pages 71-80, XP002696709, Retrieved from the Internet: URL:www.scirp.org/journal/PaperDownload.as px?FileName=SCD20110300003_65588279.pdf&pa perID=7968> [retrieved on 2013-05-06]
- DI MEGLIO F ET AL: "Epithelial-mesenchymal transition of epicardial mesothelium is a source of cardiac CD117-positive stem cells in adult human heart", JOURNAL OF MOLECULAR AND CELLULAR CARDIOLOGY, ACADEMIC PRESS, GB, vol. 49, no. 5, 4 June 2010 (2010-06-04), pages 719-727, XP027375296, ISSN: 0022-2828 [retrieved on 2010-06-04]
- URBANEK ET AL.: 'Myocardial regeneration by activation of multipotent cardiac stem cells in ischemic heart failure.' PROCEEDINGS OF THE NATIONAL ACADEMY OF SCIENCES, [Online] vol. 102, no. 24, 14 June 2005, pages 8692 - 8697, XP002520992 Retrieved from the Internet: <URL:http://www.pnas.org> [retrieved on 2010-09-02]
- OH ET AL.: 'Cardiac Muscle Plasticity in Adust and Embryo by Heart-Derived Progenitor Cells.' ANNALS OF THE NEW YORK ACADEMY OF SCIENCES, [Online] vol. 1015, no. ISS 1, 12 January 2006, pages 182 - 189, XP009039192 Retrieved from the Internet: <URL:http://www.nyas.org/publications/annal s/default.aspx> [retrieved on 2010-10-13]
- WU ET AL.: 'Cellular therapy and myocardial tissue engineering: the role of adult stem and progenitor cells.' EUROPEAN JOURNAL OF CARDIO-THORACIC SURGERY, [Online] vol. 30, 2006, pages 770 - 781, XP025227095 Retrieved from the Internet: <URL:http://ejcts.ctsnetjournals.org/cgi/co ntent/abstract/30/5/770> [retrieved on 2010-09-02]
- OH ET AL.: 'Cardiac progenitor cells from adult myocardium: Homing, differentiation, and fusion after infarction.' PROCEEDINGS OF THE NATIONAL ACADEMY OF SCIENCES, [Online] vol. 100, no. ISS 21, 14 October 2003, pages 12313 - 12318, XP002984517 Retrieved from the Internet: <URL:http://www.pnas.org> [retrieved on 2010-10-13]
- URBANEK ET AL.: 'Intense myocyte formation from cardiac stem cells in human cardiac hypertrophy.' PROCEEDINGS OF THE NATIONAL ACADEMY OF SCIENCES, [Online] vol. 100, no. ISS 18, 02 September 2003, pages 10440 - 10445, XP002520993 Retrieved from the Internet: <URL:http://www.pnas.org> [retrieved on 2010-10-13]
- OH ET AL.: 'Telomerase reverse transcriptase promotes cardiac muscle cell proliferation, hypertrophy, and survival.' PROCEEDINGS OF THE NATIONAL ACADEMY OF SCIENCES, [Online] vol. 98, no. ISS 18, 28 August 2001, pages 10308 - 10313, XP002984518 Retrieved from the Internet: <URL:http://www.pnas.org> [retrieved on 2010-10-13]
- LERI ET AL.: 'Telomerase expression and activity are coupled with myocyte proliferation and preservation of telomeric length in the failing heart. Proceedings of the National Academy of preservation of telomeric length in the failing heart.' PROCEEDINGS OF THE NATIONAL ACADEMY OF SCIENCES, [Online] vol. 98, no. ISS 15, 17 July 2001, pages 8626 - 8631, XP008150165 Retrieved from the Internet: <URL:http://www.pnas.org> [retrieved on 2010-10-13]
- SHAMBLOTT ET AL.: 'Human embryonic germ cell derivatives express a broad range of I developmentally distinct markers and proliferate extensively in vitro.' PROCEEDINGS OF THE NATIONAL ACADEMY OF SCIENCES, [Online] vol. 98, no. ISS. 1, 26 December 2000, pages 113 - 118, XP002383583 Retrieved from the Internet: <URL:http://www.pnas.org> [retrieved on 2010-10-13]
- B. G. GALVEZ ET AL: "Human cardiac mesoangioblasts isolated from hypertrophic cardiomyopathies are greatly reduced in proliferation and differentiation potency", CARDIOVASCULAR RESEARCH, vol. 83, no. 4, 20 May 2009 (2009-05-20), pages 707-716, XP055146406, ISSN: 0008-6363, DOI: 10.1093/cvr/cvp159

## Description

### FIELD OF THE INVENTION

The present invention is directed to methods and compositions for repairing damaged myocardium using human cardiac tissue-derived cells. In particular, the present invention provides methods and compositions for repairing damaged myocardium using expanded human cardiac tissue-derived cells that do not express telomerase.

### BACKGROUND

Acute myocardial infarction (AMI) is the leading cause of death in the US. AMI is caused by a sudden and sustained lack of blood flow to an area of the heart, commonly caused by narrowing of a coronary artery. Without adequate blood supply, the tissue becomes ischemic, leading to the death of myocytes and vascular structures. This area of necrotic tissue is referred to as the infarct site, and will eventually become scar tissue. The remaining cardiomyocytes are unable to reconstitute the necrotic tissue, and the heart deteriorates with time. The deterioration may be in the form of a loss of function of the heart muscle associated with remodeling of the damaged myocardium.

Some current therapies for acute myocardial infarction focus on thrombolysis or, alternatively, angioplasty, to open up the clotted vessel and restore blood supply to the infarct site. These treatments may effectively reduce infarct site size and improve cardiac systolic function, but do not reverse the loss of function of the heart muscle associated with remodeling of the damaged myocardium. Other therapies, such as, for example, angiotensin converting enzyme inhibitors (ACEI) and beta-blockers also improve global function and survival. However, the therapeutic effects from these medications may only improve survival by less than 5% in post-AMI patients.

Cell transplantation may be another potential therapy for acute myocardial infarction. For example, Orlic et al (Nature 410: 701 - 705 (2001)) report the injection of Lin⁻ c-kit⁺ bone marrow cells into damaged myocardium. Orlic *et al* state: "Our studies indicate that locally delivered bone marrow cells can generate *de novo* myocardium, ameliorating the outcome of coronary artery disease."

In another example, Nygren et al (Nature Medicine 10: 494 - 501 (2004)) state: "We show that unfractionated bone marrow cells and a purified population of hematopoietic stem and progenitor cells efficiently engraft within the infarcted myocardium. Engraftment was transient, however, and hematopoietic in nature. In contrast, bone marrow-derived cardiomyocytes were observed outside the infarcted myocardium at a low frequency and were derived exclusively through cell fusion."

However, the mechanism by which bone marrow-derived cells treat AMI is unclear. For example, Murry et al (Nature 428: 664 - 668 (2004)) state: "[W]e used both cardiomyocyte-restricted and ubiquitously expressed reporter transgenes to track the fate of haematopoietic stem cells after 145 transplants into normal and injured adult mouse hearts. No transdifferentiation into cardiomyocytes was detectable when using these genetic techniques to follow cell fate, and stem-cell-engrafted hearts showed no overt increase in cardiomyocytes compared to sham-engrafted hearts. These results indicate that haematopoietic stem cells do not readily acquire a cardiac phenotype, and raise a cautionary note for clinical studies of infarct repair."

In another example, Werner et al (Nature Clinical Practice Cardiovascular Medicine 5: 78-79 (2008)) state: "There are many questions, however, still to be answered with regard to the most effective progenitor cell subpopulation, the best technique for progenitor cell augmentation, the underlying mechanisms of action, and the long-term safety and effectiveness of the method.

Moreover, several trials of [bone marrow cell] therapy in patients with AMI have produced negative results, possibly because of variation in the timing of [bone marrow cell] administration after AMI, differences in the methods of progenitor cell preparation used, or both."

In another example, Balsam et al (Nature 428: 668 - 673 (2004)) state: "Our data suggest that even in the microenvironment of the injured heart, c-kit-enriched BM cells, Lin⁻ c-kit⁺ BM cells and c-kit⁺ Thyl.1^{lo} Lin- Sca-1⁺ long-term reconstituting haematopoietic stem cells adopt only traditional haematopoietic fates."

Another possible source of cells is embryonic stem cells. For example, Gold et al (WO2005090558) discloses methods for generating cardiomyocyte lineage cells from embryonic stem cells for use in regenerative medicine.

In another example, Gold and Hassanipour (WO2007002136) disclose methods for the differentiation of primate pluripotent stem cells into cardiomyocyte-lineage cells.

Another possible source of cells is cardiac progenitor cells. Cardiac progenitor cells have been identified in the human and rat heart. Cardiac progenitor cells are self-renewing and multipotent giving rise to all cardiac lineages.

For example, U.S. Patent Application US20040126879A1 disclose the use of cardiac stem cells that are CD31⁺, CD38⁺ and c-kit⁻ to treat damaged myocardium.

In another example, Oh et al (PNAS 100: 12313 - 12318 (2003)) disclose the existence of adult heart-derived cardiac progenitor cells, expressing Sca-1, CD31 and CD38, and lacking the expression of CD4, CD8, B220, Gr-1, Mac-1, TER119, c-kit, Flk-1, e-Cadherin, von Willebrand factor, CD45 and CD34.

In another example, U.S. Patent Application US 20080241111A1 disclose a method for preparing mammalian cardiac tissue-derived cells prepared through the steps of: (i) enzymatically treating a cardiac tissue fragment from a mammal to prepare a cell suspension; (ii) separating a group of cardiac tissue-derived cells from said cell suspension by a density gradient method; and (iii) suspension culturing the obtained group of cardiac tissue-derived cells in a culture medium containing fibroblast growth factor and epidermal growth factor, and then selecting and separating cells forming a floating sphere.

In another example, U.S. Patent Application US 20080213231A1 disclose a pluripotent stem cell group composed of pluripotent stem cells derived from a human or mouse skeletal muscle tissue, the pluripotent stem cells being c-met-negative, Pax-7-negative, Myf-5-negative, MyoD-negative, Myogenin-negative, M-cadherin-negative, CD105-positive, CD90-positive, c-kit-negative and CD45-negative, the pluripotent stem cells being CD34-negative in the case of the human-derived stem cells and being CD34-positive in the case of the mouse-derived stem cells, and the pluripotent stem cell group being obtained by proliferation of a single cell.

In another example, Laugwitz et al (Nature 433: 647 - 653 (2005) discloses isll-1⁺ cardiac progenitor cells in postnatal rat, mouse and human myocardium.

In another example, Messina at al (Circulation Research 95: 911 - 921, (2004)) disclose the "isolation of undifferentiated cells that grow as self-adherent clusters (that we have termed "cardiospheres") from subcultures of postnatal atrial or ventricular human biopsy specimens and from murine hearts. These cells are clonogenic, express stem and endothelial progenitor cell antigens/markers, and appear to have the properties of adult cardiac stem cells." Messina *at al* state: "[N]ewly developing human and mouse cardiospheres revealed expression of endothelial (KDR (human)/flk-1 [mouse], CD-31) and stem cell (CD-34, c-kit, sca-1) markers."

In another example, Smith et al (Circulation 115(7): 896 - 908 (2007) state: "Percutaneous endomyocardial biopsy specimens grown in primary culture developed multicellular clusters known as cardiospheres, which were plated to yield cardiosphere-derived cells (CDCs)."

In another example, U.S. Patent Application US20070020758 discloses a method for the isolation, expansion and preservation of cardiac stem cells from human or animal tissue biopsy samples to be employed in cell transplantation and functional repair of the myocardium or other organs.

In another example, Beltrami et al (Cell 114(6): 763 - 776 (2003)) disclose "the existence of Lin- c-kitPOS cells with the properties of cardiac stem cells. They are self-renewing, clonogenic, and multipotent, giving rise to myocytes, smooth muscle, and endothelial cells."

In another example, WO 2008054819 discloses cardiovascular stem cells positive for markers isll⁺/ Nkx 2.5⁺/ flkl⁺ and cardiovascular stem cells which can differentiate along endothelial, cardiac, and smooth muscle cell lineages.

In another example, WO 2008109839A1 discloses an enriched population of stem cells comprising a CXCR4 polypeptide and an FIk-I polypeptide, wherein said stem cells are capable of differentiating into cells that express Mef2C, GATA-4, Myocardin, and Nkx2.5 polypeptides.

In another example, WO 2008081457A2 discloses a method of isolating cardiac stem cells, the method comprising contacting a tissue which comprises the cardiac stem cells with a composition which comprises dispase Il under conditions sufficient to induce cell dissociation, thereby isolating the cardiac stem cells.

In another example, WO 2008058273A2 discloses a method for obtaining mammalian stem-cell-like myocyte-derived cells (MDCs) from atrial or ventricular heart tissue, comprising the steps of: isolating cells from atrial or ventricular heart tissue to form a cell suspension; and culturing the cells in a medium comprising a mitogen thereby forming a composition comprising MDCs.

In another example, WO 2008054819A2 discloses a method for isolating cardiovascular stem cells, the method comprising contacting a population of cells with agents reactive to Isletl, Nkx2.5 and flkl, and separating reactive positive cells from non-reactive cells.

In another example, U.S. Patent Application US 20070212423A1 discloses a method of isolating a c-kit⁻/c-met⁻ cardiomyocyte precursor cell of muscular origin, comprising separating cells of less than 40 µm in diameter from a suspension of muscle cells; culturing the cells in a tissue culture medium on a solid substrate; and isolating the cells in suspension in the medium; thereby isolating the c-kit⁻/c-met⁻ cardiomyocyte precursor cell of muscular origin.

In another example, U.S. Patent Application US 20050058633 an isolated mammalian c-kit-/c-met-cardiomyocyte precursor cell of muscular origin.

In another example, WO 2004019767 discloses an isolated mammalian cardiomyocyte stem cell having c-kitneg/CD31+ /CD38+ and expressing telomerase reverse transcriptase.

In another example, WO 2008083962A1 discloses [c]ardiomyocyte progenitor cells (CMPCs) which are characterized by Sca-1 or a Sca-1 like epitope and CD31 on their cell surface.

In another example, U.S. Patent Application 20080213230A1 discloses method of preparing an isolated cell population enriched in stem cells or progenitor cells, comprising: (a) culturing a tissue sample;(b) obtaining cells that migrate above adherent fibroblasts during said culturing;(c) cloning one or more cells obtained in (b) to produce one or more clonogenic populations;(d) identifying one or more clonogenic populations having a desired phenotype;(e) isolating stem cells or progenitor cells from the one or more clonogenic populations identified in step (d) by cell sorting using one or more cell surface or internal markers of stem cells or progenitor cells; and(f) culturing the isolated stem cells or progenitor cells in conditioned media in the absence of feeder cells; thereby obtaining an isolated cell population enriched in stem cells or progenitor cells.

However, one obstacle for the use of cardiac progenitor cells is the lack of an efficient method to isolate or expand the cells. Therefore, there still remains a need for the efficient isolation and expansion of cardiac progenitor cells in order for their effectiveness as a therapy for damages myocardium to be assessed.

### SUMMARY

The present invention provides methods to isolate and expand cells derived from human cardiac tissue. Cells isolated and expanded according the methods of the present invention do not express telomerase, and are useful to treat or repair damaged myocardium.

The present invention provides a purified population of human cardiac tissue-derived cells that do not express telomerase according to the claims.

The present invention provides a method to produce human cardiac tissue-derived cells that do not express telomerase, comprising the steps of:
a. Obtaining heart tissue,
b. Dissociating the heart tissue,
c. Digesting the heart tissue to release cells,
d. Removing the cardiomyocytes from the released cells, and
e. Culturing the remaining cells.

In one embodiment, the present invention provides a population of cardiac tissue-derived cells according to the claims for use in a method to treat or repair damaged myocardium in a patient comprising the steps of:
a. Obtaining a population of human cardiac tissue-derived cells that do not express telomerase, and
b. Administering the population of human cardiac tissue-derived cells to the patient in an amount sufficient to treat or repair the damaged myocardium.

In one disclosure, the human cardiac tissue-derived cells used to treat the patient have been cryopreserved.

### BRIEF DESCRIPTION OF THE FIGURES

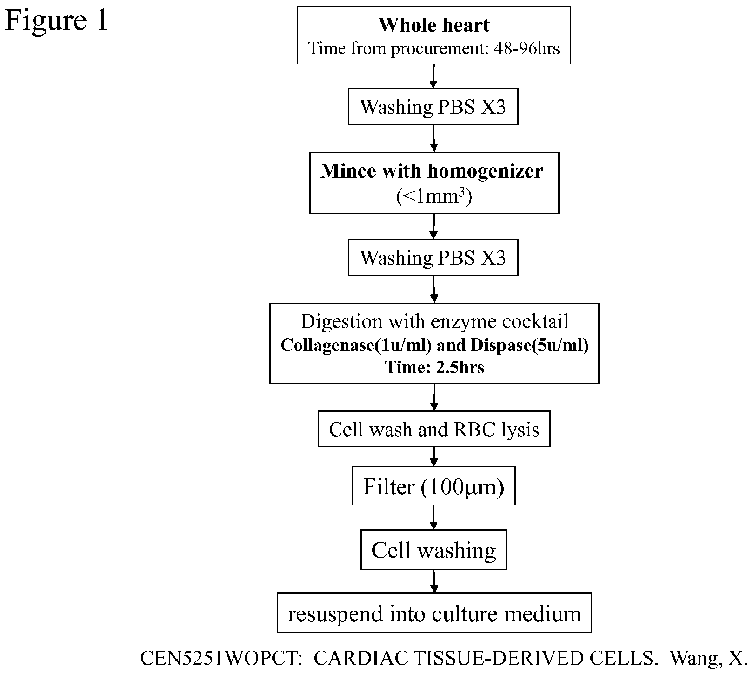

**Figure** 1 outlines the procedure by which the cells of the present invention are isolated. The details of the process to obtain the cell populations are described in **Example 1.**

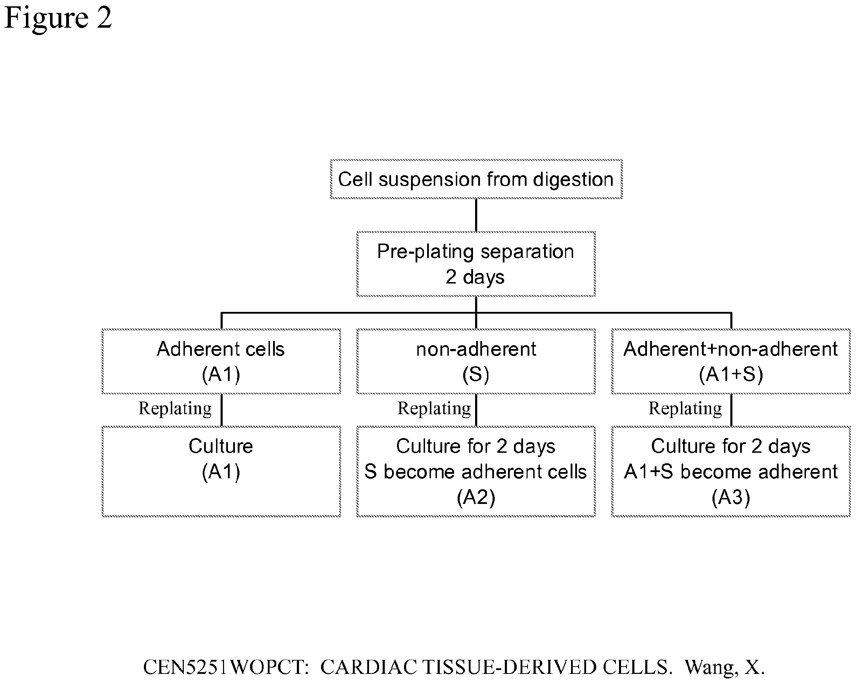

**Figure** 2 outlines the isolation of the human cardiac tissue-derived cells of the present invention. The details of the process to obtain the cell initial populations are described in **Example 1.**

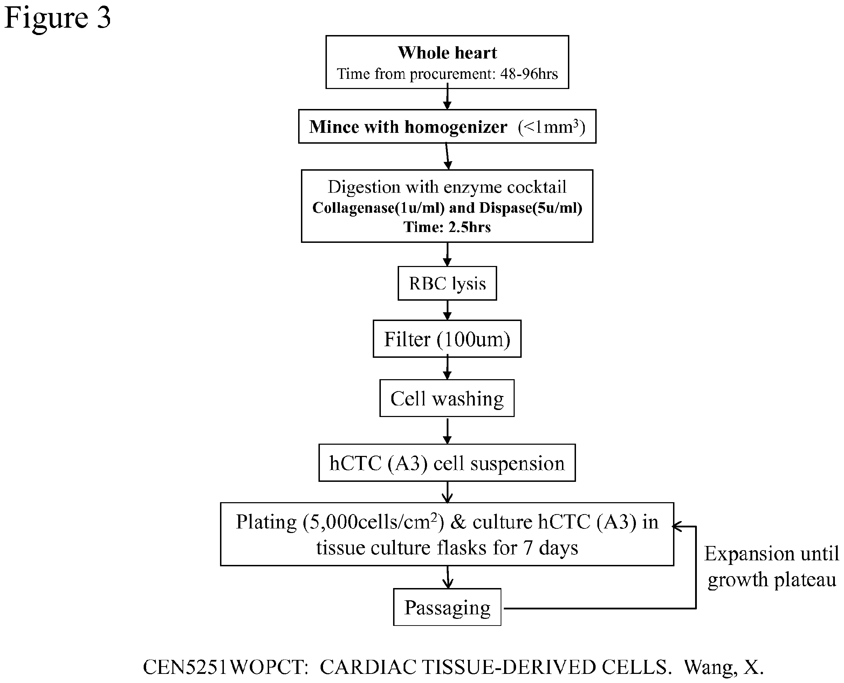

**Figure** 3 outlines an alternate method to isolate the human cardiac tissue-derived cells of the present invention.

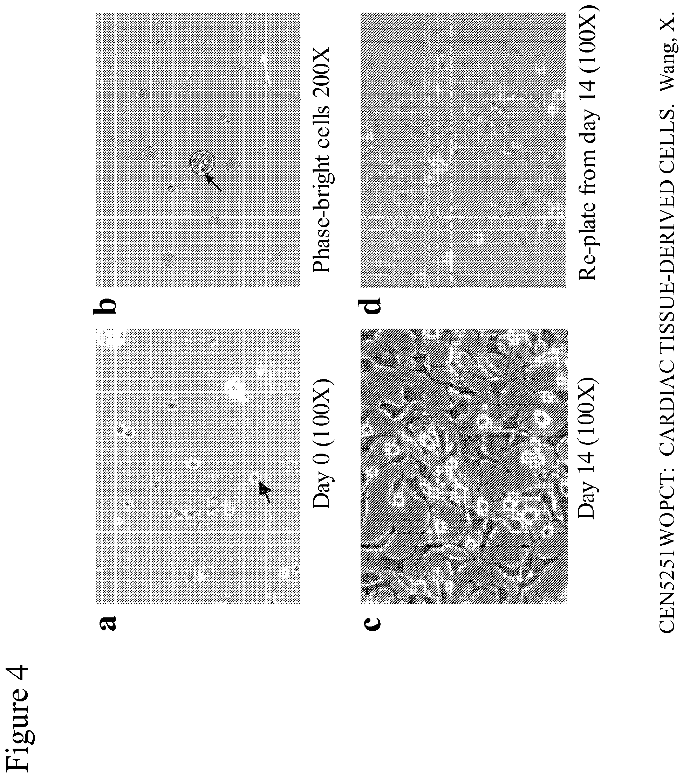

**Figure 4** shows the morphology of the human cardiac tissue-derived cells (hCTC's) of the present invention. All images shown are at 100X magnification, unless otherwise indicated. Panel a: is an image showing a cell suspension obtained from pre-plating the cells obtained from the initial digestion. The black arrow shows phase-bright non-adherent S cells; Panel b: The black arrow shows a phase-bright S cell cluster. The white arrow shows adherent cells obtained from the initial plating (the image shown is at 200X magnification); Panel c shows A2 cells, derived from replating S cell cultures; Panel d shows replated phase-bright S cells in an A2 cell culture.

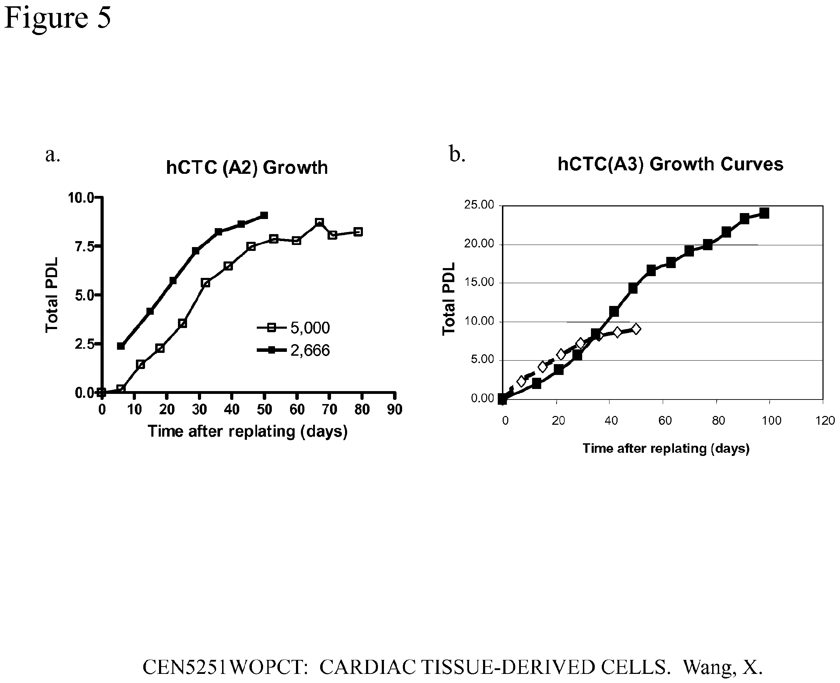

**Figure 5** shows the effect of seeding density on hCTC growth potential. The x-axis shows the days in culture after plating a mixture of hCTC (A1) and hCTC (S) cells from frozen vials. The y-axis shows the accumulative total population doublings of the hCTC (A3) cells.

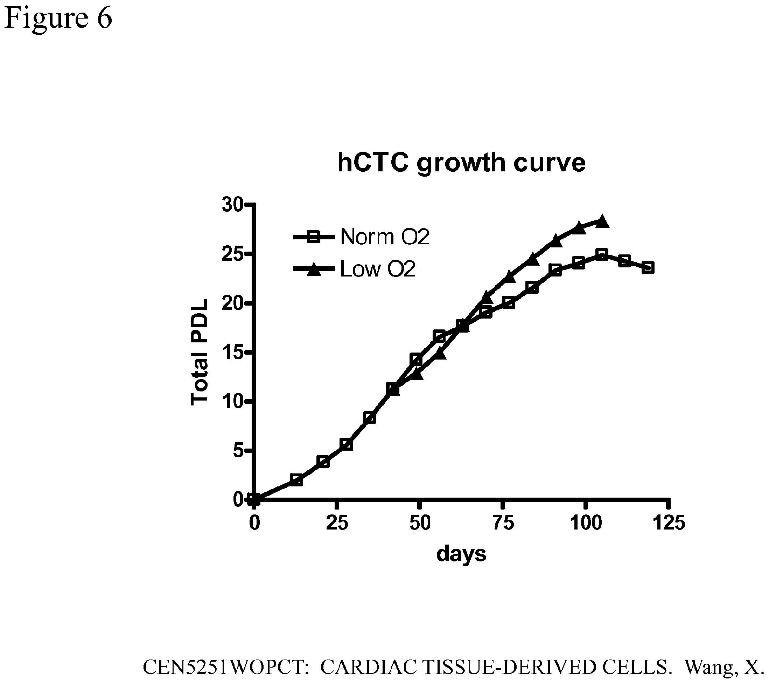

**Figure 6** shows the effect of reduced oxygen levels on hCTC (A3) cell growth potential.

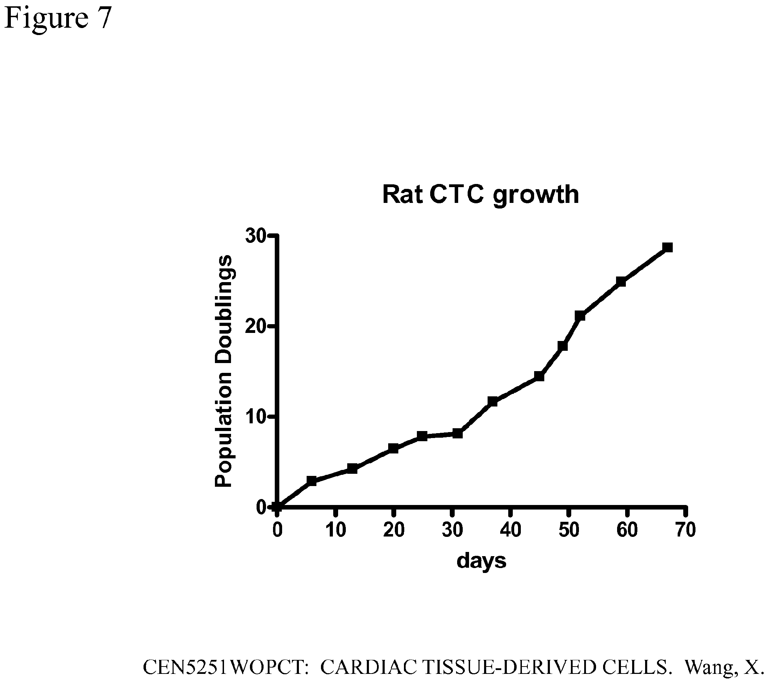

**Figure 7** shows the growth potential of rat cardiac tissue-derived rCTCA2 cells (rCTC (A2)). The x-axis shows the days in culture after replating rCTC (S) cells from frozen vials. The y-axis shows the accumulative total population doublings of rCTC (A2) cells.

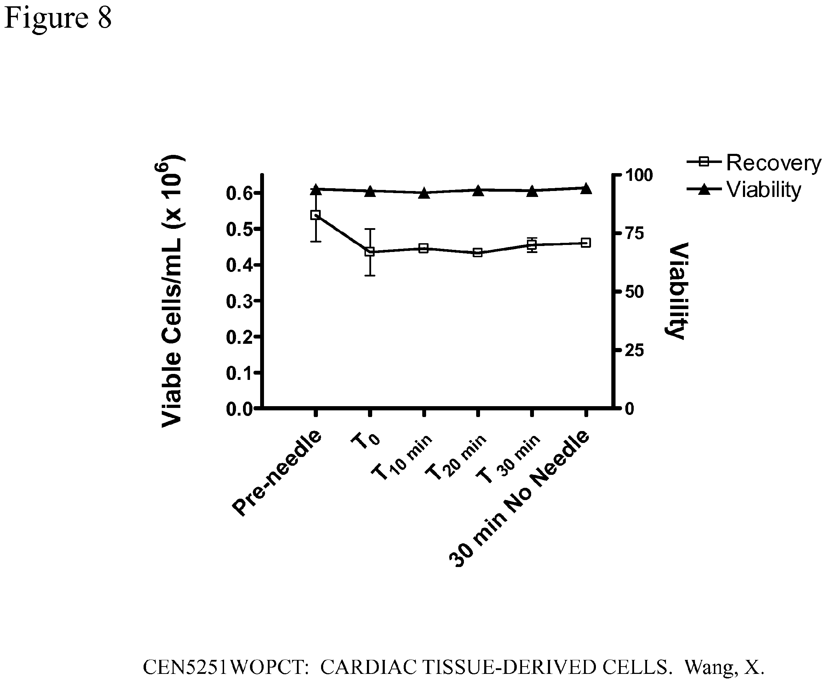

**Figure 8** shows the recovery and viability of hCTC (A3) cells, following cryopreservation and simulated delivery with a potential administration device (consisting of a 30-gauge needle). The viable cell number recovered is indicated on the left y- axis. The cell viability is indicated on the right y-axis. Filled diamonds depict cell viability; open squares depict cell recovery. Details are described in **Example 6.**

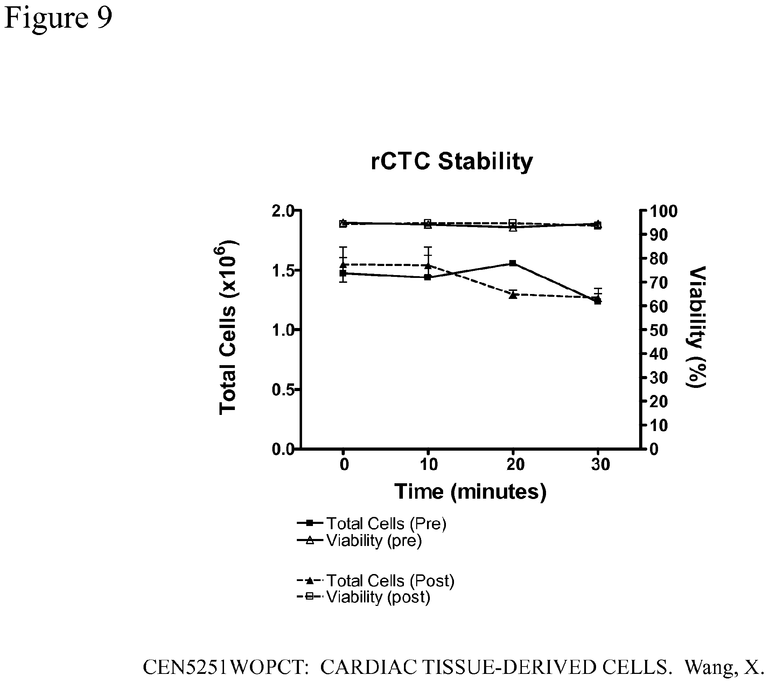

**Figure 9** shows the recovery and viability of rCTC (A2) cells following cryopreservation and simulated delivery with a potential administration device (consisting of a 30-gauge needle). The viable cell number recovered is indicated on the left y-axis. The cell viability is indicated on the right y-axis. Filled squares depict cell recovery prior to needle passage; filled triangles depict cell viability prior to needle passage; open squares depict cell viability post needle passage; open triangle depict cell recovery post needle passage. Details are described in **Example 6.**

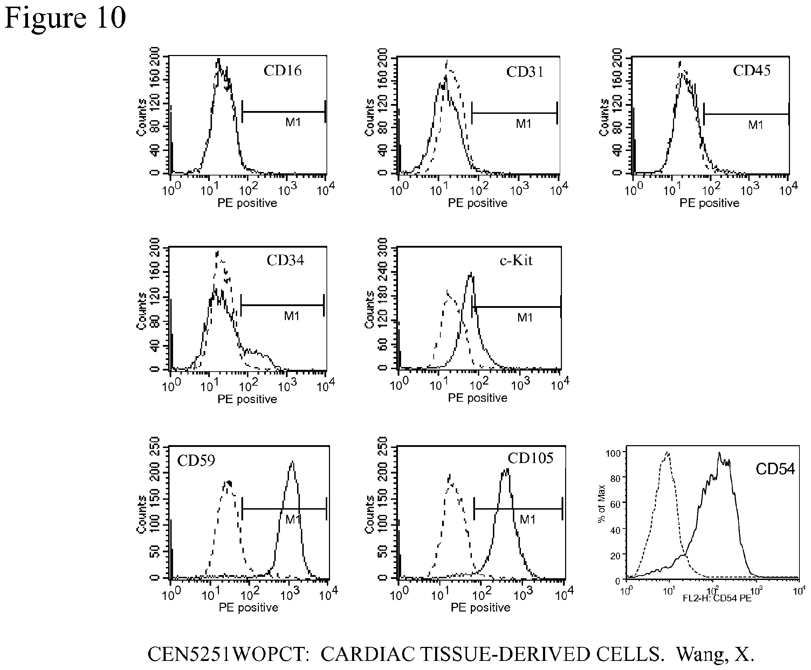

**Figure 10** shows hCTC (A3) cell surface marker expression as determined by flow cytometry. In each histogram, the dotted line is the isotype antibody control. The solid line is the antigen staining. Antigens were shown in the panels. The x-axis is the phycoerythrin (PE) intensity in logarithmic scale. The y-axis is the cell count.

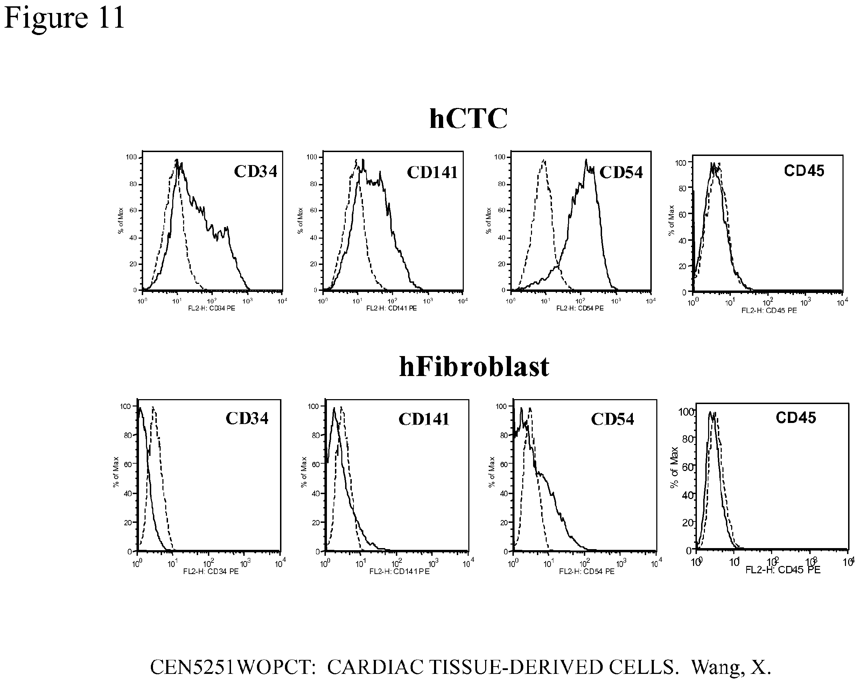

**Figure** 11 shows the comparison of cell surface marker expression between hCTC (A3) cells (upper panels) and adult human dermal fibroblasts-NHDF (catalogue number: CC-2511, Lonza, lower panels). In each histogram, the x-axis is the PE intensity in logarithmic scale. The y-axis is the cell count. The dotted line depicts antibody isotype control. The solid line is the antigen staining. The positive population was gated based on 1% positive population in isotype controls. The individual markers are indicated in each histogram.

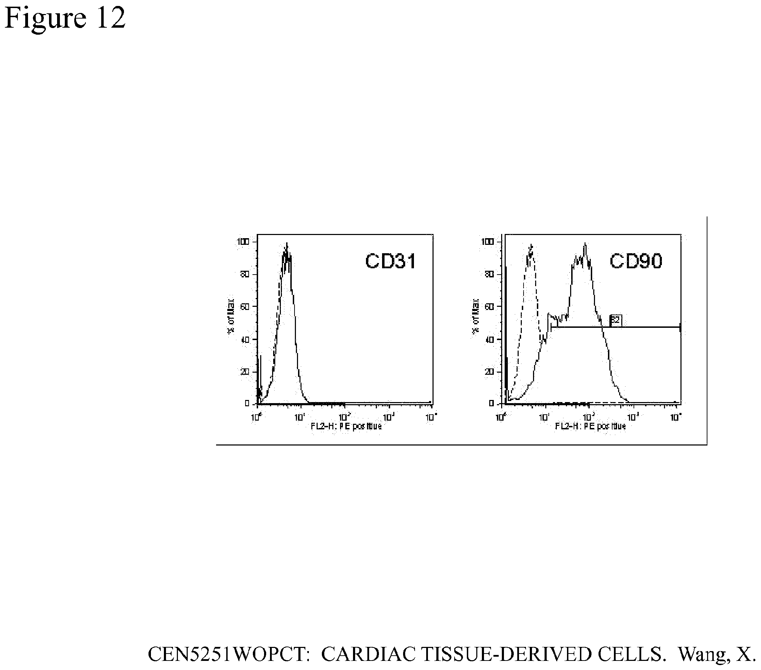

**Figure 12** shows rCTC (A2) cell surface marker expression as determined by flow cytometry. The dotted line depicts the isotype control. The solid line is the antigen staining for CD31 (left panel), and CD90 (right panel).

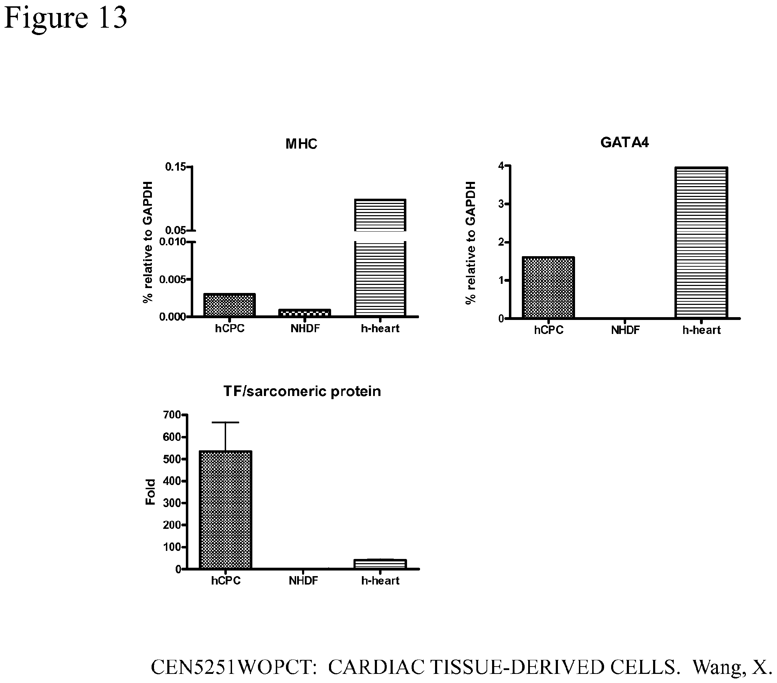

**Figure** 13 shows the gene expression of cardiac specific genes in hCTC (A3) cells as determined by quantitative real-time polymerase chain reaction (qRT-PCR). Details are described in **Example 8**. The y-axis shows the percentage of GAPDH, and is split into lower and upper scales. The lower scale ranges from 0 to 0.01%, and the upper scale ranges from 0.05% to 0.15%. The acronyms have the following meanings: MHC means myosin heavy chain; cardiac TF means transcription factor; NHDF means Neonatal human dermal fibroblast; h-heart means human heart. Data are expressed as Mean ± S.D (n=3).

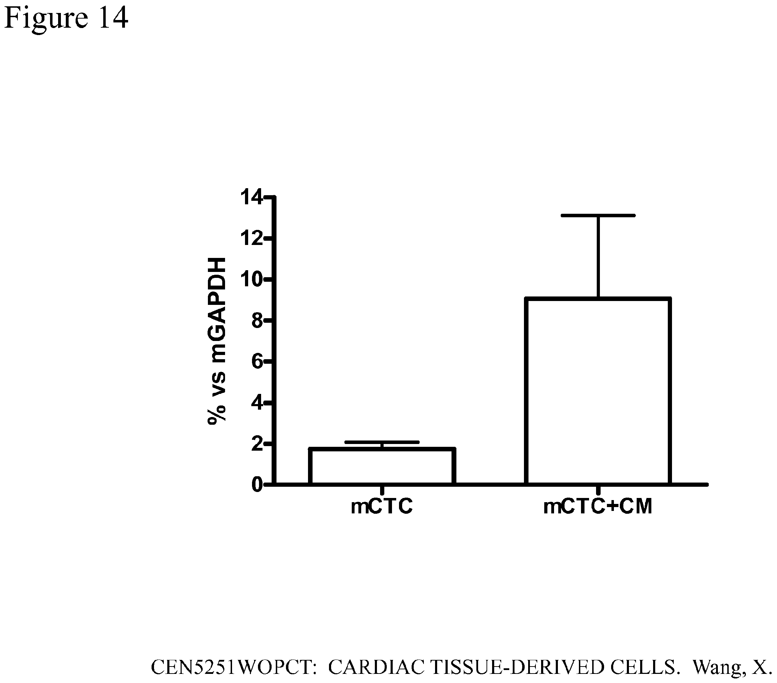

**Figure 14** shows the elevated expression of mouse specific myosin heavy chain (MHC) in a co-culture of mouse cardiac tissue-derived (A2) cells (mCTC (A2)) with rat neonatal cardiomyocytes (Catalogue # R357-6W, Cell Application, Austin, TX). The mouse MHC gene expression level was presented as the percentage of mouse GAPDH in each sample. The y-axis indicates the percentage of mouse GAPDH. The acronyms have the following meanings: mCTC means mCTC (A2) cells cultured in differentiation medium, as described in **Example 9**; CM means cardiomyocytes; mCTC+CM means mCTC co-cultured with rat cardiomyocytes CM in differentiation medium. Data are expressed as Mean ± S.D (n=3).

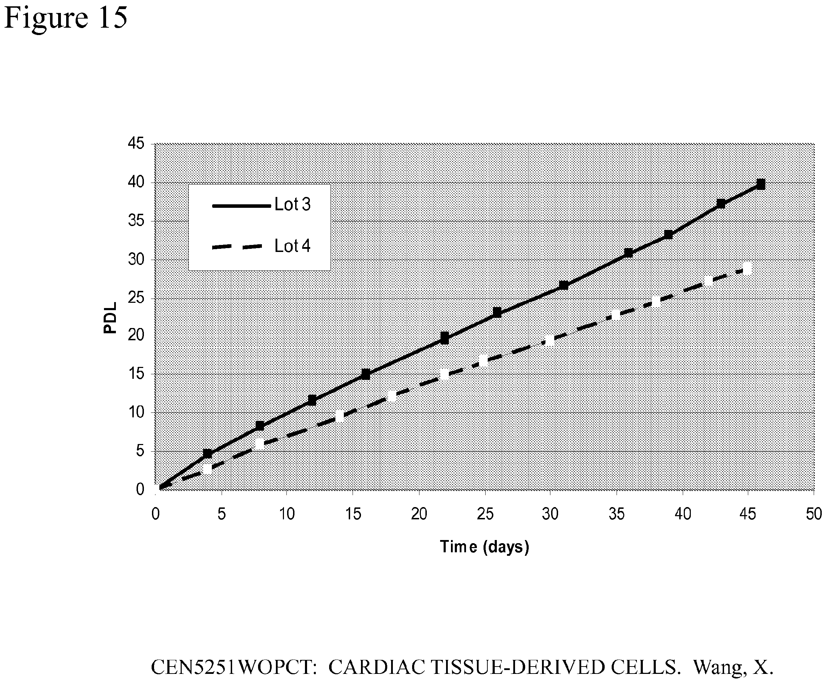

**Figure 15** shows the growth curve observed for porcine cardiac tissue-derived (A3) cells (pCTC (A3)), cultured according to the methods described in **Example 10.** The x-axis shows time in culture following plating. The y-axis shows the accumulative total population doublings.

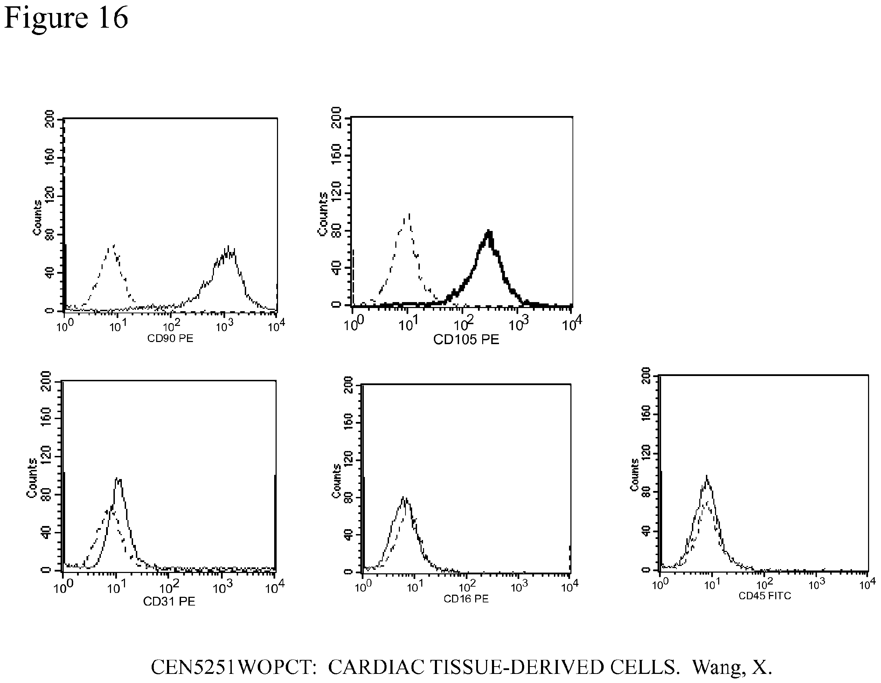

**Figure 16** shows pCTC (A3) cell surface marker expression as detected by flow cytometry. The dotted line depicts the isotype control. The solid line is the antigen staining for CD90 (upper left panel), CD105 (upper right panel), pig endothelial cell marker (lower left panel), CD16 (lower middle panel), CD45 (lower right panel).

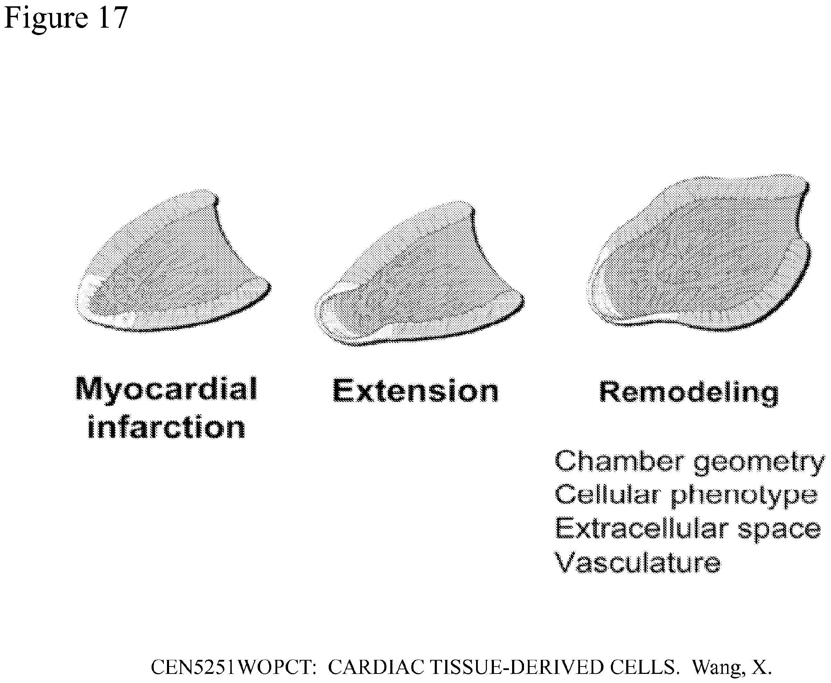

**Figure 17** shows a cartoon of the cardiac remodeling which follows acute myocardial infarction. The cartoon was reproduced from Pfeffer M. in Atlas of heart failure (Colucci W, editor, 1999).

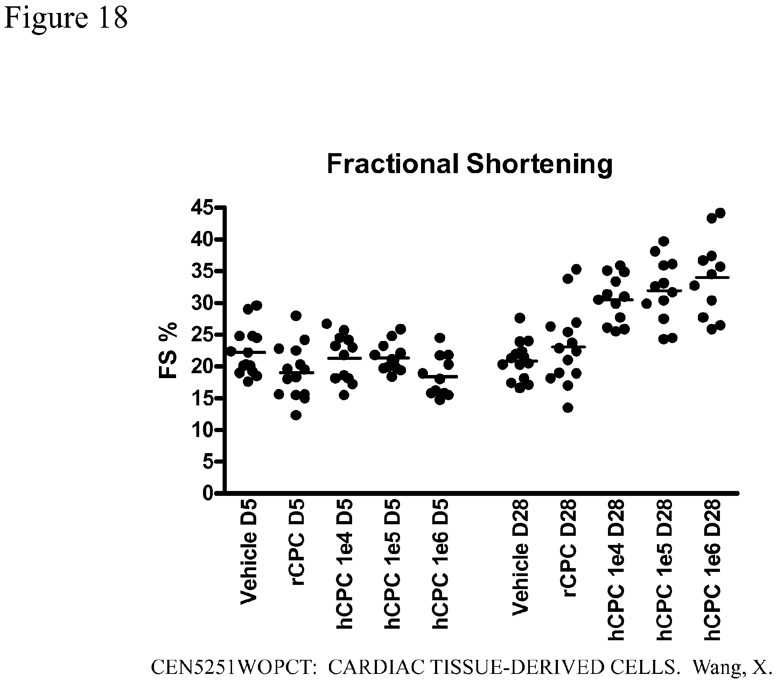

**Figure 18** shows the effect of the administration of the cardiac tissue-derived cells of the present invention on fractional shortening (FS) in animals wherein acute myocardial infarctions have been induced, as measured by echocardiography. Fractional shortening is the percent change (FS%) in systole from diastole in each cardiac cycle, and reflects the global function of the heart. Data shown is the fractional shortening recorded in an individual animal at 5 (D5) or 28 days (D28) after induction of AMI. Animals were dosed with rCTC (A2) cells, or hCTC (A3) cells at the doses indicated on the x-axis.

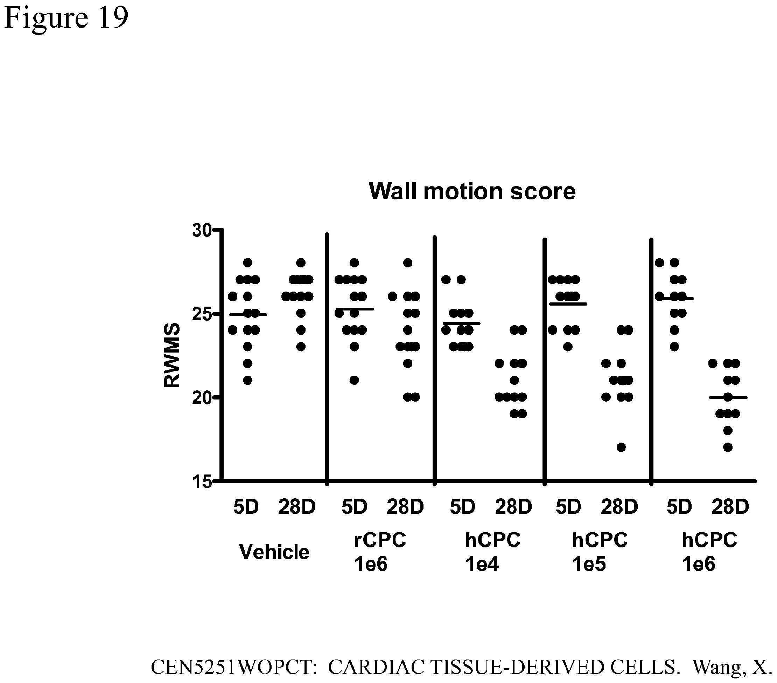

**Figure 19** shows the effect of the cardiac tissue-derived cells of the present invention on regional wall motion score (RWMS) in animals wherein acute myocardial infarctions have been induced, as measured by echocardiography. Each panel separated by a vertical solid line is an experimental arm in the study. 5D and 28D reflect 5 days and 28 days after induction of AMI respectively. The RWMS was measured at 5D as baseline and 28D as a follow-up. Animals were dosed with rCTC (A2) or hCTC (A3) cells at the doses indicated on the x-axis.

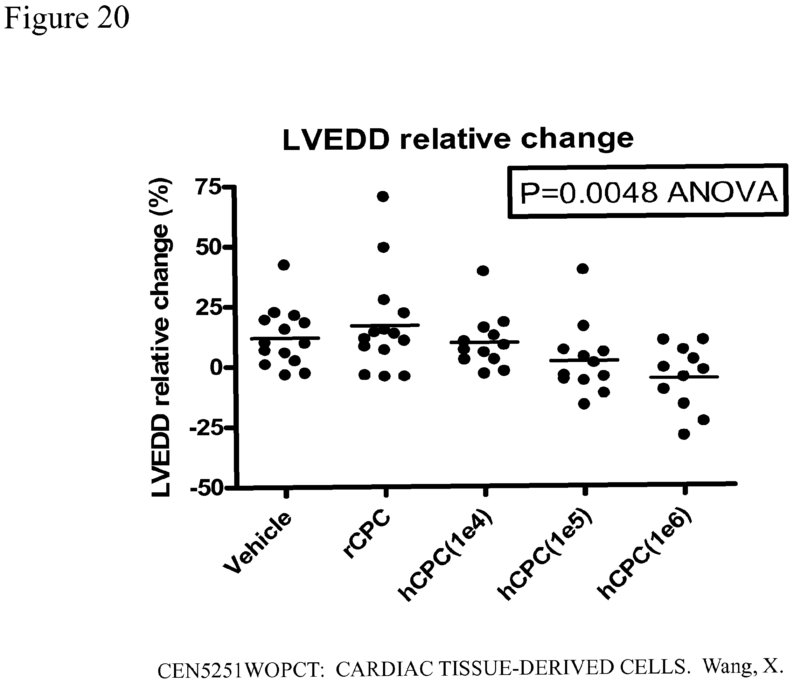

**Figure 20** shows the effect of the cardiac tissue-derived cells of the present invention on left ventricular end diastolic dimension (LVEDD) in animals wherein acute myocardial infarctions have been induced. LVEDD is a measurement of left chamber dimension at the end of diastole. LEVDD was measured at 5 days (5D) and 28 days (28D) after induction of myocardial infarction. Data shown is the relative change [(28D-5D)/5D] of individual animals. Animals were dosed with rCTC (A2), or hCTC (A3) cells at the doses indicated on the x-axis.

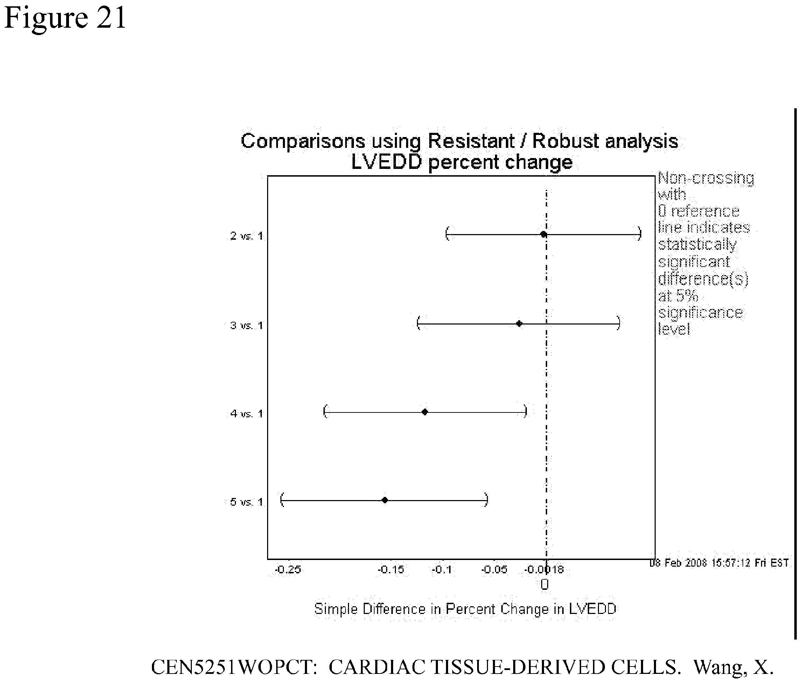

**Figure 21** shows the statistical analysis, comparing the relative change of LVEDD in each experimental group. An F-test was applied to the data using one-way analysis of variance (ANOVA). Group 1: vehicle; group 2: rCTC (A2) cells 1 x 10⁶ cells (target dose); group 3: hCTC (A3) cells 1 x 10⁴ cells (target dose); group 4: hCTC (A3) cells 1 x 10⁵ cells (target dose); group5: hCTC (A3) cells 1 x 10⁶ cells (target dose).

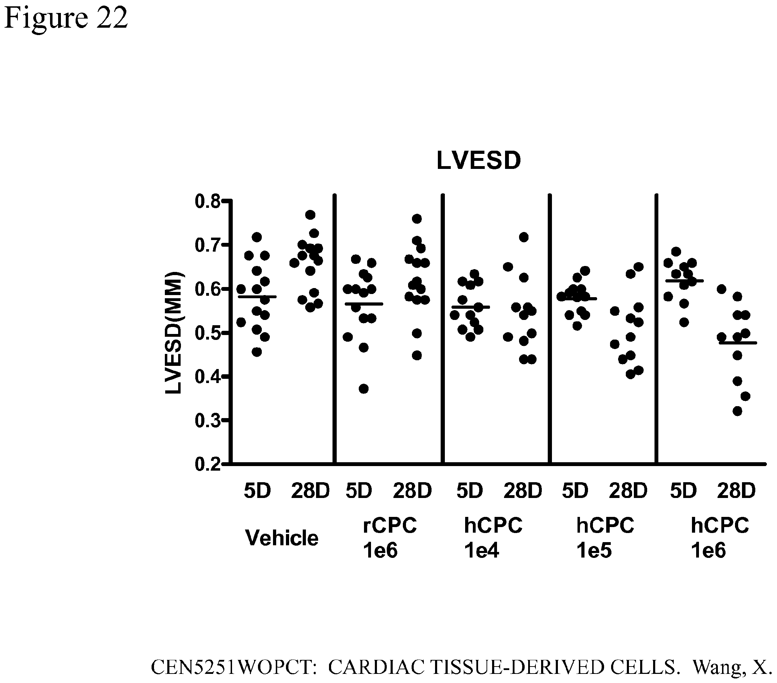

**Figure 22** shows the effect of human cardiac tissue-derived cell administration on left ventricular end systolic dimension (LVESD). LVESD is a measurement of chamber dimension at the end of systole in each cardiac cycle. Each panel separated by vertical solid line was an experimental arm in the study. LVESD was measured at 5 days (5D) and 28 days (28D) after induction of myocardial infarction. Data shown is the recordings from a single animal at each time point. Animals were dosed with rCTC (A2) or hCTC (A3) cells at the doses indicated on the x-axis.

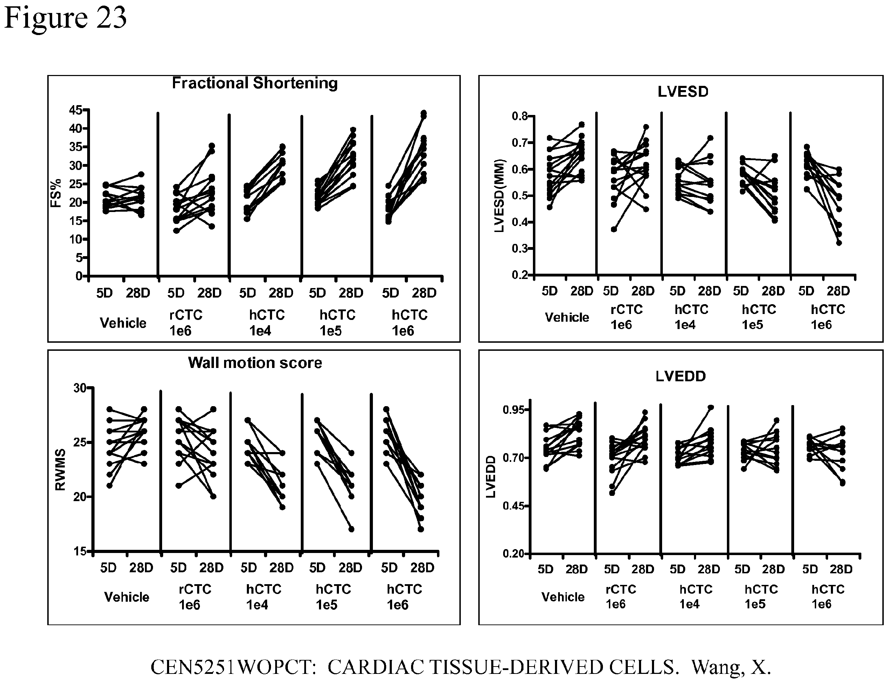

**Figure 23** shows the cardiac function at day 5 and day 28 post infarction and human cardiac tissue-derived cell administration, in individual animals in four parameters (FS, RWMS, LVESD, LVEDD) measured by echocardiography. Each black dot depicts individual animal's cardiac function at the time point indicated on the X axis. The black solid line shows the trend of change from 5D to 28D in each animal. Each panel depicts one parameter measured by echocardiography.

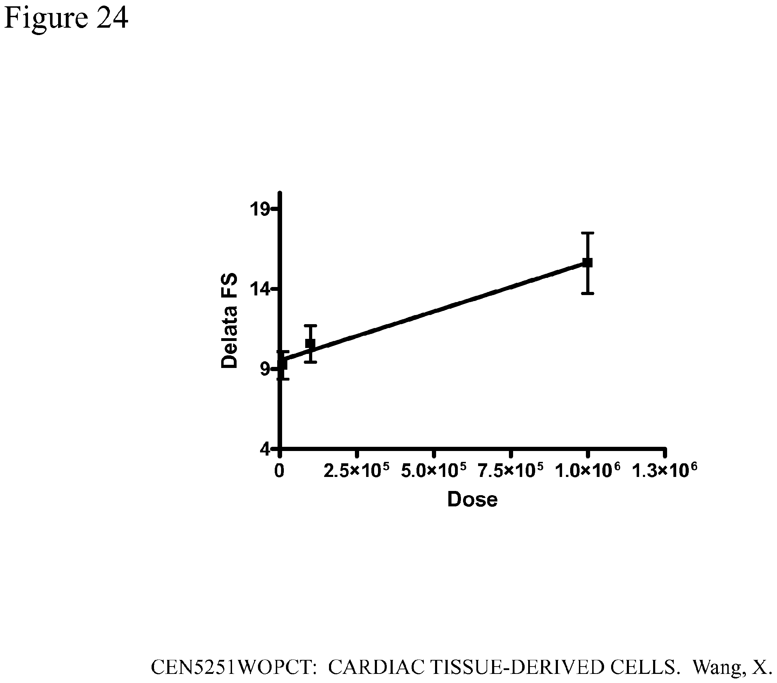

**Figure 24** shows the correlation between fractional shortening and the dose of cardiac tissue-derived cells. Y axis is the absolute change at day 28 from day 5 post infarction and cell administration (28D-5D). X axis is the dose of hCTC (A3) on linear scale. Data are expressed as Mean ± S.D (n=35).

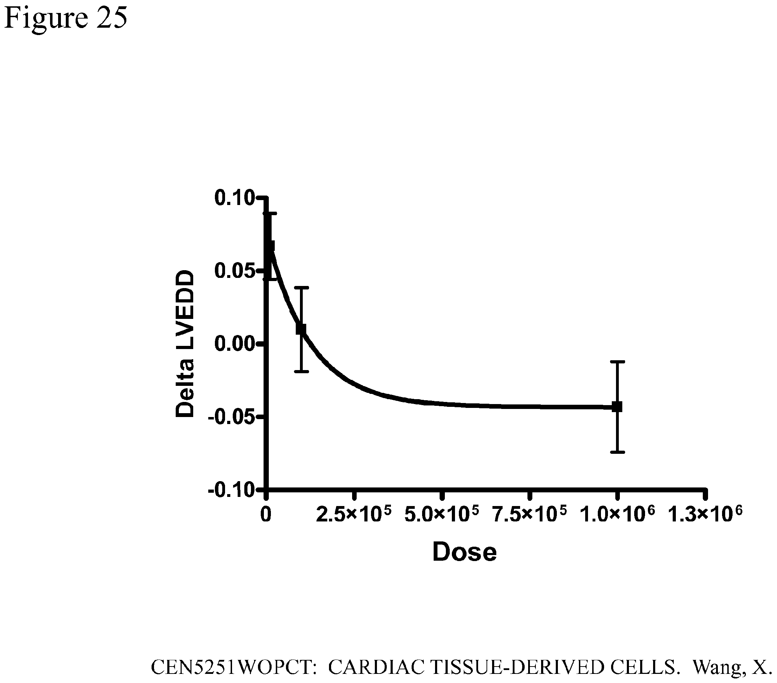

**Figure 25** shows the correlation between LVEDD change and the dosage of the human cardiac-derived tissue of the present invention. The y-axis depicts the absolute change at day 28 from day 5 post infarction and cell administration (28D-5D). The x-axis depicts the dose of hCTC (A3) cells on a linear scale. Data are expressed as Mean ± S.D (n=35).

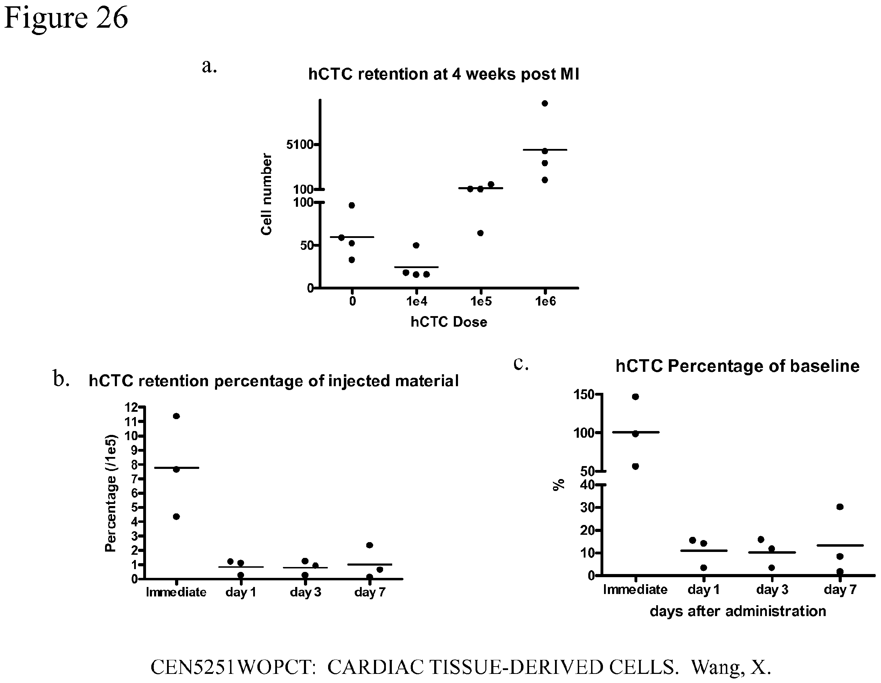

**Figure 26** shows the retention of hCTC (A3) cells administered to the heart of animals that have myocardial infactions. Retention was estimated from the level of beta-microglobulin expression detected in rat hearts. Panel "a" shows hCTC (A3) cell retention at 4 weeks after administration at the doses indicated on the x-axis. Panel "b" shows the time course of hCTC (A3) cell retention where the x-axis depicts the number of days after cell administration in rat MI heart, and the y-axis shows the percentage of the target dose. Panel "c" shows hCTC (A3) cell retention over time, using an average percentage of the cells detected, setting the amount of human cells detected immediately after cell administration at 100%. The x-axis depicts the number of days after cell administration in rat MI heart.

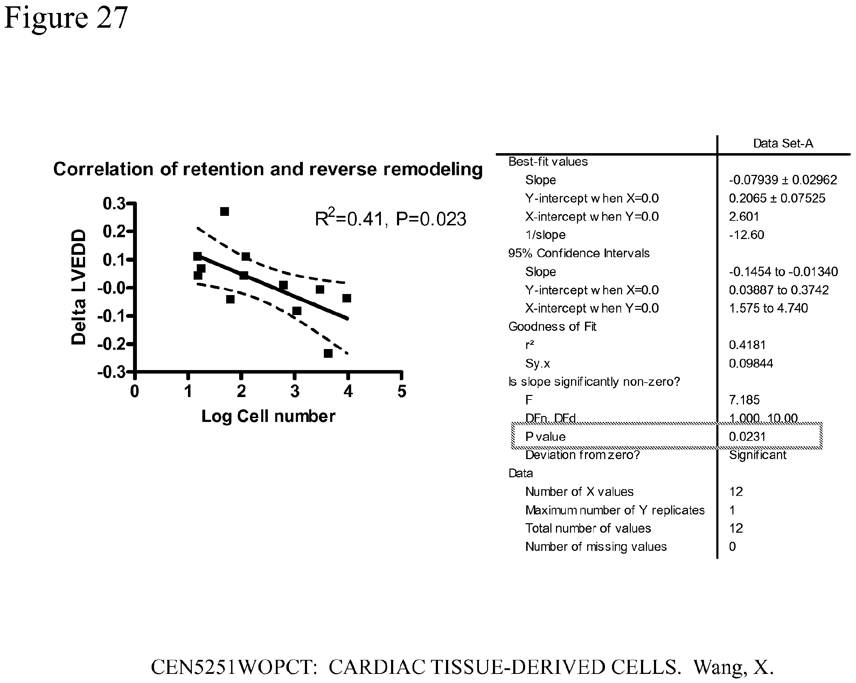

**Figure 27** shows the correlation between hCTC (A3) retention and prevention of remodeling in rat MI. Left panel shows the correlation graph. The x-axis depicts cell number on logarithmic scale; the y-axis depicts remodeling changes (delta LVEDD, 28D-5D). Each animal's cell number at 4 weeks after administration and the corresponding delta LVEDD were plotted in the graph. The right panel shows the statistical analysis of the linear regression.

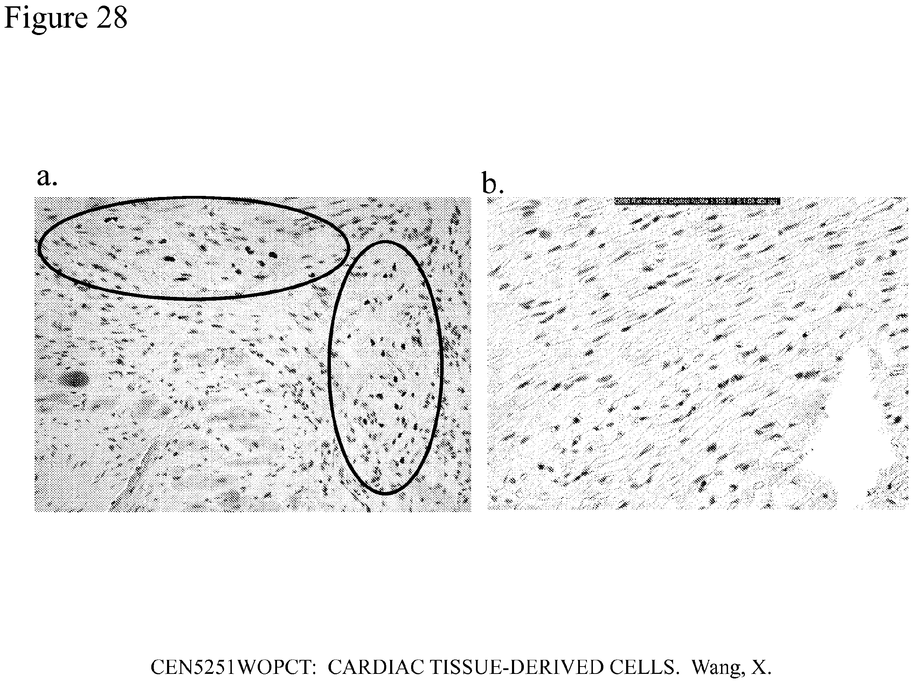

**Figure 28** shows human NuMA (Nuclear Matrix Antigen) localization in rat myocardium treated with hCTC (A3) cells (a targeted dose of 1 x 10⁶ cells). Left panel shows the positive human NuMA staining observed the target dose of 1 x 10⁶ hCTC-treated rat myocardium at 400-fold magnification. The right panel shows the staining in a vehicle control animal.

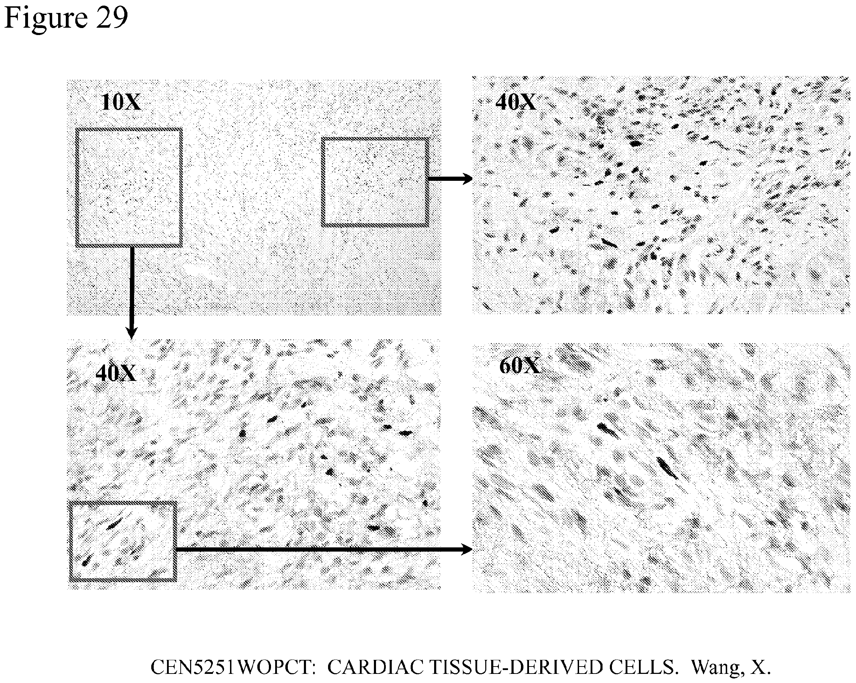

**Figure 29** shows human NuMA localization in another animal receiving a targeted dose of 1 x 10⁶ hCTC (A3) cells. The top left panel shows a low power image (100-fold magnification) showing two clusters of NuMA positive cells. The top right panel is a high magnification image (400-fold magnification), of clusters of NuMA positive cells. The bottom left panel is a high magnification image of NuMA positive cell cluster. The bottom right panel is a high magnification image showing NuMA positive cell nuclei with myocyte-like morphology.

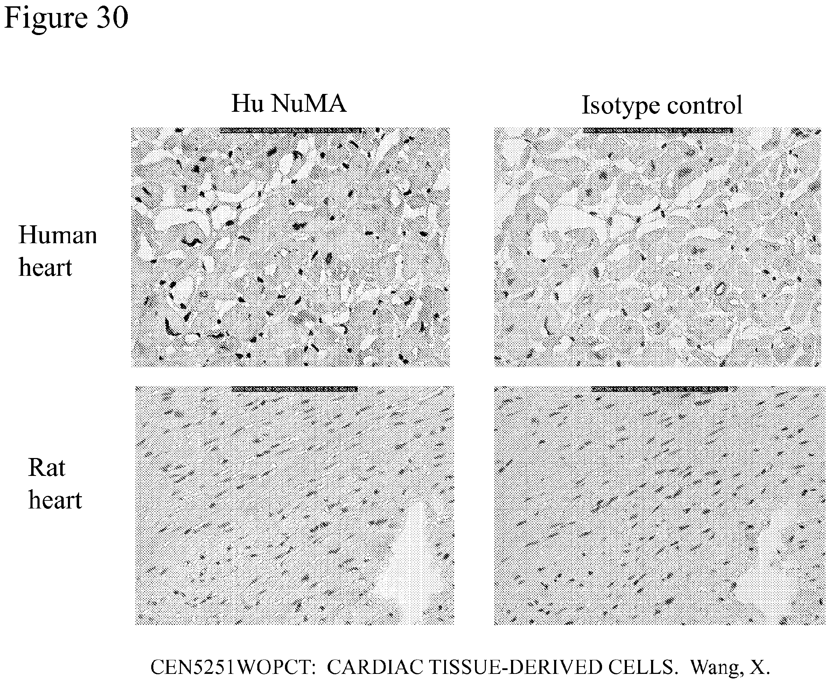

**Figure 30** shows the staining of antibody controls for NuMA observed in human and rat myocardium. The top two images show NuMA positive staining (left panel) with high nuclear specificity (non-staining with isotype control, right panel) in human heart. The bottom two images show rat heart controls demonstrating the NuMA antibody's specificity to human cells.

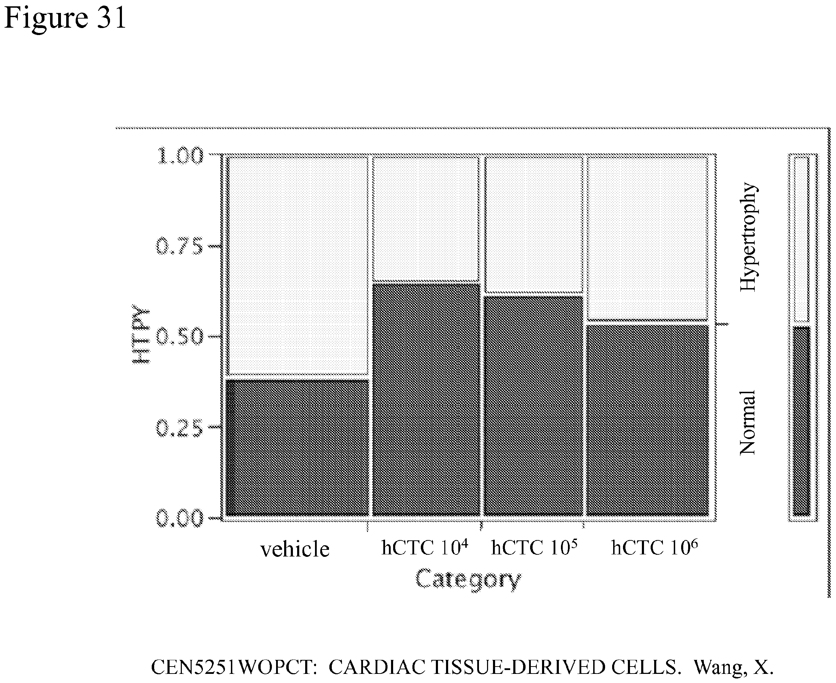

**Figure 31** shows the scoring evaluation of myocardial hypertrophy in hCTC (A3)-treated or vehicle-treated groups. The target dose is indicated on the y-axis. Animals receiving hCTC (A3) cells received a target dose of either 1 x 10⁴, 1 x 10⁵, or 1 x 10⁶ cells. The light grey area shows the proportion of non-hypertrophy sections in whole heart. The dark grey area shows the proportion of hypertrophic sections in whole heart.

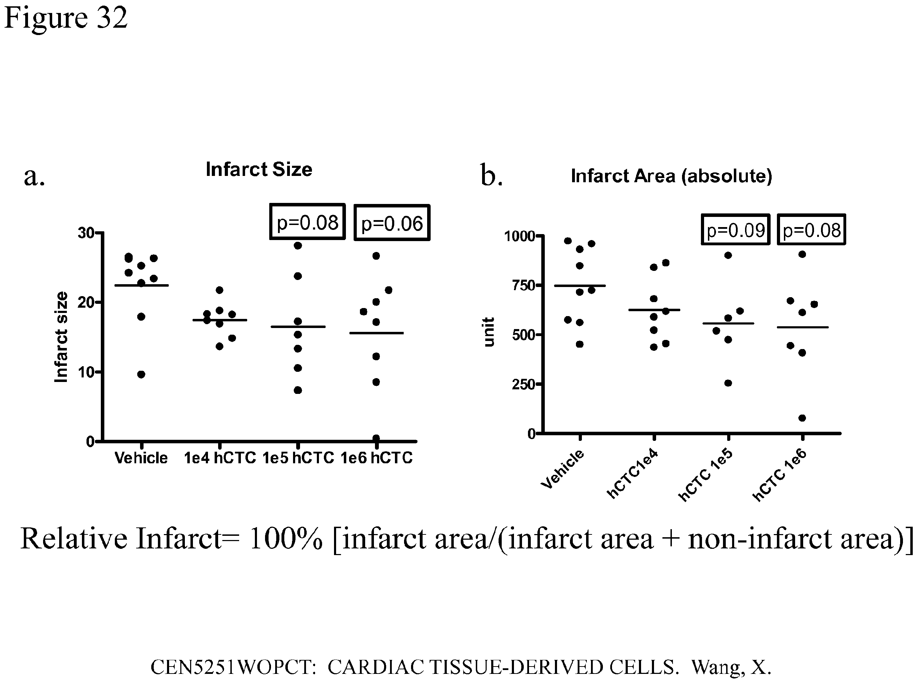

**Figure 32** shows infarct size assessment. Left panel shows the relative infarct size (percentage of infarct area in total left ventricular area); Right panel shows the absolute infarct area. The black dots depict each individual animal. The average size of infarct of the group was shown as solid black line.

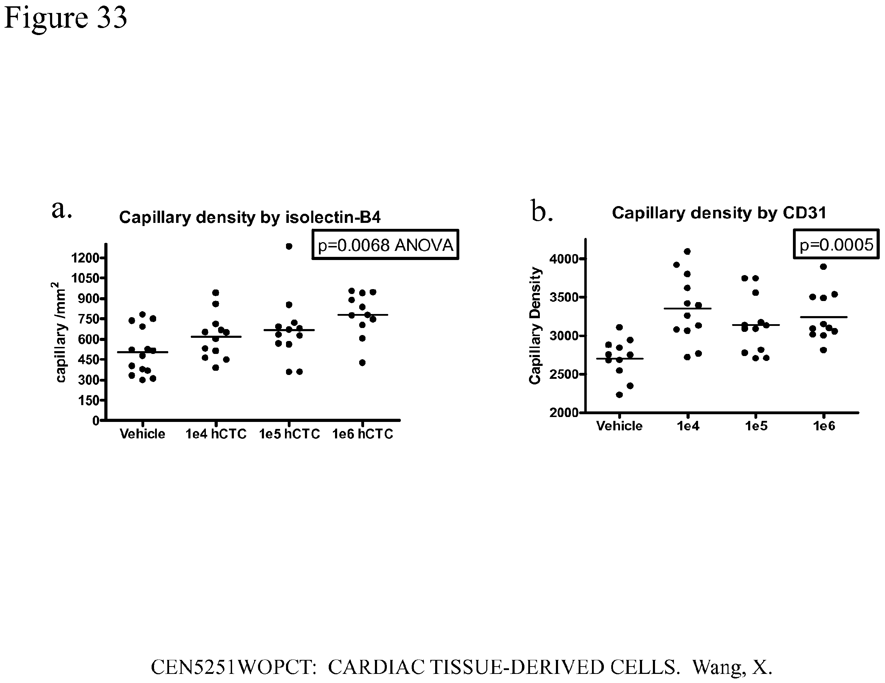

**Figure 33** shows the staining of capillary density in hCTC (A3) cell-treated, or vehicle-treated groups. Animals receiving hCTC (A3) cells received a target dose of either 1 x 10⁴, 1 x 10⁵, or 1 x 10⁶ cells. Panel "a" shows capillary density at the border zone of the infarct in myocardium, as detected with isolectin-B4 staining. Panel "b" shows capillary density at the border zone, as detected by CD31 staining.

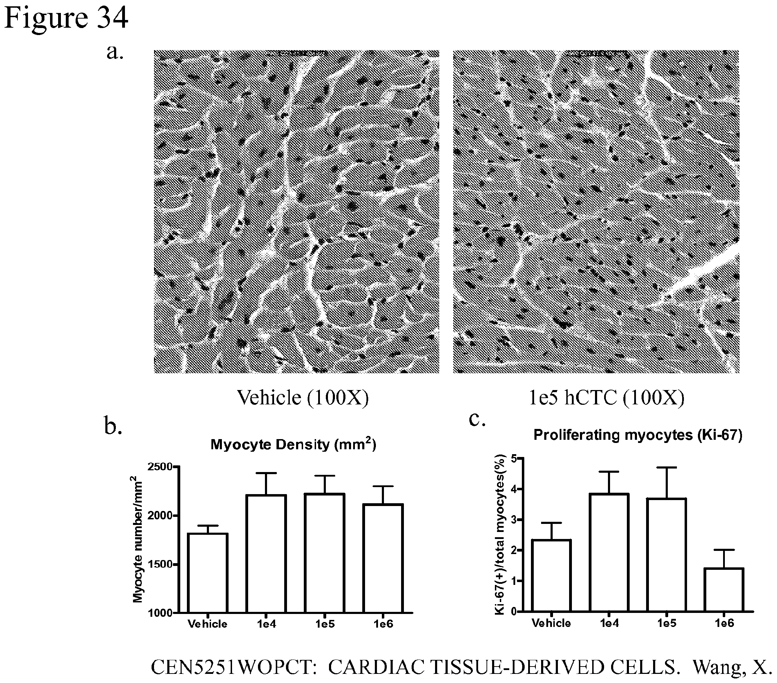

**Figure 34** shows the myocyte density at the non-infarcted area. Panel "a" shows representative images of H&E staining of the myocardium from vehicle treated animals (left panel) and animals treated with 1 x 10⁵ hCTC (A3) cells (right panel). Panel "b" shows the myocyte density observed in non-infarcted areas of the heart, expressed as myocyte numbers per mm². Data are shown as Mean ± SD (n=6); Panel c shows proliferating myocytes that were observed by double-staining of Ki-67 and myosin heavy chain. Data are expressed as Mean ± SD (n=6).

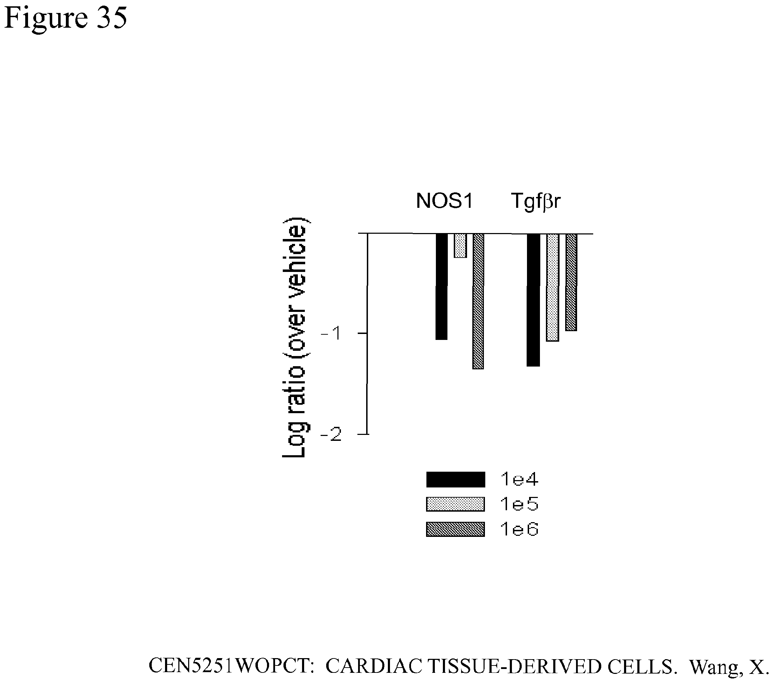

**Figure 35** shows differentially expressed genes in rat myocardium in response to hCTC (A3) cell treatment at all target doses.

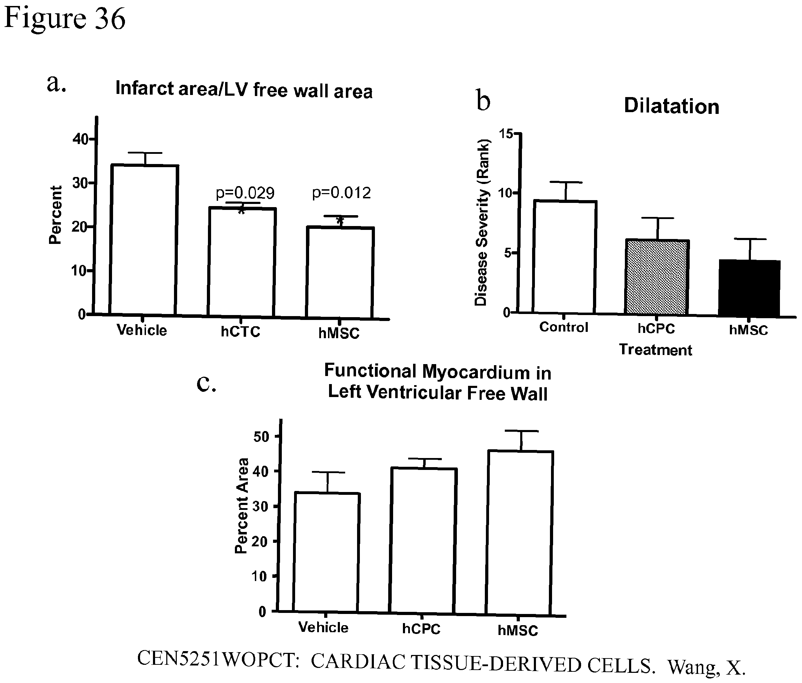

**Figure 36** shows the quantification of the effect of hCTC and hMSC cell administration on cardiac tissue in rats suffering from an acute MI. Panel "a" shows the ratio of infarct area vs. healthy tissue in the left ventricular free wall. Panel "b" shows the ranking of dilatation observed in hearts from animals in all groups. Panel "c" shows viable myocardium.

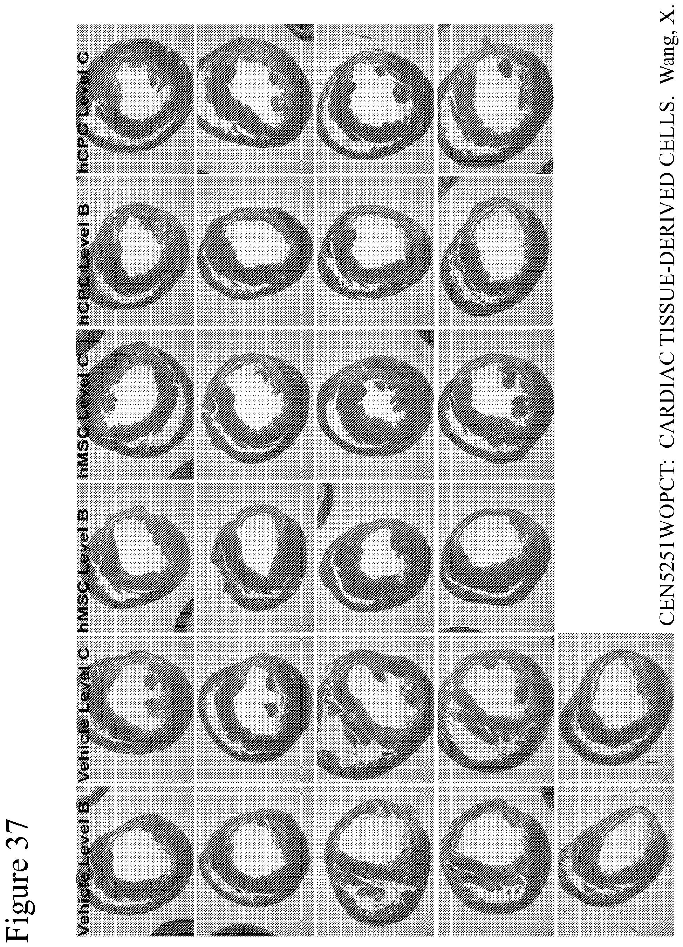

**Figure 37** shows the effects of hCTC (A3) cell and human mesenchymal stem cell (Cat # PT-2501, Lonza, Walkersville, MD) administration on the cardiac tissue in rats suffering from an acute MI. Two sections are shown side-by-side from each animal: one taken from the mid line between the papillary muscle and atrial level and one taken from the papillary muscle. The left two columns are from the vehicle treated group; the middle two columns were from hMSC treated group (1 x 10⁶ targeted dose); the right two columns were from the hCTC (A3) cell treated group (1 x 10⁵ targeted dose).

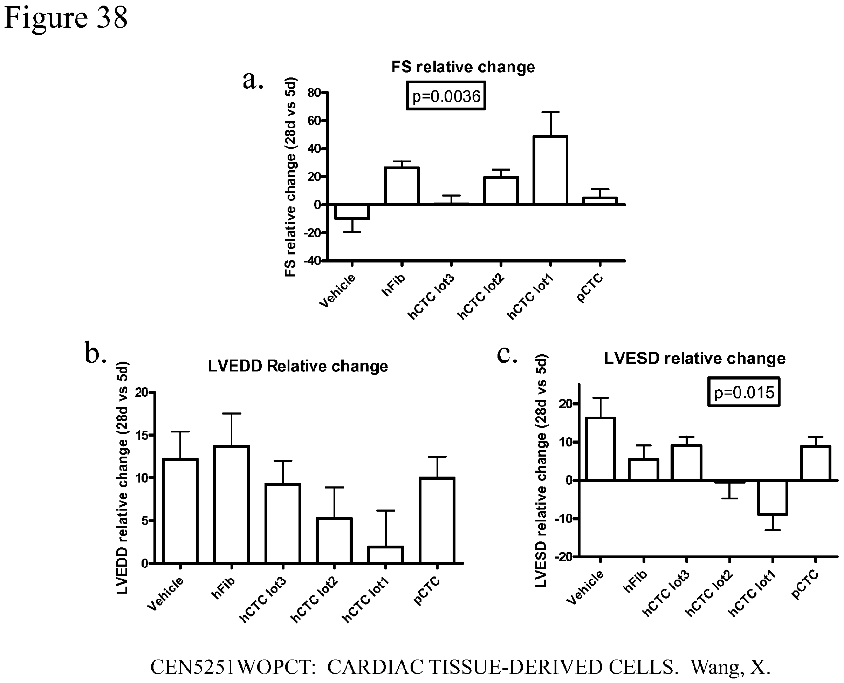

**Figure 38** shows the effect of hCTC (A3) cell administration on cardiac function in rats suffering from an acute MI, at day 28 after infarction and cell administration. Three parameters (FS, LVESD, LVEDD) were measured by echocardiography. Relative change from baseline (day 7 post infarction and cell administration) at day 28 post cell administration and infarction is presented. Three hCTC (A3) cell lots from different donors, human dermal fibroblasts and pCTC (A3) cells are shown.

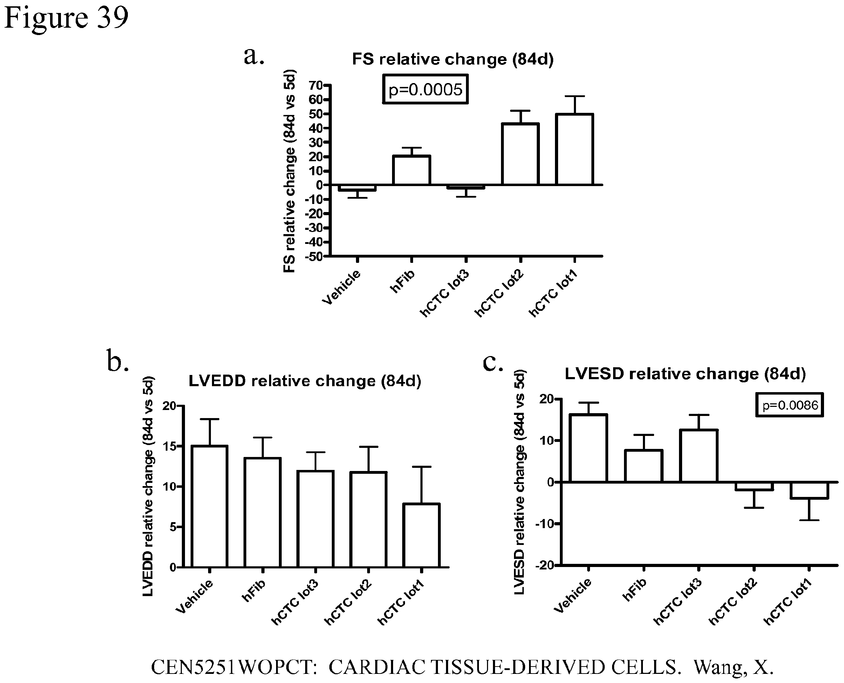

**Figure 39** shows the effect of hCTC (A3) cell administration on cardiac function in rats suffering from an acute MI, at day 84 post infarction and cell administration. Three parameters (FS, LVESD, LVEDD) were measured by echocardiography. Relative change from baseline (day 7 post infarction and cell administration) at day 84 post cell administration and infarction is presented. Human dermal fibroblasts and three different hCTC (A3) cell lots that were prepared from different donors were examined.

### DETAILED DESCRIPTION

For clarity of disclosure, and not by way of limitation, the detailed description of the invention is divided into the following subsections that describe or illustrate certain features, embodiments, or applications of the present invention.

### Definitions

As used herein, the term "damaged myocardium" refers to myocardial cells which have been exposed to ischemic conditions. These ischemic conditions may be caused by a myocardial infarction, or other cardiovascular disease or related complaint.

"Acute myocardial infarction" as used herein refers to the condition commonly known as a "heart attack," wherein when the blood supply to part of the heart is interrupted causing some heart cells to die. This is most commonly due to occlusion (blockage) of a coronary artery following the rupture of a vulnerable atherosclerotic plaque, which is an unstable collection of lipids (like cholesterol) and white blood cells (especially macrophages) in the wall of an artery. The resulting ischemia (restriction in blood supply) and oxygen shortage, if left untreated for a sufficient period, can cause damage and/or death of heart muscle tissue (myocardium).

The term "hCTC (S) population" or "hCTC (S)" as used herein refers to a non-adherent population of human cardiac tissue-derived cells that is obtained following the initial culture of cells after the human cardiac tissue has been dissociated, enzymatically digested, and filtered according to the methods of the present invention.

The term "hCTC (A1) population" or "hCTC (A1) cells" as used herein as used herein refers to an adherent population of human cardiac tissue-derived cells that is obtained following the initial culture of cells after the human cardiac tissue has been dissociated, enzymatically digested, and filtered according to the methods of the present invention.

The term "hCTC (A2) population" or "hCTC (A2) cells" as used herein refers to a population of adherent cells that result from the *in vitro* culture of hCTC (S) cells.

The term "hCTC (A3) population" or "hCTC (A3) cells" as used herein refers to a population of adherent cells that result from the *in vitro* culture of a mixture of hCTC (S) and hCTC (A1) cells.

### Methods to Derive the Cells of the Present Invention

The present invention provides a method to produce human cardiac tissue-derived cells according to the claims, that do not express telomerase, comprising the steps of:
a. Obtaining heart tissue,
b. Dissociating the heart tissue,
c. Digesting the heart tissue to release cells,
d. Removing the cardiomyocytes from the released cells and
e. Culturing the remaining cells.

The heart tissue may be dissociated manually. Alternatively, the heart tissue may be dissociated mechanically.

The cardiomyocytes may be removed from the released cells by any suitable method. For example, the cardiomyocytes may be removed by filtration, centrifugation, or by FACS.

The cells released from the digestion of the cardiac tissue may be filtered to remove the cardiomyocytes. The purpose of the filtration step is to exclude cells that are larger in size than the human cardiac tissue-derived cells of the present invention. The human cardiac tissue derived cells of the present invention may be from about 5 microns to about 50 microns in diameter, and a filter of a pore size of 50 microns is chosen to allow the human cardiac tissue-derived cells of the present invention to pass through the filter.

The human cardiac tissue-derived cells that pass through the filter may be cultured *in vitro.* The human cardiac tissue-derived cells that are cultured *in vitro* after the filtration step may be a mixture of non-adherent cells and adherent cells.

The human cardiac tissue-derived cells of the present invention may adhere to any solid substrate. The solid substrate may be polycarbonate. Alternatively, the solid substrate may be polystyrene. Alternatively, the solid substrate may be glass. The solid substrate may also be coated with an adlayer comprising an extracellular matrix protein, such as, for example, collagen or laminin, and the like.

The adherent cells that are obtained after the initial culture step are referred to herein as the human cardiac tissue-derived (A1) population of cells, or hCTC (A1) cells. The non-adherent cells that are obtained after the initial culture step are referred to herein as the human cardiac tissue-derived (S) population of cells, or hCTC (S) cells.

In one disclosure, hCTC (A1) cells are expanded in culture. The hCTC (A1) cells may be cultured in any suitable tissue culture medium. For example, the cardiac tissue-derived cells may be cultured in DMEM, supplemented with 1,000 g/l D-glucose, 584 mg/l L-glutamine, and 110 mg/l sodium pyruvate, and 10% FBS. Antibiotics such as, for example, penicillin 50 U/ml and streptomycin 50 µg/ml may be added to the culture medium. Alternatively, antibiotics may be added to the suspension of cells obtained following dissociation and enzymatic digestion of the heart tissue. The hCTC (A1) cells may be plated at a seeding density of about 1,000 to about 10,000 viable cells / cm² on tissue culture substrates. The hCTC (A1) cells may be incubated under 5-20 %v/v atmospheric oxygen.

In one disclosure, the hCTC (A1) cells are passaged once the cells reach approximately 80% confluence. Alternatively, the hCTC (A1) cells are passaged once the cells reach approximately 90% confluence. Alternatively, the hCTC (A1) cells are passaged every one to seven days.

In one disclosure, hCTC (S) cells are expanded in culture. In one disclosuret, the hCTC (S) cells of may be cultured in any suitable tissue culture medium. For example, the cardiac tissue-derived cells may be cultured in DMEM, supplemented with 1,000 g/l D-glucose, 584 mg/l L-glutamine, and 110 mg/l sodium pyruvate, and 10% FBS. Antibiotics such as, for example, penicillin 50 U/ml and streptomycin 50 µg/ml may be added to the culture medium. Alternatively, antibiotics may be added to the suspension of cells obtained following dissociation and enzymatic digestion of the heart tissue. The hCTC (S) cells may be incubated under 5-20 % v/v atmospheric oxygen. In one disclosure, the tissue culture medium is replaced every three days.

In one disclosure, the hCTC (S) cells become adherent with time in culture. The time in culture in which the hCTC (S) cells become adherent is from about 1 days to about 7 days. The population of adherent cells that result from the hCTC (S) cells becoming adherent is referred to herein as the human cardiac tissue-derived (A2) population of cells, or hCTC (A2) cells.

In one disclosure, hCTC (A2) cells are expanded in culture. The hCTC (A2) cells may be cultured in any suitable tissue culture medium. For example, the cardiac tissue-derived cells may be cultured in DMEM, supplemented with 1,000 g/l D-glucose, 584 mg/l L-glutamine, and 110 mg/l sodium pyruvate, and 10% FBS. Antibiotics such as, for example, penicillin 50 U/ml and streptomycin 50 µg/ml may be added to the culture medium. Alternatively, antibiotics may be added to the suspension of cells obtained following dissociation and enzymatic digestion of the heart tissue. The hCTC (A2) cells may be plated at a seeding density of about 1,000 to about 10,000 viable cells / cm² on tissue culture substrates. The hCTC (A2) cells may be incubated under 5-20 % v/v atmospheric oxygen.

In one disclosure, the hCTC (A2) cells are passaged once the cells reach approximately 80% confluence. Alternatively, the hCTC (A2) are passaged once the cells reach approximately 90% confluence. Alternatively, the hCTC (A2) cells are passaged every one to seven days.

In one disclosure, a mixture of hCTC (A1) cells and hCTC (S) cells are expanded in culture. In one disclosure, the mixture of hCTC (A1) cells andhCTC (S) cells form a population of adherent cells with time in culture. The time in culture in which the hCTC (S) cells become adherent is from about 2 days to about 14 days. The population of adherent cells that result from the mixture of hCTC (A1) cells and hCTC (S) cells becoming adherent is referred to herein as the human cardiac tissue-derived (A3) population of cells, or hCTC (A3) cells.

In one disclosure, hCTC (A3) cells are expanded in culture. The hCTC (A3) cells may be cultured in any suitable tissue culture medium. For example, the cardiac tissue-derived cells may be cultured in DMEM, supplemented with 1,000 g/l D-glucose, 584 mg/l L-glutamine, and 110 mg/l sodium pyruvate, and 10% FBS. Antibiotics such as, for example, penicillin 50 U/ml and streptomycin 50 µg/ml may be added to the culture medium. Alternatively, antibiotics may be added to the suspension of cells obtained following dissociation and enzymatic digestion of the heart tissue. The hCTC (A3) cells may be plated at a seeding density of about 1,000 to about 10,000 viable cells / cm² on tissue culture substrates. The hCTC (A3) cells may be incubated under 5-20 % v/v atmospheric oxygen.

In one disclosure, the hCTC (A3) cells are passaged once the cells reach approximately 80% confluence. Alternatively, the hCTC (A3) cells are passaged once the cells reach approximately 90% confluence. Alternatively, the hCTC (A3) cells are passaged every one to seven days.

One method by which to obtain the human cardiac tissue-derived cells of the present invention is outlined in **Figure 1**. An alternate method by which to obtain the human cardiac tissue-derived cells of the present invention is outlined in **Figure 2**. An alternate method by which to obtain the human cardiac tissue-derived cells of the present invention is outlined in **Figure 3**.

The cells of the present invention may be derived from dissociating the whole heart, and subsequently digesting the dissociated tissue. Alternatively, cells of the present invention may be derived from dissociating portions of heart tissue, and subsequently digesting the dissociated tissue. The portions may be obtained from any part of the heart, such as, for example, the atria, or the ventricles, the apex of the heart, or either side of the heart.

The dissociated heart tissue can be digested using enzymes such as, for example collagenase and dispase. The enzymes may be used separately or alternatively in combination. The dissociated heart tissue can be digested using a mixture of collagenase and dispase.

The collagenase may be used at a concentration from about 0.1 U/ml to about 10 U/ml. Alternatively the collagenase may be used at a concentration from about 0.1 U/ml to about 5 U/ml. Alternatively the collagenase may be used at a concentration of about 5 U/ml. Alternatively the collagenase may be used at a concentration of about 1 U/ml.

The dispase may be used at a concentration from about 0.5 U/ml to about 50 U/ml. Alternatively the collagenase may be used at a concentration from about 0.5 U/ml to about 10 U/ml. Alternatively the collagenase may be used at a concentration of about 10 U/ml. Alternatively the collagenase may be used at a concentration of about 5 U/ml.

The dissociated tissue may be treated with the enzymes for about 5 minutes to about 5 hours. Alternatively the dissociated tissue may be treated with the enzymes for about 30 minutes to about 5 hours. Alternatively the dissociated tissue may be treated with the enzymes for about 30 minutes to about 4 hours. Alternatively the dissociated tissue may be treated with the enzymes for about 30 minutes to about 3 hours. Alternatively the dissociated tissue may be treated with the enzymes for about 30 minutes to about 2 hours. Alternatively the dissociated tissue may be treated with the enzymes for about 30 minutes to about 1 hour. The dissociated tissue can be treated with the enzymes for about 2.5 hours.

If desirable, the cardiac tissue-derived cells of the present invention may be exposed, for example, to an agent (such as an antibody) that specifically recognizes a protein marker expressed by the cardiac tissue-derived cells, to identify and select cardiac tissue-derived cells, thereby obtaining a substantially pure population of cardiac tissue-derived cells.

### The Cells of the Present Invention

The present invention provides a human cardiac tissue-derived cell population that does not express telomerase, according to the claims, that can be maintained and expanded in culture, and is useful in the treatment and repair of damaged myocardium. The properties of the cardiac tissue-derived-cells of the present invention are summarized in **Table 1.**

In one embodiment, according to the claims, the human cardiac tissue-derived cells of the present invention that do not express telomerase, express at least one of the following markers: CD49e, CD105, CD59, CD54, CD90, CD34, and CD117.

In one embodiment, according to the claims, the human cardiac tissue-derived cells of the present invention that do not express telomerase do not express at least one of the following markers: MDR, CD19, CD16, CD31, CD45 and Isl-1.

In one embodiment, according to the claims, the human cardiac tissue-derived cells of the present invention that do not express telomerase, express the following markers: CD49e, CD105, CD59, CD54, CD90, CD34, and CD117.

In one embodiment, according to the claims, the human cardiac tissue-derived cells of the present invention that do not express telomerase do not express the following markers: MDR, CD19, CD16, CD31, CD45 and Isl-1.

In one embodiment, the human cardiac tissue-derived-cells of the present invention are further differentiated into cardiomyocytes. This differentiation may be prior to, or, alternatively after, administration into the patient. Differentiation refers to the act of increasing the extent of the acquisition or possession of one or more characteristics or functions, which differ from that of the original cell (i.e., cell specialization). This can be detected, for example, by screening for a change in the phenotype of the cell (i.e., identifying morphological changes in the cell and/or surface markers on the cell). Any method capable of differentiating the cardiac tissue-derived cells of the present invention into cardiomyocytes may be used.

For example, the cardiac tissue-derived-cells of the present invention may be further differentiated into cardiomyocytes according to the methods disclosed in U.S. Patent Application US20040126879.

In another example, the cardiac tissue-derived-cells of the present invention may be further differentiated into cardiomyocytes according to the methods disclosed in WO2004019767.

### Methods to Treat or Repair Damaged Myocardium

Damaged myocardium results from a variety of cardiac diseases, such as, for example acute myocardial infraction, chronic myocardial infraction, congestive heart failure, and the like. The cardiac tissue-derived cells of the present invention may be used a therapy to repair damaged myocardium. In one embodiment, the cardiac tissue-derived cells of the present invention are used as a therapy to repair myocardium that is damaged as a result of acute myocardial infarction.

In one embodiment, the present invention provides a population of cardiac tissue-derived cells according to the claims for use a method to treat damaged myocardium in a patient comprising the steps of:
a. Obtaining a population of human cardiac tissue-derived cells that do not express telomerase, and
b. Administering the population of human cardiac tissue-derived cells to the patient in an amount sufficient to treat the damaged myocardium.

In one embodiment, the present invention provides a population of cardiac tissue-derived cells according to the claims for use a method to repair damaged myocardium in a patient comprising the steps of:
a. Obtaining a population of human cardiac tissue-derived cells that do not express telomerase, and
b. Administering the population of human cardiac tissue-derived cells to the patient in an amount sufficient to repair the damaged myocardium.

In one embodiment, the population of human cardiac tissue-derived cells are prepared and administered to the patient without culturing the cells. In an alternate embodiment, the population of cardiac tissue-derived cells are prepared and cultured *in vitro,* prior to administering to the patient.

In the case where the population of human cardiac tissue-derived cells are cultured and expanded *in vitro,* the population of cells that is cultured and expanded may be a population of hCTC (A1) cells. Alternatively, the population of cells that is cultured and expanded may be a population of hCTC (A2) cells. Alternatively, the population of cells that is cultured and expanded may be a population of hCTC (A3) cells.

The human cardiac tissue-derived cells of the present invention may be administered to a patient suffering from damaged myocardium by any suitable method in the art. Such methods are readily selected by one of ordinary skill in the art.

For example, administration of the human cardiac tissue-derived cells of the present invention to the damaged myocardium may be via direct injection of the damaged myocardium. Alternatively, the human cardiac tissue-derived cells of the present invention may be administered systemically. Where the human cardiac tissue-derived cells of the present invention are delivered systemically, the efficiency of delivering the cells to the damaged myocardium may be enhanced, for example, by treating the patient with at least one other agent, or by another method capable of enhancing the delivery of cells to the damaged myocardium.

For example, the human cardiac tissue-derived cells of the present invention may be administered along with another agent selected from the group consisting of: stem cell factor (SCF), granulocyte-colony stimulating factor (G-CSF), granulocyte-macrophage colony stimulating factor (GM-CSF), stromal cell-derived factor-1, steel factor, vascular endothelial growth factor, macrophage colony stimulating factor, granulocyte-macrophage stimulating factor, and Interleukin-3.

In one embodiment, the human cardiac tissue-derived cells of the present invention are administered along with another agent according to the methods disclosed in U.S. Patent Application US20020061587.

In one embodiment, the human cardiac tissue-derived cells of the present invention are administered along with another agent according to the methods disclosed in U.S. Patent Application US2002162796.

For example, the delivery of cells to the damaged myocardium may be enhanced by isolating the patient's cardiac circulation from the patient's systemic circulation and perfusing a solution comprising cells into the cardiac circuit. An example of this method is disclosed in WO2007067502.

In one embodiment, the human cardiac tissue-derived cells of the present invention are administered to the patient according ο the methods disclosed by Iwasaki, Kawamoto et al. (Circulation 113: 1311-1325; 2006).

In an alternate embodiment, the human cardiac tissue-derived cells of the present invention are administered to the patient using a catheter that can be inserted into coronary artery according to the methods disclosed by Sherman, Martens et al. (Nature Clinical Practice Cardiovascular Medicine 3, suppl 1: S57-64; 2006).

In an alternate embodiment, the human cardiac tissue-derived cells of the present invention are administered to the patient using a catheter that is capable of mapping the electrical activity of the myocardium. In one embodiment, the cardiac tissue-derived cells of the present invention are administered to the patient using a catheter that is capable of mapping the electrical activity of the myocardium according to the methods disclosed by Perin, Dohmann et al. (Circulation 107: 2294-2302; 2003); and Perin, Silva et al. (Journal of Molecular and Cellular Cardiology 44: 486-495; 2008). In an alternate embodiment, the cardiac tissue-derived cells of the present invention are administered to the patient according to methods disclosed by Sherman, Martens et al. (Nature Clinical Practice Cardiovascular Medicine 3, suppl 1: S57-64; 2006).

In an alternate embodiment, the human cardiac tissue-derived cells of the present invention are administered to the patient using a catheter that is capable of intra-myocardium injection according to the methods disclosed by Hashemi, Ghods et al. (European Heart Journal 29: 251-259; 2008).

The present invention is further illustrated, but not limited by, the following examples.

### Example 1

### Isolation of Human Cardiac Tissue-Derived Cells

*Materials and methods -digestion enzyme cocktail preparation:* The digestion enzyme cocktail used in the present invention to isolate cardiac tissue-derived cells from human heart was prepared as 2X cocktail stock solutions in Phosphate Buffered Saline (PBS, Gibco, Invitrogen, Carlsbad, CA). The concentrations are 0.2U/ml or 2U/ml Collagenase (Serva Electrophoresis GmbH, Heidelberg, Germany) and 10U/ml Dispase II (Roche Applied Science, Indianapolis, Indiana). These enzyme cocktail stocks were stored at - 40°C. Prior to digestion, the enzyme cocktail was filtered through 0.22 µm vacuum filter system (Corning Incorporated, Acton, MA). For human heart digestion, the 2U/ml Collagenase stock was used in the digestion procedure. The final concentrations of digestion enzymes are 1 U/ml collagenase and 5U/ml dispase. The process of digestion of the whole human heart and the isolation of human cardiac tissue-derived cells (hCTC) is summarized in **Figure 1****.**

*Material and methods* - *human heart:* Transplant-discard whole hearts were obtained from the National Development and Research Institutes (NDRI, New York, NY). The procurement time of the transplant-discard hearts was between 40-98 hours. The whole organ was immerged into growth medium (DMEM+10% fetal bovine serum) and stored at 4°C until being processed for cell isolation.

*Materials and methods* - *human heart tissue processing:* The whole heart tissue was transferred to a biosafety cabinet and placed into a square bioassay dish (Corning Inc., Acton, MA). The excess fat tissue was removed using sterile scalpels (Bard Parker, Becton Dickinson, Hancock, NY). The first whole heart was cut into small pieces (2-3 cm³). Three quarters of the small tissue pieces were then minced manually to fine pieces (1-2 mm³ in size). This procedure took two hours to complete. One quarter of the pieces were transferred to the PRO250 homogenizer chamber (Pro Scientific, Oxford, CT). The lid was placed on the chamber and the PRO250 generator attached with the speed of the generator set to 3. The tissue pieces were homogenized for 10 seconds at room temperature with no addition of any buffer and the tissue was visually inspected. The homogenizing was complete when the tissue was finely minced (resulting in fragments less than 1mm³ in size).

*Materials and methods* - *human tissue digestion:* The tissue pieces, both manually processed and homogenized-were transferred to separate 250 ml conical tubes (Corning Inc., Corning, NY). The tissue in each tube was washed three times by adding 100 ml room temperature PBS and inverting the tube five times. The tube was then placed upright and the tissue allowed to settle. The supernatant was aspirated using a 2 ml aspirating pipette (BD falcon, BD Biosciences, San Jose, CA). The digestion enzyme cocktail stock (2X) was added to the 250 ml tube at an enzyme to tissue ratio of 1:1. The final concentration of the enzymes was 1U/ml Collagenase and 5U/ml Dispase II. The tubes containing the tissue and enzymes were transferred to a 37°C orbital shaker set for 225 rpm (Barnstead Lab, Melrose Park, IL) and incubated for 2.5 hours. After incubation, the tube was transferred back to the biosafety cabinet. The cell suspension was diluted by filling the tubes with room temperature PBS. In order to remove any remaining undigested tissue, the cell suspension was filtered through an 8-inch diameter 250 µm standard testing sieve (Sigma-Aldrich, St. Louis, MO) and into a 500 ml glass beaker. Following this step, the cell suspension in the glass beaker was further filtered through 100 µm cell strainers (BD Falcon) and into multiple 50 ml conical tubes (BD Falcon). The cell suspension was then washed by centrifuging at 338 *x* g for 5 minutes at room temperature using a Sorvall Legend T centrifuge (Thermo Fisher Scientific, Inc, Waltham, MA) to pellet the cells. The supernatant was aspirated off and the cell pellets resuspended in PBS and pooled into separate 50 ml tubes, one each for the manually minced process and the homogenizing process. The cell suspension was washed three more times with 40 ml room temperature PBS. After washing, the pellet was resuspended in 20 ml ACK lysing buffer (Lonza, Walkersville, MD) and incubated for 10 minutes at room temperature to lyse any remaining red blood cells. After incubation the cell suspension was washed two more times with 40 ml room temperature PBS.
Following the final centrifugation, the pellet was resuspended in 20 ml room temperature growth medium (DMEM, 1,000 mg/L D-glucose, L-glutamine, and 110 mg/L sodium pyruvate, 10% fetal bovine serum, Penicillin 50U/ml, Streptomycin 50ug/ml) and counted.

*Materials and methods* - *Cell counting:* The total viable cell density and viability analysis was performed using the Vi-Cell™ XR (Beckman Coulter, Fullerton, CA). The Vi-Cell™ cell viability analyzer automates the trypan blue dye exclusion method for cell viability assessment using video captures technology and image analysis of up to 100 images of cells in a flow cell. The Vi-Cell has a counting accuracy of ± 6%. Samples were prepared and analyzed according to the manufacturer's instructions (Reference Manual PN 383674 Rev.A). Briefly, a 500µL aliquot of the final cell suspension obtained after RBC lysis was transferred to a Vi-Cell™ 4 ml sample vial and analyzed using a Vi-Cell™ XR Cell Viability Analyzer. The default cell type profile was used:

| | |
|---|---|
| Cell brightness | 85% |
| Cell sharpness | 100 |
| Viable cell spot brightness | 75% |
| Viable cell spot area | 5% |
| Minimum circularity | 0 |
| Decluster degree | Medium |
| Minimum diameter | 5 microns |
| Maximum diameter | 50 microns |
| Images | 50 |
| Aspirate cycles | 1 |
| Trypan blue mixing cycles | 3 |

*Results* - *Cell yield obtained from a whole heart digestion:* From the first whole heart, after digestion, the yield from the manually minced process produced 43 million viable cells after dissociation and enzymatic digestion. The viability was 65%. The mechanical homogenization procedure produced 12 million viable cells and viability was 63%. Since 3-times more tissue went through the manual procedure, there were no differences in yield and viability between the 2 procedures. Based on the results, subsequent human hearts were processed using mechanical homogenization. After digestion, total yield was typically 34-64 million viable cells per heart. The viability was 55-81%, as shown in **Table** 2.

### Example 2

### Selection and In Vitro Culture of the Human Cardiac Tissue-Derived Cells of the Present Invention

The cell suspension obtained following the dissociation and enzymatic digestion of a human heart was expanded for further experimental analysis.

*The initial plating of the cells obtained from dissociation and enzymatic digestion of a human heart:* The cell suspension obtained from the dissociation and enzymatic digestion of a human heart, according to the methods described in **Example 1** was added to T225 tissue culture flasks (Corning Inc., Corning, NY) flasks. 10 ml of the cell suspension was added to each flask, which contained 50 ml growth medium (DMEM, 1,000 mg/L D-glucose, 584 mg/L L-glutamine, and 110 mg/L sodium pyruvate, 10% fetal bovine serum, Penicillin 50U/ml, Streptomycin 50 µg/ml, Invitrogen, Calsbald, CA). The final volume of the initial culture was 60 ml. The cells were incubated at 37°C in an atmosphere comprising 20% O₂ and 5% CO₂, for 2 days. After this time, a heterogeneous cell culture was observed. Non-adherent, phase bright cells were observed (referred to herein as hCTC (S) cells), and adherent cells were observed (referred to herein as hCTC (A1) cells). See **Figure 4****.**

The hCTC (S) cells obtained after the initial culture step had a similar morphology to the cells described as cardiac stem cells by Anversa (Beltrami, Barlucchi et al. 2003; Cell 114(6): 763-76). See **Figure 4a****.** In addition, cell clusters, similar to those described by Messina *et al* (Messina, De Angelis et al. 2004; Circulation Research 95(9): 911-21) were observed, shown in **Figure 4b****.**

*Expansion of the hCTC (S) population*: In one study, the hCTC (S) cells were removed from the culture flasks after the initial two day culture period. The hCTC (S) cells were transferred to 50 ml conical tubes. The hCTC (S) cells were centrifuged at 338 x g for 5 minutes at room temperature. The supernatant was discarded and the cell pellet re-suspended in 20 ml fresh growth medium. hCTC (S) cells were counted after resuspension. The total number of hCTC (S) cells obtained from the initial culture step was about 10-14 million total cells. A fraction of the hCTC (S) cells were cryo-preserved in preservation medium at 1-1.5million/ml and stored at -140°C. The remainder were expanded in culture. The hCTC (S) cells were replated in flasks at seeding density of 5,000 cells/cm². After 2 days in culture, hCTC (S) cells became adherent, and formed a homogeneous adherent cell population, referred to herein as the hCTC (A2) population, or hCTC (A2) cells. Once the hCTC (A2) cells of the present invention reached 80% confluency at about 10-14 days after hCTC (S) plating, the cells were trypsinized by aspirating off the growth medium, washed with 60 ml room temperature PBS, and then 4 ml Trypsin-EDTA (Invitrogen, Carlsbad, CA) was added to each flask. The hCTC (A2) cells were incubated for approximately 5 minutes at room temperature until the cells had detached. To each flask, 6 ml growth medium was added and the cell suspension was transferred to a fresh 50 ml conical tube (BD Falcon, BD Biosciences, San Jose, CA). A 500 µL aliquot was removed and transferred to a Vi-cell sample cup for counting using the Vi-Cell™ Cell Viability Analyzer as described in **Example** 1. These cells were replated at 5,000 cells/cm² or 3,000 cells/cm².

hCTC (S) cell expansion was also performed using cryopreserved cells. A frozen vial of S cells was thawed at 37°C and was washed with PBS once. Then cells were counted and plated at 5,000 cells/cm² in growth medium in culture flasks. Cell expansion and confluency was observed each day.

By visual observation, non-adherent hCTC (S) cells were significantly reduced after 2 days in culture and the number of non-adherent hCTC (S) did not increase in culture over a period of 10-14 days. hCTC (S) cells in culture started attaching to flask after 2 days in culture and grew as adherent cells. They reached 80% confluency at 10-14 days after seeding of hCTC (S) cells. Although in culture some hCTC (S) cells were still observed, the number of this non-adherent population did not increase in culture. This was possibly due to the morphology change of non-adherent hCTC (S) to adherent hCTC (A2) in culture. Since the hCTC (S) cells attached to the flask after 2 days, the number of non-adherent cells became very low and was not counted.

In contrast, the adherent hCTC (A2) cells that were derived from hCTC (S) cells demonstrated some growth potential, up to 10 PDL shown in **Figure 5a****.** The growth rate of this population was between 1-3 PDL/passage until it reached plateau at 9-10 PDL, when the hCTC (A2) cells were seeded at either 5,000 cells/cm² or at 3,000 cells/cm². However, the PDL calculation is based on the initial cell number plated into the culture (i.e. the hCTC (S) cell number), the estimation of PDL may not be entirely accurate.

*Expansion of the hCTC (A1) population:* After the medium containing the hCTC (S) cells was removed from the flasks containing the cells from the initial two day plating, 60 ml fresh growth medium was added to the remaining adherent cells present in the flasks. The hCTC (A1) cells were cultured until the cells reached 80% confluency. After this time, the cells were trypsinized by aspirating off the growth medium, washed with 60 ml room temperature PBS and adding 4 ml Trypsin-EDTA (Invitrogen, Carlsbad, CA). Cells were incubated for approximately 5 minutes at room temperature until the cells had detached. To each flask, 6 ml growth medium was added and the cell suspension was transferred to a fresh 50 ml conical tube (BD Falcon, BD Biosciences, San Jose, CA). A 500 µL aliquot was removed and transferred to a Vi-cell sample cup for counting using a Vi-Cell™ Cell Viability Analyzer. A portion of the cells were then re-suspended with cryo-preservation medium (90% FBS and 10% DMSO) and saved at 1-1.5 million cells/ml and stored at -140°C. The remaining cells were expanded by replating the cells frozen vials at 3,000 cells/cm². The spent medium was replaced three days after replating and cells were passaged at day 7. These cells were passaged every 7 days with medium replacement at day 3, using trypsinization.

*Expansion of the hCTC (A3) population:* A vial of hCTC (A1) and a vial hCTC (S) cells was washed and then combined into a 50 ml conical tube (BD Falcon, BD Biosciences, San Jose, CA). Either a mixture of 5,000 cells/cm² or 3,000 cells/cm² of the combined cell suspension was added into separate T225 flasks. Each flask was filled with fresh growth medium to 60 ml per flask, and the cells incubated at 37°C, 20% atmospheric O₂, for 2 days. After this time, the majority of the cells formed an adherent cell population, referred to herein as the hCTC (A3) population, or hCTC (A3) cells. Once the cells had reached 80% confluency, the cells were passagaged by trypsinization and replating at seeding density of either 5,000 cells/cm² or 3,000 cells/cm² to identify the appropriate seeding density and incubated at 37°C, 20% atmosphere O₂. hCTC (A3) cells typically reached 80% confluency in 7 days after seeding. Non-adherent hCTC (S) cells were visually observed daily, and were significantly reduced after 2 days in culture, such that the hCTC (A3) cells became a homogeneous population of cells. On average, this took about 2 days. The hCTC (A3) population was capable of expanding of a rate of 1-3 PDL per passage. When hCTC (A3) cells were seeded at a density of 5,000 cells/cm², the hCTC (A3) were capable of reaching 9-10 PDL before reaching senescence. In contrast, when hCTC (A3) cells were seeded at a density of 3,000 cells/cm², the hCTC (A3) were capable of reaching 24 PDL before reaching senescence. See **Figure 5b****.**

*Characterization of the human cardiac tissue-derived cells of the present invention*: hCTC (A2) andhCTC (A3) cells demonstrated similar growth rate of 1-3 PDL at each passage. They both required about 7 days for cells to reach 80-90% confluency for passaging. The differences in the total PDL observed between the two cell populations may possibly be due to the initial underestimated PDL in hCTC (A2) cells when they were derived from hCTC (S) cell population.

There were no differences observed in the expression of cell surface makers or genes in all the populations of human cardiac tissue-derived cells isolated by the methods of the present invention. hCTC (A1), hCTC (S), hCTC (A2), and hCTC (A3) cells did not express telomerase. See **Table 1** for a list of genes expressed in the human cardiac tissue-derived cells of the present invention, and cardiac progenitor cells in the art. All the cell populations isolated according to the methods of the present invention demonstrated positive gene expression of GATA4 and Nkx2.5. No expression of myosin heavy chain was observed. The stem cell marker c-kit was detected by gene expression in all the cell populations of the present invention. See Table 8. By flow cytometry, the human cardiac tissue-derived cells of the present invention were positive for CD105 and CD90. The cells of the present invention did not express CD31, CD45 and CD16. See **Table 8.**

There were no significant differences observed in the characteristics of the populations of human cardiac tissue-derived cells of the present invention. The hCTC (A3) population was selected for further characterization.

### Example 3

### In Vitro Cell Culture of Human Cardiac Tissue-Derived Cells

Cell density and hypoxia have impact on cell growth (Tavaluc R et al, Cell Cycle 6:20, 2554-2562, 15 October 2007). Cell-cell contact can reduce cell growth potential and low seeding density reduced the opportunity for cell contact and enhances growth potential. Hypoxia, or low O₂ tension has been shown to reduce contact inhibition of cell growth (Nonomura Y. et al; The Journal of Rheumatology April 1, 2009 vol. 36 no. 4 698-705). In current invention, seeding density at 3,000 cells/cm² demonstrated more growth potential compared to 5,000 cells/cm².

To compare the effect of O₂ levels on cell growth, hCTC (A3) cells were seeded at 3,000 cells/ cm² after each passage in T225 flasks. The cells were incubated in an atmosphere of either 20% O₂ or 5% O₂. On day 3, the spent medium was replaced with 60 ml fresh growth medium. On day 7, hCTC (A3) cells were harvested according to the methods described in **Example 2.** The growth kinetics was determined by examining the accumulative total PDL until senescence was observed. The total duration of the experiment was greater than 100 days, during which, the cells were passaged 16-17 times. The hCTC (A3) cells cultured in normal oxygen conditions (20% O₂), the growth curve reached a plateau at PDL 24. However, when hCTC (A3) cells at PDL 12 were cultured in low oxygen conditions (5% O₂), the growth curve reached a plateau at PDL 28, as shown in **Figure 6****.**

### Example 4

### Isolation of Rat Cardiac Tissue-Derived Cells

A Sprague-Dawley rat at 8-12 weeks old was anesthetized by isofluorane, and the abdominal cavity was opened. The intestines were displaced and the aorta was severed. A 27-guage needle was inserted into the thoracic vena cava and the heart was perfused with 10 ml PBS, containing 5U/ml heparin. Retrograde perfusion of the heart was then performed by injecting 10 ml PBS, containing 5U/ml heparin through the thoracic aorta. Care was taken to ensure the heart remained beating throughout this procedure. The whole heart was then removed from the chest cavity, and placed in ice-cold Hank's buffer. Five isolated rat hearts were combined together for dissociation and enzymatic digestion.

The isolated rat hearts were then washed twice with 20ml room temperature PBS, and the supernatant discarded. The hearts were then manually minced with surgical scalpels at room temperature and the chopped tissue was transferred to three 50-ml tubes. The chopped tissue was then washed three times with 25 ml PBS and the tube was inverted five times.

The tissue pieces were transferred to separate 50 ml conical tubes (Corning Inc., Corning, NY). The tissue in each tube was washed three times by adding 30 ml room temperature PBS and inverting the tube five times. The tube was then placed upright and the tissue allowed to settle. The supernatant was aspirated using a 2 ml aspirating pipette (BD falcon, BD Biosciences, San Jose, CA). The digestion enzyme cocktail stock (2X) was added to the 50 ml tube at an enzyme to tissue ratio of 1:1. The final concentration of the mixed enzymes was 1U/ml Collagenase and 5U/ml Dispase II. The tubes containing the tissue and enzymes were transferred to a 37°C orbital shaker set for 225 rpm (Barnstead Lab, Melrose Park, IL) and incubated for 2.5 hours. After incubation, the tube was transferred back to the biosafety cabinet. The cell suspension was diluted by filling the tubes with room temperature PBS. In order to remove any remaining undigested tissue, the cell suspension was filtered through an 8-inch diameter 100 µm cell strainer (BD Falcon), and then a 40 µm cell strainer (BD Falcon) and into six 50 ml conical tubes (BD Falcon). The filter size for rat CTC was smaller than the ones used for human cells because of the myocyte size difference between rat and human. The cell suspension was then washed by centrifuging at 338 *x* g for 5 minutes at room temperature using a Sorvall Legend T centrifuge (Thermo Fisher Scientific, Inc, Waltham, MA) to pellet the cells. The supernatant was aspirated off and the cell pellets resuspended in growth medium and pooled into one 50 ml tube in 20 ml growth medium, and a sample removed to determine cell yield. Typical yields obtained were 10 million cells per heart, with a viability of 70%.

In the preparation of rat cardiac tissue-derived cells, either 0.1U/ml or 1U/ml collagenase stocks were used to digest the cardiac tissue. After the 3-hour incubation, 20 ml growth medium was added to each of the tubes, as described in **Example** 1. However, rat cardiac tissue digested with 0.1 U/ml collagenase did not yield any cells.

The cell suspension obtained from the dissociation and enzymatic digestion of the cardiac tissue was seeded into T225 tissue culture flasks (Corning Inc., Corning, NY), by transferring 10 ml into each flask. To each flask, 35 ml growth medium (DMEM, 1,000 mg/L D-glucose, 584 mg/L L-glutamine, and 110 mg/L sodium pyruvate, 10% fetal bovine serum, Penicillin 50U/ml, Streptomycin 50 µg/ml, Invitrogen, Calsbald, CA) was added, bringing the final volume inside each flask to 45ml. The initial cell culture was for two days at 37°C in an atmosphere of 20% O₂ and 5% CO₂. After the initial two day culture, the non-adherent rCTC (S) cells were removed and transferred into 50 ml conical tubes, and centrifuged at 338 x g for 5 minutes at room temperature. The supernatant is discarded and the cell pellet re-suspended in 20 ml growth medium. The cells were counted and reseeded into T225 flasks at a seeding density of 5,000 cells/cm². The rCTC (S) cells were cultured in growth medium. After an additional two days in culture, it was noted that the rCTC (s) cells became adherent. The adherent cell population that formed from the rCTC (S) cells that became adherent was refereed to as the rCTC (A2) population of cells, or rCTC (A2) cells.

rCTC (A2) cells were harvested and passaged at day 7 by trypsinization, according to the methods described in **Example 2.** rCTC (A2) cells were plated at a density of 5,000 cells/cm², in T225 flasks, with 45 ml growth medium in each flask. Cells were passaged when the cells reached approximately 80%. The growth curve ofrCTC (A2) cells observed is shown in **Figure** 7.

### Example 5

### Isolation of GFP Expressing Mouse Cardiac Tissue-Derived Cells

Five FVB.Cg-Tg(ACTB-EGFP)B5Nagy/J mice (GFP mice, Jackson Lab, Bar Harbor, Maine) at 8-12 weeks old were anesthetized by isofluorane, and the abdominal cavity was opened. The intestines were displaced and the aorta was severed. A 27-guage needle was inserted into the thoracic vena cava and the heart was perfused with 10 ml PBS, containing 5U/ml heparin. Retrograde perfusion of the heart was then performed by injecting 10 ml PBS, containing 5U/ml heparin through the thoracic aorta. Care was taken to ensure the heart remained beating throughout this procedure. The whole heart was then removed from the chest cavity, and placed in ice-cold Hank's buffer.

Five isolated GFP mouse hearts were combined for dissociation and enzymatic digestion. The isolated mouse hearts were then washed twice with 20 ml room temperature PBS, and the supernatant discarded. The hearts were then manually minced with surgical scalpels at room temperature and the chopped tissue was transferred to three 50-ml tubes. The chopped tissue was then washed three times with 25 ml PBS and inverting the tube five times. The tissue pieces were transferred to separate 50-ml conical tubes (Corning Inc., Corning, NY). The tissue in each tube was washed three times by adding 30 ml room temperature PBS and inverting the tube five times. The tube was then placed upright and the tissue allowed to settle. The supernatant was aspirated using a 2 ml aspirating pipette (BD falcon, BD Biosciences, San Jose, CA). The digestion enzyme cocktail stock (2X) was added to the 50 ml tube at an enzyme to tissue ratio of 1:1. The final concentration of the mixed enzymes was 1U/ml Collagenase and 5U/ml Dispase II. The tubes containing the tissue and enzymes were transferred to a 37°C orbital shaker set for 225 rpm (Barnstead Lab, Melrose Park, IL) and incubated for 2.5 hours. After incubation, the tube was transferred back to the biosafety cabinet. The cell suspension was diluted by filling the tubes with room temperature PBS. In order to remove any remaining undigested tissue, the cell suspension was filtered through an 8-inch diameter 100 µm cell strainer (BD Falcon), and then a 40 µm cell strainer (BD Falcon) and into 6 50-ml conical tubes (BD Falcon). The filter size for rat CTC was smaller than the ones used for human cells because of the myocyte size difference between rat and human. The cell suspension was then washed by centrifuging at 338 *x* g for 5 minutes at room temperature using a Sorvall Legend T centrifuge (Thermo Fisher Scientific, Inc, Waltham, MA) to pellet the cells. The supernatant was aspirated off and the cell pellets resuspended in growth medium and pooled into one 50 ml tube in 20 ml growth medium, and a sample removed to determine cell yield. Typical yields obtained were 10 million cells per heart, with a viability of 70%.

After the 3-hour incubation, 20 ml growth medium as described in **Example** was added to each of the tubes. In order to remove any remaining undigested tissue, the cell suspension was filtered through an 8-inch diameter 100 µm cell strainer (BD Falcon), and then a 40 µm cell strainer (BD Falcon) and into six 50 ml conical tubes (BD Falcon). The cell suspension was washed by centrifuging at 338 *x* g for 5 minutes at room temperature using a Sorvall Legend T centrifuge (Thermo Fisher Scientific, Inc, Waltham, MA) to pellet the cells. The supernatant was aspirated off and the cell pellets resuspended in growth medium and pooled into one 50 ml tube in 20 ml growth medium, and a sample removed to determine cell yield. Typical yields obtained were 10 million cells per heart with a viability of 70%, based on 2 isolations The mCTC (A2) population was used in subsequent studies. The cells were expanded for two passages prior to study.

### Example 6

### Cell Cryopreservation, Viability and Recovery

Rat and human cardiac tissue-derived cells of the present invention were prepared for cryopreservation. Briefly, cells from either the hCTC (A3), or the rCTC (A2) populations were obtained by expanding cryopreserved hCTC (A3) and rCTC (A2) cells at earlier passages. Cells were seeded at 3,000 cells/ cm², incubated at 37°C, under 20% atmospheric O₂, and passaged 7 days after in culture with medium replacement at day 3 in culture. They were collected at 12-14 PDLs.

Cells were trypsinized and resuspended for cryopreservation in CRYOSTOR D-LITE™ (Biolife Solutions, Inc, Bothell, WA), containing 2% DMSO was cryopreserved in Nalgene 2 mL Polypropylene, Sterile, Internal Thread with Screw Cap Cryovials (Nalgne Nunc, Rochester, NY), using a Integra 750 Plus programmable freezer (Planer, Middlesex, U.K.) with DeltaT software. Cell and solutions were at room temperature prior to loading into the programmable freezer, which was held at 15°C. A sample temperature probe was placed in a vial of freezing buffer. The following program was used for cryopreservation:

| Step No. | Rate (°C/min) | End Temp (°C) | Trigger |
|---|---|---|---|
| 1 | -1 | -6 | Sample |
| 2 | -25 | -65 | Chamber |
| 3 | +10 | -19 | Chamber |
| 4 | +2.16 | -14 | Chamber |
| 5 | -1 | -100 | Chamber |
| 6 | -10 | -140 | Chamber |

When the temperature reached -140 °C, samples were transferred to liquid nitrogen tank for storage.

*Viability and recovery of the human cardiac tissue-derived cells of the present invention following cryopreservation:* After a one-month storage in liquid nitrogen tank (-140 °C), one vial of hCTC (A3) cells in CRYOSTOR D-LITE™ at 1 million cells/vial was thawed at room temperature. The vial was then transferred to the biosafety cabinet. A 50µL (containing 0.5 million cells) sample was transferred to a 1.8mL microfuge tube containing 50µL of trypan blue solution. Duplicate counts were taken from this cell preparation by transferring 10µL to a hemacyometer and counted. These counts determined the to pre-needle recovery and viability. To determine cell viability and recovery post-needle passage without room temperature incubation (to post-needle), 100µL of cell suspension was drawn into a 1 mL tuberculin syringe (BD cat# 309602) through a 30 gauge needle (BD cat# 305106). The sample was then passed through the needle again and into a 1.8 mL microfuge tube. To this tube, 100µL of trypan blue was added and duplicate counts were performed as described above. This procedure was performed after incubation times of 10 min, 20 min, 30 min at room temperature, and also at 30 minutes with no passage through the needle.

After one-month storage, hCTC (A3) cell viability was determined to be 94% following thawing. The recovery of cells was 0.54 million, similar to the original cell number before cryopreservation as shown in **Table 3** and **Figure 8****.**

The viability of the human cardiac tissue-derived cells tested after passing through a 30 gauge needle administration needle was above 90% after 30 minutes incubation at room temperature, which is the required time for cell administration during rat infarction procedure. The recovery was similar to the cell number prior to needle passage, as shown in **Table 3** and **Figure 8****.**

hCTC (A3) cells were expanded to PDL 12 and were banked for future *in vivo* studies. Samples of the banked cells were examined for any karyotype abnormality. The results are summarized in **Table 4.**

*Rat CTC Biocompatibility*: One vial of rCTC (A2) cells in CRYOSTOR D-LITE™ at 2 million cells/vial was thawed as described above. The vial was then transferred to the biosafety cabinet. A 50µL sample was transferred to a 1.8mL microfuge tube containing 50µL of trypan blue solution. Triplicate counts were taken from this cell preparation by transferring 10µL to a hemacyometer and counted. To determine the baseline for post-needle cell yield and viability, the cell counts were done both prior to needle passage and post needle passage, with incubation time of 0, 10, 20, 30 mins. At each time point, 100µL of cell suspension was drawn into a 1 mL tuberculin syringe (BD cat# 309602) through a 30 gauge needle (BD cat# 305106). The sample was then passed through the needle again and into a 1.8 mL microfuge tube. To this tube, 100µL of trypan blue was added and triplicate counts were performed as described above. This procedure was performed after incubation times of 10 min, 20 min, and 30 min at room temperature to simulate the potential procedure of cell administration in the rat acute myocardial infarction model.

After one-month storage in liquid nitrogen, rCTC (A2) cell viability following thawing was 94%. The recovery of cells was 1.4 million/ml, about 70% of the original cell concentration (2 million/ml). The viability of rCTC (A2) cells after passing through injection needle was above 90% after 30 minutes incubation at room temperature, which is the required time frame for injection during rat infarction procedure. The recovery was similar to prior to needle passage, as shown in **Table 5 and** **Figure 9****.**

### Example 7

### Characterization of the Cardiac Tissue-Derived Cells of the Present Invention

The expression of cell surface proteins was determined on populations of cardiac tissue-derived cells, obtained by the methods of the present invention from rat and human cardiac tissue. The cell surface markers tested are shown in **Table 6.** Populations of human dermal fibroblasts were included as a control.

Greater than 90% of the population of hCTC (A3) cells expressed CD59, CD105, CD54 and CD90 (analysed separately). Approximately 30% of the population of hCTC (A3) cells expressed CD34, a stem cell marker for endothelial progenitor cells. Also, about 30% hCTC (A3) showed positivity for c-Kit. In contrast, less than 5% of the population of hCTC (A3) cells expressed either CD31, CD45 or CD16. See **Figures 10****,** **11** and **Table 7.** Populations of hCTC (A1), hCTC (A1) andhCTC (S) cells also showed similar cell surface marker expression. See **Table 8.** Furthermore, similar results were observed a population of rCTC (A3) cells. See **Figure 12****.** CD54, Intercellular Adhesion Molecule-1 (ICAM), binds to integrins on leukocytes and mediate the transmigration of leukocytes through vascular barrier into tissues (Yang L et al, Blood 106 (2): 584-92, July, 2005). Thus, cell surface expression of this molecule may facilitate the translocation of hCTC (A3) from the vasculature into myocardium, when administered into coronary artery.

### Example 8

### Gene Expression Analysis of Cardiac Tissue-Derived Cells

The RNA samples from the following cardiac tissue -derived cell populations were collected: hCTC (A1), hCTC (A2), hCTC (A3), rCTC (A2), and mCTC (A2) (RNA was collected from one million cells of each cell population).

The expression of a panel of genes was determined via real-time PCR in the samples collected. The real-time PCR reaction was initiated according to the reaction mix defined in **Table 9,** and the primers for the genes tested are shown in **Table 10.** Two categories of genes were examined: cardiac -specific genes and stem cell genes. Cardiac specific genes were further separated into differentiated markers such as myosin heavy chain (MyHC) and undifferentiated cardiac markers such as GATA-4 and Nkx2.5. The stem cell genes were further categorized as the stem cell marker, c-kit; embryonic cardiac marker islet-1, and cell division marker, telomerase. A housekeeping gene-Glyceraldehyde 3-phosphate dehydrogenase (GAPDH) was used as benchmark to normalize the expression levels in each sample.

The expression of the genes tested was found to be similar in the hCTC (A1), hCTC (A2), and hCTC (A3) populations. See **Table 10.** The stem cell marker c-kit was expressed (Ct: 27-29), while the expression of telomerase and islet-1 was undetectable in the hCTC (A1), hCTC (A2), andhCTC (A3) populations: No message for the genes was detected at a Ct value of 40. The cardiac markers GATA 4 and Nkx2.5 were expressed at a CT value of 25 and 32-34 respectively, in the hCTC (A1), hCTC (A2), and hCTC (A3) populations, while neither myosin heavy chain, or cardiac actin expression was observed in any of the hCTC (A1), hCTC (A2), and hCTC (A3) populations. See **Table 10.**

These data suggest that the cardiac tissue-derived cells of the present invention are "progenitor-like", namely that the ratio of progenitor cell marker v. differentiated cell marker expression was greater than 50,000 in the hCTC (A1), hCTC (A2), and hCTC (A3) populations, compared to cardio myocytes (1%) and human fibroblast cells (12%). See **Table 10, Table 11, and** **Figure 13****.**

rCTC (A2) cells also expressed cardiac lineage genes such as GATA-4 (Ct: 28) and Nkx2.5 (Ct: 27). However, unlike human cardiac tissue-derived cells, the expression of Nkx2.5 was at a higher level in rat cardiac tissue-derived cells than that observed in human cardiac tissue-derived cells. The markers c-kit, Islet-1 and telomerase were also expressed in rCTC (A2) cells. See **Table 12.** Similar cardiac tissue-derived cells obtained from rat heart, mouse cardiac tissue derived cells also expressed Nkx2.5, c-kit, Islet-1 and telomerase. See **Table 13.**

### Example 9

### Cardiac Tissue-Derived Cells can Differentiate into Cardiomyocytes

mCTC (A2) cells (200K) obtained according to the methods described in **Example 5** were first cultured in growth medium for 2 days and then were collected by trypsinization and counted before being mixed with rat cardiac myocytes (1 million, Cat # R357, Cell Application, Inc. Austin TX), at a ratio of 1:5. Rat cardiac myocytes were in culture for 5 days before being trypsinized and counted, then mixed with mCTC (A2) cells. The mixture of cells was plated on to a laminin-treated 6-well plate (Cat # 354595 BD Biosciences, NJ) for 5 days. The ability of the mCTC (A2) cells to differentiate was tested by incubating the mixture of cells in tissue culture medium comprising DMEM-F12(1: 1) +10% horse serum (Sigma), hereinafter referred to as differentiation medium. The cells were incubated in differentiation medium for 5 days in an atmosphere of 20% O₂, at 37°C. After this time, cells were harvested, and RNA extracted. Total RNA from co-cultures of mCTC (A2) cells and rat cardiomyocytes and from parallel cultures of mCTC (A2) cells was tested for the gene expression of murine myosin heavy chain. The following murine myosin heavy chain primers were used:

| Type | Name | Sequence |
|---|---|---|
| Forward Primer | MHC-mouse-F | GAAACACCTGAAGAATTCTCAAGCT |
| Reverse Primer | MHC-mouse-R | TTGGCATGGACAGCATCATC |
| Probe | MHC-mouse-P | ACTTGAAGGACACCCAGC |

The co-culture of mCTC (A2) cells with rat cardiomyocytes resulted in the 9-fold increase in expression of murine myosin heavy chain, compared to parallel cultures of mCTC (A2) cells alone. See **Figure 14****.** These data suggest that the cardiac-derived cells of the present invention are capable of differentiating into cardiomyocytes, and co-culturing the cells of the present invention with cardiomyocytes may enhance the differentiation.

### Example 10

### Isolation, Expansion and Characterization of Porcine Cardiac Tissue Derived Cells

A single heart from a Göttingen mini swine at 8-12 weeks of age was obtained at each isolation from Marshall Bioresources (North Rose, NY). The heart was perfused to deplete blood prior to collection and the whole organ was emerged in DMEM + 10% FBS on ice during shipment. The time from procurement to tissue digestion was between 48-96 hours. Four separate isolations were performed according to the procedures described below.

The hearts were cut into small pieces (approximately 2 to 3 cm³ in size). These tissue pieces were homogenized via mechanical homogenization, as described in **Example 1** to yield heart tissue fragments of less than 1mm³ in size, and then were transferred to one 250 ml conical tube (Corning Inc., Corning, NY) and washed three times. The digestion enzyme cocktail stock (2X) was added to the 250 ml tube at an enzyme to tissue ratio of 1:1. The final concentration of the enzymes was 1U/ml Collagenase and 5U/ml Dispase II. The tubes containing the tissue and enzymes were transferred to a 37°C orbital shaker set for 225 rpm (Barnstead Lab, Melrose Park, IL) and incubated for 2.5 hours. After incubation, in order to remove any remaining undigested tissue, the cell suspension was filtered through an 8-inch diameter 250 µm standard testing sieve to eliminate the undigested connective tissue and adipose tissue (Sigma-Aldrich, St. Louis, MO) and then further filtered through 100 µm cell strainers to eliminate cardiomyocytes (BD Falcon). The medium, containing the cells that passed through the filter was transferred into multiple 50-ml conical tubes (BD Falcon). The cell suspension was then washed. After washing, the pellet was resuspended in 20 ml ACK lysing buffer (Lonza, Walkersville, MD) and incubated for 10 minutes at room temperature to lyse any remaining red blood cells. After incubation the cell suspension was washed two more times with 40 ml room temperature PBS. Following the final centrifugation, the pellet was resuspended in 20 ml room temperature growth medium and counted. After dissociation and enzymatic digestion, the yield of cells was typically 27 million cells, in a volume of 20 ml. The viability was typically 80%.

The cell suspension obtained from the dissociation and enzymatic digestion was added to T225 tissue culture flasks (Corning Inc., Corning, NY) flasks. 10 ml of the cell suspension was added to each flask, which contained 50 ml growth medium (DMEM, 1,000 mg/L D-glucose, 584 mg/L L-glutamine, and 110 mg/L sodium pyruvate, 10% fetal bovine serum, Penicillin 50U/ml, Streptomycin 50 µg/ml, Invitrogen, Calsbald, CA). The final volume of the initial culture was 60 ml. The cells were incubated at 37°C in an atmosphere comprising 20% O₂ and 5% CO₂, for 2 days. After this time, a heterogeneous cell culture was observed. Non-adherent, phase bright cells were observed (referred to herein as pCTC (S) cells), and adherent cells were observed (referred to herein as pCTC (A1) cells).

Dissociation and enzymatic digestion of porcine heart according to the methods of the present invention, and subsequent expansion of the cells resulted in the following cell populations: pCTC (S), pCTC (A1), pCTC (A2), and pCTC (A3) cells. The morphology of the porcine cardiac tissue-derived cells of the present invention was similar to the human cardiac tissue-derived cells of the present invention. The pCTC (A3) population was selected for further characterization and subsequent *in vivo* studies.

The pCTC (S) cells and pCTC (A1) cells were initially expanded in culture as a mixture in T225 flasks. Each flask was filled with fresh growth medium to 60 ml per flask, and the cells incubated at 37°C, 20% O₂, for 2 days. After this time, the majority of the cells formed an adherent cell population, referred to herein as the pCTC (A3) population, or pCTC (A3) cells. After 2 days in culture, by visual observation, the number of non-adherent cells declined, such that the pCTC (A3) cells became a homogeneous population of cells. On average, this took 2 days. pCTC (A3) cells were passaged once the cells reached 90-100% confluency, and were re-seeded at 3000 cells/cm². The expansion of pCTC (A3) cells in culture exceeded the growth of the human cardiac tissue-derived cells. pCTC (A3) cells grew to above 90% confluence in 3-4 days. See **Figure 15** for the growth curve observed for the pCTC (A3) cells of the present invention.

pCTC (A3) cells did not express telomerase or myosin heavy chain, as determined by real-time PCR. However, pCTC (A3) cells expressed GATA-4. The expression of Nkx2.5 was not examined in the porcine cardiac tissue-derived cells of the present invention. Single staining of cell surface markers demonstrated that greater than 90% of the population of pCTC (A3) cells was positive for the expression of CD10⁵ and CD90. Less than 5% of the pCTC (A3) cells expressed either CD45, CD16, or porcine endothelial cell marker (Cat # MCA1752, Serotec). This marker is a histocompatibility complex class II molecule, which has been identified on capillary endothelium in a wide range of tissues, shown by Wilson et al (Immunology. 1996 May; 88(1):98-103). See **Figure 16** and **Table 14.** Other cell populations such as pCTC (A1), or pCTC (A2) cells were not examined.

### Example 11

### Treatment of Acute Myocardial Infarction with the Cardiac Tissue-Derived Cells of the Present Invention

*Rat Acute Myocardial Infarction Model:* The rat myocardial infarction model has been used successfully to test the efficacy of agents such as ACEI and beta-blockers as therapies for human AMI. At all stages of the experiment, the animals were treated in accordance with local institutional guidelines.

The rat myocardial infarction model is well established to simulate human pathophysiology post myocardial infarction and further deterioration of the cardiac function post infarction (Pfeffer M. A. et al, Circ Res 1979; 44: 503-12; Litwin S. E. et al, Circulation 1994; 89: 345-54; Hodsman G. P. et al Circulation 1988; 78: 376-81).

Female nude rats (weight, 250 to 300 g; Shizuoka Agricultural Cooperation Association, Shizuoka, Japan) were anesthetized with ketamine and xylazine (60 and 10 mg/kg IP, respectively), and positive-pressure respiration was applied through an endotracheal tube. The thorax was opened at the fourth left intercostal space, the heart was exteriorized, and the pericardium was incised. Thereafter, the heart was held with forceps, and a 6-0 Proline suture was looped under the left anterior descending coronary artery, approximately 2 mm from its origin. AMI was induced in the heart by pulling the ligature, occluding the artery permanently. Discoloration of the infracted myocardium was visually observed. A suspension of cardiac tissue-derived cells, or the vehicle was injected at the border zone of the discolored area about 20 mins after infarction was induced, as described below in *cell administration.* After the injection, the thorax was closed, and the rats were returned to their cages. At each specified time after surgery, the rats were sacrificed by excision of the heart under anesthesia.

Crypreserved populations of hCTC (A3) and rCTC (A2) cells that were stored at -80°C were thawed on ice, and their viability determined prior to administration to the test animals. Cell viability was above 95% in all cell populations employed in this investigation.

Cells were administered to test animals 20 minutes after the ligation of the left anterior descending coronary artery. Crypreserved populations of hCTC (A3) cells were injected at the border zone of the discolored area. Test animals received one of the following target doses in 120 µl Cryostor D-lite (15 animals per target dose): 1x10⁴ cells (low dose), 1x10⁵ cells (mid dose), or 1x10⁶ cells (high dose). In parallel, crypreserved populations of rCTC (A2) cells were injected at the border zone of the discolored area. Test animals received one of the following target doses in 120 µl Cryostor D-lite (15 animals per target dose): 1x10⁶ cells.

In all test animals, the cryopreserved cells were in a total volume of 120µl of cryopreservation medium. The cells of one target dose were injected into five separate sites around the discolored area of the heart. A control group, receiving an injection of cryopreservation medium (120µl) was also included in the study.

Transthoracic echocardiography (SONOS 5500, Philips Medical Systems) was performed to evaluate left ventricle (LV) function at 5 and 28 days after the induction of AMI. Rats were anesthetized with ketamine and xylazine while echocardiography was performed. LV end-diastolic and end-systolic dimensions (LVEDD and LVESD, respectively) and fractional shortening (FS) were measured at the mid-papillary muscle level. FS reflects the pumping effect of the heart by measuring the percent difference between systolic diameter (end of contraction) and diastolic diameter (end of filling). Regional wall motion score (RWMS) was evaluated per published criteria: Score 1: normal wall motion and thickening; Score 2: reduced wall motion and thickening; Score 3: absence of wall motion and thickening; Score 4: outward motion or bulging. (See for example, Schiller, Shah et al. (Journal of American Society of Echocardiography vol 2: 358-367; 1989)).

Briefly, seventeen serial sectional images were obtained from echocardiogram and each section was given a wall motion score based on the definitions in **Table 15.** A sum of the score of all 17 segments was used as the indication of wall contractility. RWMS is a direct measurement of contraction. A reduction in RWMS indicates an improvement in contraction and reflects improved function of the cardiac muscle. **Table 15** describes the criteria for each score.

The observed mortality rate was 16% in the study. There was no significant difference for mortality between groups as shown in **Table 16.**

### Results

**Figure 17** is reproduced from the Atlas of Heart Failure by Pffeffer *et al* (1999), demonstrates the pathological changes observed in the heart, following infarction. The rat acute myocardial infarction model has been established to simulate AMI and chronic heart failure in human patients. After infarction, as in human, the ventricle undergoes series of pathophysiological alterations, starting with the replacement of myocardium with fibrotic tissue at the infarct area. The contraction and the pressure in the ventricle causes the extension of the infarct, gradually the expansion of the ventricular chamber, and ultimately results in remodeling of the left ventricle, demonstrated by the geometry change of the chamber from elliptical to globular, and cellular changes as myocyte hypertrophy.

Cryopreserved populations of hCTC (A3) cells improved global cardiac function and cardiac contractility, as measured by fractional shortening (FS) and regional wall motion score (RWMS) respectively. Improvements in global cardiac function and cardiac contractility were observed at all target doses of hCTC (A3) cells. See **Figures 18** and 19.

At five days post administration, animals dosed with rCTC (A2) cells, or the target dose of 1x 10⁶ hCTC (A3) cells demonstrated a FS of 3.3% (hCTC (A3)) and 3.8% (rCTC (A2)) less than vehicle treated animals. At four weeks post cell administration, the absolute value of fractional shortening (calculated by subtracting the FS value observed at day five from the FS value observed at day 28) was improved. See **Figure 18** and **Table 17** and **18.** The absolute value of FS in animals treated with 1 x 10⁴ hCTC (A3) cells was 9.687 ± 1.329% (n= 12, P less than 0.001). The absolute value of FS in animals treated with 1 x 10⁵ hCTC (A3) cells was 10.9 ± 1.6% (n=11, P<0.001). The absolute value of FS in animals treated with 1 x 10⁶ hCTC (A3) cells was 12.9 ± 1.8% (n=10, P less than 0.001). Several possible reasons may explain the inefficacy of rCTC cells in the experimental model used in the present invention. Although nude rats are immune-compromised, their rejection to foreign cells was not completely eliminated. In the current study, rCTC may be more susceptible to immune rejection by nude rats than human cells. Their retention in the myocardium compromised because of immune rejection, and thus their effect on myocardium can be affected.

A reduction of RWMS was also observed in animals treated with hCTC (A3) cells, four weeks post cell administration. The RWMS score in animals treated with 1 x 10⁴ hCTC (A3) cells was 24.42 ± 1.4 at 5 days post infarction and cell administration, but was reduced to 21.08 ± 1.7 (n= 12, P less than 0.001) at 4 weeks after infarction and cell administration. The RWMS score in animals treated with 1 x 10⁵ hCTC (A3) cells was 25.58 ± 1.4 at 5 days post infarction and cell administration, and was reduced to 21.08 ± 1.9 (n=11, P less than 0.001). The RWMS score in animals treated with 1 x 10⁶ hCTC (A3) cells was 25.91 ± 1.6 at 5 days post infarction and cell administration, and was reduced to 20 ± 1.7 (n=10, P less than 0.001) at 4 weeks after infarction and cell administration. While rCTC (A2) treatment did not appear to reduce fractional shortening, a slight reduction of RWMS was observed. The RWMS score in animals treated with 1 x 10⁶ rCTC (A2) cells was 25.29 ± 1.9 at 5 days post infarction and cell administration, and was reduced to 23.86 ± 2.3 (n=12, P=0.09) at four weeks post cell administration. See **Figure 19** and **Table 17** and **19.** The data observed in rCTC (A2) treated animals suggest that while rCTC (A2) cells did not improve global function, they improved cardiac contractility, as shown by RWMS.

On the other hand, the data observed in animals treated with hCTC (A3) cells suggest that human cardiac tissue-derived cells improved global cardiac function and cardiac contractility.

Cardiac remodeling was also prevented in animals receiving hCTC (A3) cells. Cardiac remodeling refers to the changes in size, shape, and function of the heart that are observed after ischemic injuries, such as, for example, myocardial infarction. The changes observed include myocardial cell death and a disproportionate thinning of the chamber wall at the infarct zone. The thin chamber wall is unable to withstand the pressure and volume load on the heart. As a result there is dilatation of the chamber arising from the infarct region, spreading to the compensating non-infarcted cardiac muscle. Over time, as the heart undergoes ongoing dilatation, the ventricle enlarges in size, and becomes less elliptical and more spherical in shape as demonstrated by increased dimension in echocardiography. The increases in ventricular mass and volume adversely affect cardiac function even further. The increased volume at the end of diastole eventually impairs with the heart's ability to relax between contractions, resulting in a further decline in function. The severity of the enlargement of the ventricle determines the prognosis of patients. The enlargement of the chamber correlated with shortened life expectancy in heart failure patients.

The degree of cardiac remodeling in the left ventricle of animals following induction of an acute myocardial infarction was determined by measuring the dimension of left ventricle at the end of diastole (left ventricle end diastolic dimension, LVEDD) and systole (left ventricle end systolic dimension, LVESD) via echocardiography. An increase of LVEDD and LVESD denotes an increase in the severity of cardiac remodeling. Conversely, a reduction in observed values of LVEDD and LVESD denoted a reversal of cardiac remodeling, or an improvement in cardiac function.

In vehicle treated animals, LVEDD increased from 0.74±0.020 mm at five days post cell administration to 0.83±0.019 mm at 4 weeks post cell administration. This corresponded to a 12% relative increase [100%(D28-D5)/D5] in the left ventricle. In animals treated with rCTC (A2) cells, LVEDD increased from 0.69±0.022 mm at five days post cell administration to 0.80±0.018 mm at 4 weeks post cell administration. In animals treated with 1 x 10⁴ hCTC (A3) cells, LVEDD increased from 0.70±0.012 mm at five days post cell administration to 0.77±0.022 mm at 4 weeks post cell administration.

In animals treated with 1 x 10⁵ hCTC (A3) cells, LVEDD did not appear to change significantly, wherein LVEDD was 0.73±0.012 mm at five days post cell administration, and 0.74±0.023 mm at 4 weeks post cell administration, a relative change of 1.4% (p less than 0.01, compared to vehicle group). Similarly, animals treated with 1 x 10⁶ hCTC (A3) cells, LVEDD also did not appear to change significantly, wherein LVEDD was 0.76±0.011 mm at five days post cell administration, and 0.71±0.028 mm at 4 weeks post cell administration a relative change of 6.6% reduced from five days after cell administration (p less than 0.001, compared to vehicle group). These data suggest that the 1 x 10⁵ hCTC (A3) dose and the 1 x 10⁶ hCTC (A3) dose prevented cardiac remodeling. See **Figure 23****, Table 17** and **Table 20.** The relative change in LVEDD (100%(28D-5D)/5D) is shown in **Table 21** and **Figures 20-21****.**

In animals treated with 1 x 10⁴ hCTC (A3) cells, LVEDD was 0.71±0.045 mm at day 5 and 0.78±0.079 mm at day 28. In animals treated with 1 x 10⁶ rCTC (A2) cells, LVEDD was 0.70±0.083 mm at day 5 and 0.80±0.071 mm at day 28. There was no significant difference from vehicle-treated animals in LVEDD, suggesting no improvement in remodeling compared to vehicle group. See **Table 17** and **Figure 23****.**

Animals treated with human cardiac tissue-derived cells also demonstrated a reduction in left ventricle end systolic dimension (LVESD). LVESD measures the size of the ventricle at the end of contraction. This parameter not only represents remodeling but also indicates contractility of the cardiac muscle. A reduction in LVESD corresponds to an increase in the strength of contraction.

LVESD was increased from day 5 to day 28 in vehicle treated animals. LVESD was maintained at the same level in animals treated with 1 x 10⁴ hCTC (A3) cells (0.56±0.05cm at day 5 and 0.54±0.08cm at day 28). LVESD was reduced in animals treated with 1 x 10⁵ (0.58±0.04cm at day 5 and 0.51±0.08cm at day 28) and 1 x 10⁶ hCTC (A3) cells 0.62±0.05cm at day 5 and 0.48±0.09cm at day 28). See **Figure 22** and **Table 22.** Functional data by all four parameters measured by echocardiography from each animal at each time points were shown in **Figure 23****.** The trend changes between day 5 and day 28 are consistent within each group.

Analysis of the targeted dose of hCTC (A3) cell administration and cardiac function, as determined by global function measured by fractional shortening (FS) demonstrated a correlation (p=0.001, n=35) between cell dose and functional improvement. See **Figure 24****.**

Similarly, analysis of the targeted dose of hCTC (A3) cell administration and cardiac remodeling, as determined by the absolute change of LVEDD from day 5 to day 28 (28D-5D) was observed (p=0.0002, n=35). See **Figure 25****.** A correlation was established and it appears to be exponential, instead of linear.

### Example 12

### Retention of Human Cardiac Tissue-Derived Cells in Rat Model of Acute Myocardial Infarction

To further understand the mechanisms and the biological benefits of human cardiac tissue-derived cells as a therapy for damaged myocardium, tissue samples were taken from the hearts of the animals treated with human cardiac cells in the previous example, to determine the retention of human cardiac tissue-derived cells in animals four weeks post administration.

Hearts were removed from the animals treated with human cardiac tissue-derived cells in the previous example were collected a four weeks post cell administration. Cell retention was determined by histology (n=6 per cell dose), and quantitative real-time PCR (n=4 per cell dose).

To establish base-line cell retention values, hCTC (A3) cell were administered to test animals 20 minutes after the ligation of the left anterior descending coronary artery. Crypreserved populations of hCTC (A3) cells were injected at the border zone of the discolored area in five separate injections sites per animal. Animals were administered target doses of either 1 x 10⁴, 1 x 10⁵, or 1 x 10⁶ cells. Animals were sacrificed at 0, 1, 3 and 7 days, and the hearts removed for cell retention analysis by quantitative real-time PCR (n=3 per treatment group).

In the cases where samples were taken for quantitative real-time PCR, heart tissues were processed to obtain total RNA. Retention of human cells was estimated, based on the amount of human RNA detected in the heart samples.

RNA from human cardiac tissue-derived cells was detected at 4 weeks post cell administration in animals treated with hCTC (A3) cells. The cell retention appeared to be dose-dependent, with animals receiving 1 x 10⁶ hCTC (A3) cells demonstrating more cell retention than animals receiving 1 x 10⁵ hCTC (A3) cells. In animals receiving 1 x 10⁴ hCTC (A3) cells, cell retention was estimated to be at background levels, based on the amount of human RNA detected in the samples.

Human RNA was detected in hearts from animals sacrificed at 0, 1 day, 3 days, and 7 days post cell administration. Cell retention dropped rapidly immediately after administration, and declined still further at 24 hours post cell administration. As shown in **Figure 26****, panels c** and **d,** immediately after cell administration, only about 8% of the target dose remained, as estimated by the amount of human RNA detected in the heart samples. Using the 0 time point as the baseline, the level of cell retention declined still further, when determined at 24 hours post cell administration, to approximately 10% of the 0 time point. The level of cell retention remained at the same level at day 7 post cell administration.

A correlation was observed between human cardiac tissue-derived cell retention and the prevention of cardiac remodeling. In animals receiving human cardiac tissue-derived cells, the change in LVEDD (D28-D5) correlated with the retention of human cardiac tissue-derived cells. As can be seen in **Figure 27**, the trend of the correlation is significant with a p value of 0.023, and an *r²* value of 41%. In a clinical pharmacology study (Lilian Murray et al in Br J Clin Pharmacol. 1998; 45(6): 559-566) of Enalapril, a well-prescribed medicine for hypertension, a significant correlation of enalapril and reduction in blood pressure was observed in the study (*p* less than 0.01). However, "the predictive power of the model increased (*r*² = 23.6%, p less than 0.01) but left the majority of the variability in response unexplained".

In the cases where samples were taken for immunohistochemistry, the hearts were embedded in OCT media and flash-frozen in liquid nitrogen (n=6 per group). Sections were cut at the basal, middle and apex level of the heart. The embedded frozen tissues were sent to QualTek Technology (Santa Barbara, CA) for further histology evaluation. The tissues were thawed at room temperature and re-fixed in formalin and embedded in paraffin and sectioned into 5µm sections. Sections were stained with an antibody against human Nuclear Matrix Antigen (hu NuMA) in order to discern human cardiac tissue-derived cells within rat myocardium.

The immunohistochemistry results were consistent with the results from qPCR. Positive human NuMA staining was identified in myocardium from animals receiving the target dose of 1 x 10⁶ hCTC (A3) cells. See **Figure 28****, panel a,** and **Figure 29****.** NuMA positive cells that stain dark brown and similar in staining characteristics seen with human tissue controls are shown here in the two oval circles and with no background staining present. The estimate of cell number was approximately 100 human cells /section. Under high magnification, myocyte-like human cells were also identified as shown in **Figure 29****, panel d.** No staining for human NuMA was observed in vehicle treated animals. See **Figure 28****, panels a** and **b,** **Figure 29** and **Figure 30****.**

### Example 13

### Human Cardiac Tissue-Derived Cells Reduced Hypertrophy in an Animal Model of Acute Myocardial Infarction

To further understand the mechanisms and the biological benefits of human cardiac tissue-derived cells as a therapy for damaged myocardium, tissue samples were taken from the hearts of the animals treated with human cardiac cells in **Example 11,** to determine the effect of administration of human cardiac tissue-derived cells on the infarct size general pathology of the heart.

Histopathology was evaluated by a pathologist at QualTek (Santa Barbara, CA). The pathologist was blinded to the study treatment. Heart tissues were embedded in paraffin blocks. Sections were obtained at every 5µm through the whole organ and evaluated for general pathology. Hypertrophy evaluation was performed by a scoring system. In hypertrophic myocardium (score 1), i.e. myocardial cells with enlarged cytoplasm and odd nuclei were commonly found. Otherwise, the myocardium is scored 0. The number of sections with hypertrophy and without hypertrophy was counted and presented in **Figure 31** as proportion of total sections to represent the proportion of hypertrophy in the whole heart.

Myocardial hypertrophy was observed in the hearts of vehicle treated animals, wherein approximately 70% of the myocardium showed hypertrophy (score 1). See **Figure 31****.** Treatment with human cardiac tissue-derived cells significantly reduced the hypertrophy observed in hearts, when compared to vehicle treated animals. In hearts receiving hCTC (A3) cells at either 1 x 10⁴, 1 x 10⁵, or 1 x 10⁶ target doses, hypertrophic myocardium was reduced to 30%-50%. See **Figure 31****.**

To elucidate the severity of myocardial infarction, Masson trichrome staining was performed on sections at the papillary muscle level from each heart. Infarct size was determined by direct measurement of the infarct area and the non-infarcted area. The relative infarct size was estimated by 100% [infarct area/(infarct area +non-infarct area)]. All morphometric studies were performed according to the methods described in Iwasaki et al in Circulation. 2006; 113:1311-1325.

A trend towards reduction in the relative infarct size was observed in animals receiving either 1 x 10⁵ (16.5±7.3%, p=0.02), or 1 x 10⁶ hCTC (A3) cells (14.8±8.6%, p=0.01), compared with vehicle group (24.1±2.9%). See **Figure 32****, panel a.** Similarly, a trend towards reducing infarct size by the actual infarct area was also observed in animals receiving either 1 x 10⁵ (557±221, p=0.09), or 1 x 10⁶ (537±261, p=0.08) hCTC (A3) cells, compared with the vehicle treated group (748±191). See **Figure 32****, panel b.**

Myocardial hypertrophy was observed in the hearts of vehicle treated animals, wherein approximately 70% of the myocardium showed hypertrophy (score 1). See **Figure 31****.** Treatment with human cardiac tissue-derived cells significantly reduced the hypertrophy observed in hearts, when compared to vehicle treated animals. In hearts receiving hCTC (A3) cells at either 1 x 10⁴, 1 x 10⁵, or 1 x 10⁶ target doses, hypertrophic myocardium was reduced to 30%-50%. See **Figure 31****.** The reduction of hypertrophy achieved by hCTCs may be attributed directly via trophic or paracrine effects, i.e. cytokines secreted by hCTC, as shown in **Table 24** and/or due to a secondary effect of increased de novo myocyte generation as discussed in **Example 14** below.

### Example 14

### Human Cardiac Tissue-Derived Cells Increased capillary Density in an Animal Model of Acute Myocardial Infarction

To further understand the mechanisms and the biological benefits of human cardiac tissue-derived cells as a therapy for damaged myocardium, tissue samples were taken from the hearts of the animals treated with human cardiac cells in **Example 11,** to determine the effect of administration of human cardiac tissue-derived cells on the capillary density at the border zone of the infarcted area.

Five tissue sections of the left ventricles from each heart, taken at the border zone of the infracted area were selected at random, and capillary density was morphometrically evaluated by histological examination, wherein the capillaries were visualized using an antibody to isolectin B4 (Vector Laboratories, Burlingame, CA), or CD31. Isolectin B4 is specific for endothelial cell surface sugar residues and has been documented to recognize endothelial cells in many settings as described by Vasudevan et al in Nature Neuroscience 11: 429-439 (2008) and by Schmidt et al in Development 134, 2913-2923 (2007). CD31, also known as platelet endothelial cell adhesion molecule (PECAM) has been applied extensively to identify endothelial cells and thus, vasculature in various tissues including the heart (Tabibiazar and Rockson Eur Heart J 2001vol 22; 903-918).

Visualization of capillaries either by isolectin B4, or CD31, demonstrated that administration of human cardiac tissue-derived cells increased capillary density at the border zone of the infracted area. Administration of hCTC (A3) cells at all doses resulted in the increase in capillary density, compared to vehicle treated groups, four weeks post cell administration. See **Figure 33****, panels a** and **b.** (p=0.0068 for Isolectin-B4 staining and p=0.0005 for CD31 staining).

The increase in capillary density may have been due, in part, to the secretion of factors from the human cardiac tissue-derived cells of the present invention. These trophic factors may act, for example, in a paracrine manner on the heart cells. The trophic factors may affect, either directly, or indirectly, blood vessel formation, blood vessel function and hemodynamics, cardiac muscle remodeling and function, myocyte proliferation (such as myogenesis), myocyte hypertrophy, fibrosis or increasing cardiac cell survival. The trophic factors may also regulate the recipient's immune response. To determine whether human cardiac tissue-derived cells secrete trophic factors, culture media was collected from populations of hCTC (A3) cells that had been cultured *in vitro* for seven days. Samples of the media were stored at -80°C, prior to assaying for the presence of secreted cytokines.

Cytokines secreted by hCTC (A3) cells included vascular endothelial growth factor (VEGF) and angiopoietin 2 (ANG2). See **Table 24.** , These cytokines play a significant role in angiogenesis. More importantly, the combination of VEGF and ANG2 can synergistically initiate and enhance capillary sprouting process, as has been documented by Maisonpierre et al in Science 277:55-60 (1997) and reviewed by Ramsauer et al in Journal of Clinical Investigation 110: 1615-1617 (2002).

### Example 15

### Human Cardiac Tissue-Derived Cells Increased Myocyte Density in The Non-Infarcted Area in Animals Receiving the Human Cardiac Tissue-Derived Cells of the Present Invention

To further understand the mechanisms and the biological benefits of human cardiac tissue-derived cells as a therapy for damaged myocardium, tissue samples were taken from the hearts of the animals treated with human cardiac cells in **Example 11,** to determine the effect of administration of human cardiac tissue-derived cells on the proliferation of rat myocytes at the border zone of the infarcted area, and the density of myocytes in non-infarcted regions of the heart.

Formalin-fixed, paraffin-embedded tissue samples were sectioned at 4µm. One slide at approximately 17^{th} section was selected from each animal. The sections were incubated with an antibody to Ki-67 (MIB-5) for 60 minutes at room temperature, washed in PBS, and incubated with a micropolymer labeled affinity mouse IgG secondary antibody. The slides were washed in PBS and then developed with a Vector SG Substrate that produces a navy blue/gray reaction product. The slides rinsed in PBS counterstained using DAPI (KPL Gaithersburg, Maryland). Positive and negative controls were included in each staining protocol.

Parallel formalin-fixed sections were incubated with an antibody to cardiac myosin for 45 minutes at room temperature, washed in PBS, and incubated with a biotinylated mouse IgG secondary antibody. After the secondary incubation was complete, Vectastain ABC-AP reagent (Vectastain Universal ABC-AP Kit, Vector Laboratories, Inc., Burlingame, CA) was applied for 30 minutes. The slides were washed in PBS and then developed using Liquid Permanent Red Chromogen (Dako, Carpinteria, CA) that produces a dark pink to red reaction product. The slides rinsed in PBS counterstained using DAPI (KPL Gaithersburg, Maryland). Positive and negative controls were included in each staining protocol.

Proliferating myocytes were measured by double staining of Ki-67 and myosin heavy chain (MHC). Total myocytes, recognized by MHC staining were counted. The number of total myocytes in one high-power field was similar between vehicle and cell treated groups at all doses. The ratio of proliferating myocytes among total myocytes was higher in animals receiving either 1 x 10⁴ (3.8±0.02%) or 1 x 10⁵ (3.7±0.02%) hCTC (A3) cells, compared to vehicle treated (2.3±0.01%) animals, or animals recieving 1 x 10⁶ (1.2±0.01%) cells. See **Table 25** and **Figure 34****.**

One possible explanation for the lower ratio of proliferating myocytes among total myocytes in animals receiving 1 x 10⁶ cells may be due to the myocytes entering into G0 in response to hCTC (A3) treatment. Ki-67 is a cell proliferating marker, present in all phases during cell cycle. However, when cycling cells exit into G0 phase, Ki-67 is no longer present. See, for example, (Thomas Scholzen 2000; Journal of Cellular Physiology; 182 (3), 311-322).

*H&E staining*: Slides were deparaffinized with 2 changes of xylene, 10 minutes per slide, then re-hydrated in 2 changes of absolute alcohol, 5 minutes each, then 95% alcohol for 2 minutes and 70% alcohol for 2 minutes. Slides were washed briefly in distilled water, then stained in hematoxylin solution for 8 minutes, washed in running tap water for 5 minutes, differentiated in 1% acid alcohol for 30 seconds, washed running tap water for 1 minute, stained in 0.2% ammonia water for 30 seconds to 1 minute. Then the slides were washed in running tap water for 5 minutes, rinsed in 95% alcohol (10 dips), then counterstained in eosin-phloxine B solution for 30 seconds to 1 minute, dehydrated with 95% alcohol, 2 changes of absolute alcohol, 5 minutes each, washed in 2 changes of xylene, 5 minutes each, and mounted with xylene based mounting medium.

For H&E stained slides, one level was sampled in each animal. In each level, five 400X fields (67,500 µm² per field) containing transversely cut myofibrils with mostly cross-sectioned capillaries in the left ventricular wall remote from the infarct were chosen. Myocyte density was reported for each level as the average of five fields and expressed in mm². The mean, standard deviation, and standard error of the mean were calculated for each treatment group.

Individual myocytes were generally visible in the hematoxylin and eosin (H&E) stained tissue at 400X magnification. **Figure 35** shows representative images obtained from samples of hearts receiving 1 x 10⁵ hCTC (A3) cells, together with samples obtained from vehicle treated animals. In **Table 26,** the mean for the vehicle group (1813 ± 84/mm²) was lower than the mean for any of the treatment groups (1 x 10⁴ hCTC (A3) cells: 2210 ± 227, 1 x 10⁵ hCTC (A3) cells: 2220 ± 186, 1 x 10⁶ hCTC (A3) cells: 2113 ± 186). The increased myocyte density may be attributed by the increased proliferating myocytes (myogenesis) and/or by the reduced myocyte hypertrophy, such as that described in **Example 13.**

### Example 16

### Human Cardiac Tissue-Derived Cells Treatment Induced Differential Gene Expression in Rat Myocardium

In order to understand the molecular alterations induced by human cardiac tissue-derived cell administration, a gene profiling study was conducted to compare gene expression levels in vehicle and human cardiac tissue-derived cell treated groups. Rat hearts were collected from animals that had received 1 x 10⁴, 1 x 10⁵, 1 x 10⁶ hCTC (A3) cells, or vehicle, four weeks after cell administration, from animals used in the study described in Example 11. Total RNA was collected from the samples.

The HG-U133_Plus_2 gene chip from Affymetrix was used to perform the analysis of gene expression in the samples. Using Spotfire DecisionSite the microarray data set was normalized across the microarray chips by the "Normalize by mean" function. The individual chips were organized into groups for comparison (1 X10⁴, 1 X10⁵, and 1 X10⁶ hCTC (A3) target dose, and vehicle). Using Spotfire DecisionSite, the p-Values and Fold Change between groups where established. Genes were filtered out that did not have a P in the Present Call column of the data in at least 3 columns, have a group comparison p-Value less than or equal to 0.05 in at least 2 columns, and any genes that did not have a fold change greater than or equal to 2.0 or less than or equal to 0.5 in at least 2 columns. The filtered set comprised 45 genes of interest. The 45 genes were then entered into the Principle Component Analysis (PCA) program from Spotfire DecisionSite to visually show group separation using this subset of genes. See **Table 27,** wherein the differentially expressed genes were identified and listed.

Among the genes identified, transforming growth factor-beta receptor (TGFβR) was down regulated in animals that received hCTC (A3) cells, at any dose. See **Figure 35****.** The TGFβR pathway has been previously implicated to an enhanced hypertrophy (see, for example, Watkins, Jonker et al. Cardiovasc Res. 2006 Feb 1; 69 (2):432-9) and remodeling after infarction in myocardium (Ellmers, Scott et al. Endocrinology. 2008 Nov;149(11):5828-34.). Blockade of TGFβ and TGFβR has been reported to reduce remodeling and fibrosis following hypertrophy (see, for example, Ellmers, Scott et al. Endocrinology. 2008 Nov; 149(11): 5828-34). In addition, activation of the TGFβ and TGFβR pathway post infarction has been reported to increase myocyte and ventricular hypertrophy and remodeling (see, for example, Matsumoto-Ida, Takimoto et al. Am J Physiol Heart Circ Physiol. 2006 Feb; 290(2): H709-15).

Another gene that was identified by differential gene expression analysis was neuronal nitric oxide synthase (NOS1). Post infarction, the expression of NOS 1 in the heart increased. The over-expression of NOS 1 has been reported to reduce the contractility of myocardium (see, for example, (Burkard, Rokita et al. Circ Res. 2007 Feb 16; 100(3): e32-44). In a human failing heart, NOS1 expression at mRNA and protein level has been reported to increase significantly, indicating a role of NOS 1 in the pathogenesis of cardiac dysfunction (see, for example, Damy, Ratajczak et al. Lancet. 2004 Apr 24; 363 (9418):1365-7). In hCTC (A3) cell-treated myocardium, at all doses, NOS1 expression was reduced compared with vehicle-treated myocardium, by more than 10 fold.

### Example 17

### Human Cardiac Tissue-Derived Cell Treatment Reduced Infarct Size and Prevented Hypertrophy in a Rat Model of Acute Myocardial Infarction

The efficacy of the human cardiac tissue-derived cells to treat damaged myocardium was compared to bone marrow-derived mesenchymal stem cells, in a rodent model of acute myocardial infarction. 96 female nude rats (Charles River Laboratories) at 8-10 weeks old were used for this study. Surgical procedures were performed as described in **Example 11.** 1 x 10⁵ hCTC (A3) cells (Lot 1) were administered in a volume of 100 µl CryoStor D-lite. In parallel, 1 x 10⁶ human mesenchymal stem cells (Cat# PT-2501, Lonza) were administered in a volume of 100 µl Cryostor D-lite. A similar procedure to that described in Example 11 was used with the following modifications based on surgeon's preference: cells were injected into two sites with 50 µl each at border zone of infarct, using a 0.3 ml insulin syringe fitted with a 29-gauge needle, roughly 10 minutes post-LAD ligation when discoloration of infarct area was clearly observed. Animals were sacrificed at 28 days post cell administration and the hearts removed for subsequent analysis.

The atria were trimmed and ventricles were flushed with saline. The hearts were immersed in 10% neutral buffered formalin (NBF) for 24 h before being cut into four 2 mm slices, Each slice was processed for microscopic examination, embedded in paraffin, sectioned at 5 µm, and stained with hematoxylin and eosin (H&E) and/or Masson's Trichrome. Two sections are shown side-by-side from each animal: one taken from the mid line between the papillary muscle and basal level and one taken from the papillary muscle.

Tissue sections from all groups and time points were blinded and put into rank order according to severity of disease (decompensation/dilatation and hypertrophy) from worst to least. Each ordinal was assigned a number, with the highest number corresponding to greatest severity of disease. The blind was then broken and all rank values from each group compiled.

All images were collected throughout the left ventricular free wall, which included the infarct. Low magnification (2X) images were collected from 2 tissue slices stained with trichrome from each animal and analyzed for infarct size. Image-Pro Plus v 5.1 software (Media Cybernetics, Inc., Bethesda, MD) was used to perform the automatic morphometric analysis of infarct size on the collected images. The perimeter of left ventricular free wall was traced and designated as the area of interest (AOI). The percent occupied by blue staining, representing infarct, and the percent by red staining, representing functional myocardium, were the two measurements collected from each image.

Tissue sections of heart stained with H&E and/or Masson's Trichrome were examined 28 days post-infarction. Animals treated with 1 x 10⁵ hCTC (A3) cells, as well as animals treated with 1 x 10⁶ human mesenchymal stem cells showed a reduced infarct area, compared to vehicle treated animals. A reduction in the dilatation of both the left and right ventricles was also observed. See **Figure 36****, panels a** and **b.** Additionally, there was preservation of functional myocardium in the left ventricular free wall in animals that had received 1 x 10⁵ hCTC (A3) cells, as well as animals treated with 1 x 10⁶ human mesenchymal stem cells. See **Figure 36****, panel c.**

Interestingly, hCTC (A3) cells and human mesenchymal stem cells demonstrated differential effects on the interventricular septum (IVS), with hMSC showing hypertrophic enlargement of IVS, while no such change was observed in animals that received hCTC (A3) cells. See **Figure 37****.** Evidence of hypertrophic changes of the cardiomyocytes was present in the septa of both groups, but was more pronounced in the animals that received hMSC. The hypertrophic myocytes and IVS contribute to the eventual remodeling in the heart, suggesting the human cardiac tissue-derived cells of the present invention may have more beneficial effects on cardiac function than human mesenchymal stem cells.

### Example 18

### Multiple-Lots and Long-Term Efficacy of the Human Cardiac Tissue-Derived Cells of the Present Invention in an Animal Model of Acute Myocardial Infarction

Multiple lots of human cardiac tissue-derived cells were prepared from three donors. The donor information is described in **Table 28.** Briefly, lot 1 is from a transplant-grade heart organ; lot 2 from a healthy heart but donor failed age criteria for transplantation; lot 3 from a donor diagnosed with dilated cardiomyopathy, a failing heart condition.

Female nude rats (weight, 250 to 300 g) were anesthetized with ketamine and xylazine (60 and 10 mg/kg IP, respectively). The thorax was opened at the fourth left intercostal space, the heart was exteriorized, and the pericardium was incised. Thereafter, the heart was held with forceps, and a 6-0 Proline suture was looped under the left anterior descending coronary artery, approximately 2 mm from its origin. AMI was induced in the heart by pulling the ligature, occluding the artery. Discoloration of the infracted myocardium was visually observed.

Twenty minutes after induction of MI, rats received an intramyocardial transplantation of 1x10⁶ of hCTC (A3) cells from either lot 1, 2, or 3 hCTC (A3). In parallel, animals received 1x10⁶ of pCTC (A3) cells or human neonatal dermal fibroblast cells (Cat # CC-2509, Lonza) or vehicle. All cell populations had been cryopreserved prior to administration and were injected into the heart in a final volume of 120 µl in CryoStor Dlite. Cells were administered at 2 sites, 50 µl cell suspension or CryoStor Dlite was injected at each site. After the injection was completed, the thorax was closed. 10 animals were enrolled in each group. The observed mortality rate was 32% in the study. There was no significant difference for mortality between groups as shown in **Table 29.** Absolute values of cardiac function are shown in **Table 30.**

Transthoracic echocardiography (SONOS 5500, Philips Medical Systems) was performed to evaluate LV function at 1, 4, and 12 weeks after cell administration. The following parameters were measured: left ventricular (LV) end-diastolic dimension (LVEDD), LV end-systolic dimension (LVESD), fractional shortening (FS), regional wall motion score (RWMS).

Administration of hCTC (A3) cells from Lot 1 improved cardiac function in all the parameters tested, at 28 days and at 84 days post cell administration. See **Figures 38** and 39. Administration of hCTC (A3) cells from Lot 2 was able to improve cardiac contractility and global cardiac function at 84 days after infarction, as measured by regional wall motion score RWMS and FS.

FS at 7 days post cell administration was similar between vehicle and cell treated groups. However, 84 days post cell administration, cardiac function measured by FS was improved by 9.5 ± 6.0% (n= 8, p<0.01), 8.9 ± 4.1% (n=8, p<0.001) in hCTC (A3) cells Lot 2 and hCTC (A3) cells from 1 treated groups, respectively. See **Figure 39** and **Table 30.** Minimal to no change in FS was observed in vehicle treated groups (-1.0±3.3%), hCTC lot 3 (-0.4±3.0%) and human fibroblast (4.1±2.1%) groups. See Figure 39 and **Table 30.** Consistent with the global function, RWMS was also reduced in animals that received either hCTC (A3) lot 1 from 24.83±1.64 at day 7 to 22.13±1.5 (N=8, p less than 0.05) or lot 2 cells from 25.44±1.0 to 23.13±2.2 (N=8, p less than 0.05), compared to vehicle treated animals. See **Table 30.**

In the current study, hCTC (A3) cells from either lot 1 or lot 2 prevented remodeling at 28 and 84 days after infarction, demonstrated by LVESD. At 28 days after cell administration, LVESD was reduced in animals that had received hCTC (A3) cells from lot 1 (-8.9 ± 4.1%). LVESD in animals that had received hCTC (A3) cells from lot 2 was maintained at baseline (-0.5±4.3%). Conversely, in vehicle treated animals, at 28 days post cell administration, LVESD increased by 16.4 ± 5.2%. In animals that had received hCTC (A3) cells from lot 3, or human fibroblasts, LVESD was increased by 9.1 ± 2.3% and 5.4 ± 3.6%, respectively. See **Figure 38** **and Table 30.**

At 84 days after cell administration, in vehicle group, LVESD was increased by 16.3± 2.8%. Similarly, the animals that had received hCTC (A3) cells from lot 3, or human fibroblast treated animals also showed enlargement of left ventricle at 84 days. LVESD was increased by 12.5±3.7% and 7.6±3.7%, respectively. In contrast, cardiac remodeling did not occur in animals that had received hCTC (A3) cells from lot 1 (-3.9 ± 5.2%), or in animals that had received hCTC (A3) cells from lot 2 (-1.8±4.2%). See **Figure 39** **and Table 30.**

The increase in the dilatation of left ventricle, as measured by LVEDD, at the end of diastole was prevented in animals that had received hCTC (A3) cells from lot 1 (7 days: 0.80±0.10 cm 84 days: 0.84±0.07 cm, 5% increment) and in animals that had received hCTC (A3) cells from lot 2 (7 days: 0.74±0.07 cm 84 days: 0.82±0.06 cm; 6.7% increment). Conversely, LVEDD was increased in vehicle treated animals (7 days: 0.75±0.03 cm; 84 days: 0.86±0.06 cm; 14.6% increment), and animals that had received human fibroblasts (7 days: 0.73±0.034 cm; 84 days: 0.83±0.06 cm; 13.7% increment). Animals that had received hCTC (A3) cells from lot 3 also showed an increase in LVEDD (7 days: 0.73±0.04 cm; 84 days: 0.82±0.06 cm; 12.3% increment). See **Figure 39** **and Table 30.**

### Example 19

### Cardiac Tissue-Derived Cell Size

Methods and Materials: Cell size of the cardiac tissue-derived cells, obtained from human, mouse, pig and rat hearts, according to the methods of the present invention was analyzed during cell counting. The total viable cell counting was performed after digestion and before replating of the cell populations using the Vi-Cell™ XR (Beckman Coulter, Fullerton, CA). The Vi-Cell™ cell viability analyzer automates the trypan blue dye exclusion method for cell viability assessment using video captures technology and image analysis of up to 100 images of cells in a flow cell.

Samples were prepared and analyzed according to the manufacturer's instructions (Reference Manual PN 383674 Rev.A). Briefly, a 500µL aliquot of the final cell suspension obtained after RBC lysis was transferred to a Vi-Cell™ 4 ml sample vial and analyzed using a Vi-Cell™ XR Cell Viability Analyzer. Cell size was determined by the diameter of the average of the counted cells.

The average of the diameter of hCTC (A3) cells was 16.7 ±2.13µm. The diameter of rCTC (A2) cells was 18.4±1.02µm and the diameter of pCTC(A3) cells was 17.2±0.42µm. Based on these data, the filter size of greater than or equal to 20µm would allow the cardiac tissue-derived cells of the present invention to pass through the filter to be collected, and exclude other cell types.

### Example 20

### Cryopreservation of the Cardiac Tissue-Derived Cells of the Present Invention

It is advantageous to generate a product that can be administered directly without further processing at clinics. To generate such a product, cryopreservation of human cardiac tissue-derived cells was tested using a clinically approved cryopreservation solution. In addition, the toxicity of the cryopreservation solution in myocardium was also tested.

For cryopreservation, hCTC (A3) cells were collected from flasks by trypsinization. Cell banks were cryopreserved in CryoStor Dlite™ (BioLife Solutions, Inc. Bothell, WA) containing 2% v/v DMSO. CryoStor Dlite is an animal-origin-free cryopreservation designed to prepare and preserve cells in ultra low temperature environments (-80°C to - 196°C) according to the principles described in Advances in Biopreservation edited by J.G. Baust and J.M. Baust. Other solutions that provide, for example, necessary electrolyte, osmotic and buffering conditions for hypothermic storage may also be used.

The cell suspensions were cryopreserved in Nalgene 2 mL polypropylene, sterile, internal thread with Screw Cap cryovials (Nalgne Nunc, Rochester, NY) using an Integra 750 Plus programmable freezer (Planer, Middlesex, U.K.) with DeltaT software. Cell and solutions were at room temperature prior to loading into the programmable freezer, which was held at 15°C. A sample temperature probe was placed in a vial of freezing buffer. The following program was used to cryopreserve cells:

| Step No. | Rate (°C/min) | End Temp (°C) | Trigger |
|---|---|---|---|
| 1 | -1 | -6 | Sample |
| 2 | -25 | -65 | Chamber |
| 3 | +10 | -19 | Chamber |
| 4 | +2.16 | -14 | Chamber |
| 5 | -1 | -100 | Chamber |
| 6 | -10 | -140 | Chamber |

When the temperature reached -140 °C, samples were transferred to liquid nitrogen tank for storage.

**Table 1: Comparison of hCTC with other cardiac derived cells.**

| | hCTC | rCTC | Anversa | Marban | Schneider | Chien |
|---|---|---|---|---|---|---|
| Tissue source | Transplant-discard whole heart | 8-12 weeks whole heart | Human: Atrial biopsies(20-100mg) Rat: 6-12 weeks old whole heart | Biopsies (1-2mm³) Mouse: whole heart | Mouse: whole heart | Moue whole heart |
| Digestion enzyme | Collagenase (GMP grade) Dispase (GMP grade) | Collagenase (GMP grade) and Dispase(GMP grade) | Collagenase II | Trypsin Collagenase IV | Collagenase II | Collagenase II |
| Enzyme concentration | 1u/ml 5u/ml | 1u/ml 5u/ml | No information | 0.2% 0.1% | 0.1% (800u/ml) | 240u/ml |
| Digestion time | 2.5 hr continuous | 2.5 hr continuous | No information | 5 mins | 30 mins continuous | 10 mins 4 round |
| Filter | 70u | 70u | none | N/a | 70u | None |
| Sorting | N/a | N/a | c-kit | N/a | Sca-1 | Islet-1 |
| Pre-plating | | Cell suspension on uncoated culture flask | tissue specimen on uncoated petri dish | Tissue specimen on fibrinectin | N/a | 2 round 1-hr on plastic |
| Pre-plating medium | DMEM+10%F BS | DMEM+10% FBS | DMEM+F12+5-10% FBS+insulin-transferrin | IMDM+10%FB S+2 mmol/L L-glutamine+ 0.1 mmol/L 2-mercaptoethanol | N/a | DMEM/M199( 4:1)+ 10% horse serum+ 5% FBS |
| Time to harvest cells | 2 days | 2 days | No information | 1-3 weeks | N/a | 2 hrs |
| Cells harvest | Phase-bright non-adherent | Phase-bright non-adherent | Sorting c-kit + cells | Sphere- forming cells | N/a | Sorting Islet-1+ cells |
| Cardiac gene expression | +: GATA4, Nkx2.5 | +: GATA 4, NKx2.5, Islet-1 | +: GATA4; Nkx2.5 | +: GATA4; Nkx2.5 | +: GATA4; | +: GATA4 Nkx2.5; Islet-1 |
| | -: MyHC; Islet-1 | -: MyHC | -: MyHC | -: MyHC | -: MyHC | -: MyHC |
| Stem cell gene expression | +: c-kit, CD34 | +: c-kit, telomerase, Nestin | +: c-kit, telomerase, Mdr | +: telomerase, c-kit | +: Sca-1 (mouse), telomerase | +: islet-1, Sca-1 (mouse), telomerase |
| | -: telomerase, Mdr | -: Mdr | -: CD34 | -: CD34 | -: c-kit, CD34 | -: |
| Surface marker | +:CD49e,CD59, CD105 CD34, c-kit | +: CD90, | +: c-kit, Mdr | +: CD105, CD90 | +: Sca-1 | +: Sca-1 |
| | -: CD16, CD31, CD45 | -: CD31, CD45 | -: Cd45, CD34, CD31, CD90 | -: CD45, CD31 | -: c-kit, CD31 | -: c-kit, CD45, CD31 |
| Reference | N/a | N/a | (Beltrami, Barlucchi *et* al. 2003) | (Messina, De Angelis et al*.* 2004; Smith, Barile et al. 2007) | (Oh, Bradfute et al. 2003) | (Laugwitz, Moretti et al. 2005) |

**Table 2: Yield and cell viability after digestion of heart tissue**

| **Isolation** | **Yield (million)** | **Viability** |
|---|---|---|
| 20061130 | 61.9 | 65% |
| 20071116 | 49.2 | 78% |
| 20071127 | 64 | 55% |
| 20080116* | 34 | 81% |

| | | |
|---|---|---|
| ***: half of the heart was processed** | | |

**Table 3: Viability after cryopreservation and needle passage**

| **Time (min)** | **Cells/mL (x 10⁶)** | **Viability** |
|---|---|---|
| **Pre-Needle** | **0.54** | **93.9** |
| **0** | **0.44** | 93.2 |
| **10** | **0.46** | 92.4 |
| **20** | **0.43** | 93.5 |
| **30** | **0.46** | 93.3 |
| **30-no needle** | **0.46** | 94.4 |

**Table 4: Karyotype of hCTC (A3)**

| | |
|---|---|
| Cell count | 20 |
| Cell analyzed | 20 |
| Karyotype: | 5 |
| Normal Karyotype | 5 |

**Table 5: Rat CTC recovery and viability after cryopreservation and needle passage.**

| | **Pre-Needle*** | | **Post-Needle*** | |
|---|---|---|---|---|
| **Time (min)** | **Cells/mL (x 10⁶)** | **Viability** | **Cells/mL (x 10⁶)** | **Viability** |
| **0** | **1.43** | 94.9% | 1.54 | 94.2% |
| **10** | **1.44** | **94.1%** | **1.54** | **94.7%** |
| **20** | **1.56** | 93.0% | 1.30 | 94.6% |
| **30** | **1.23** | 94.4% | 1.27 | 93.5% |

| | | | | |
|---|---|---|---|---|
| *** n = 3, data represents the average. Time indicates the incubation time at room temperature, which reflects the preparation time during cell injection procedure in myocardial infarction model.** | | | | |

**Table 6: Antibodies used in the 96-well plate flow cytometry assay**

| | | |
|---|---|---|
| MigG1 | BD pharmingen | 550083 |
| MigG2a | BD pharmingen | 349053 |
| MigG2b | R & D Systems | IC0041P |
| CD9 | BD pharmingen | 555372 |
| CD11a | BD pharmingen | 555380 |
| CD16 | Caltag Laboratories | MHCD1604 |
| CD29 | BD pharmingen | 556049 |
| CD31 | BD pharmingen | 555446 |
| CD34 | BD pharmingen | 550761 |
| CD44 | BD pharmingen | 550989 |
| CD45 | BD pharmingen | 555483 |
| CD49b | BD pharmingen | 555669 |
| CD49e | BD pharmingen | 555617 |
| CD54 | BD pharmingen | 347977 |
| CD59 | BD pharmingen | 555764 |
| CD62E | BD pharmingen | 551145 |
| CD62L | BD pharmingen | 555544 |
| CD62P | BD pharmingen | 555524 |
| CD63 | BD pharmingen | 556020 |
| CD73 | BD pharmingen | 550257 |
| CD81 | BD pharmingen | 555676 |
| CD90 | BD pharmingen | 555596 |
| CD104 | BD pharmingen | 555720 |
| CD105 | Caltag Laboratories | MHCD10504 |
| CD106 | BD pharmingen | 555647 |
| CD117 | BD pharmingen | 340529 |
| CD140b | BD pharmingen | 558821 |
| CD141 | BD pharmingen | 559781 |
| CD142 | BD pharmingen | 550312 |
| CD146 | BD pharmingen | 550315 |
| CD147 | Serotec | MCA1876PE |
| CD184 | BD pharmingen | 555974 |
| MDR | BD pharmingen | 557003 |

**Table 7: Mean fluorescence intensity (MFI) and delta MFI**

| | Antibody | isotype | Delta MFI | percent of total populaiton |
|---|---|---|---|---|
| CD16 | 127.05 | 108.21 | 18.84 | 2.27% |
| CD31 | 105.16 | 99.36 | 5.8 | 1.45% |
| CD45 | 109.37 | 99.36 | 10.01 | 5.26% |
| CD34 | 178.41 | 99.36 | 79.05 | 16.29% |
| CD59 | 1039.5 | 166.59 | 872.91 | 95.55% |
| CD105 | 394.58 | 99.36 | 295.22 | 94.61% |
| c-Kit | 67.63 | 24.75 | 42.88 | 30.60% |

**Table 8: Cell surface markers in three hCTC cell populations.**

| Surface marker | A1 | A2 | A3 |
|---|---|---|---|
| isotype | 1% | 1% | 1% |
| CD16 | 2.5% | 0.8% | 2.27% |
| CD31 | 2.49% | 0.64% | 1.45% |
| CD45 | 7.5% | 2.94% | 5.26% |
| CD34 | 42.8% | 6.27% | 16.29% |
| CD49e | 97.7% | 85.9% | N/A |
| CD59 | 96.5% | 93.3% | 95.55% |
| CD105 | 97.8% | 95.6% | 94.61 % |
| c-Kit | 22.1% | 2.54% | 30.6% |

**Table 9: Primer sets used in qPCR**

| Primer | Catalog number |
|---|---|
| Cardiac Actin | Hs00606316_m1 |
| C-kit | Hs00174029_m1 |
| GAPDH | Hs99999905_m1 |
| GATA4 | Hs00171403_m1 |
| Isl-1 | Hs01099687_m1 |
| Myh7 | Hs00165276-m1 |
| Nestin | Hs00156568_m1 |
| Nkx2.5 | Hs00231763-m1 |
| Telomerase | Hs0162669_m1 |

**Table 10: comparison of different cells obtained from the procedure**

| **Category** | **Sub-category** | **Gene** | **A1** | **A2** | **A3** |
|---|---|---|---|---|---|
| House-keeping | | GAPDH | 17.48 | 18.61 | 18.62 |
| Cardiac specific | lineage commitment | GATA4 | 24.87 | 24.8 | 25.64 |
| | | Nkx2.5 | 32.45 | 31.89 | 34.77 |
| | Differentiation | actin 1 | n/a | n/a | n/a |
| | | MyHC | 39.05 | 38.06 | 38.27 |
| Stem cell marker | Multipotent | c-kit | 27.38 | 29 | 27.37 |
| | Cell division | Telomerase | Undetectable | Undetectable | undetectable |
| | embryonic | Islet-1 | Undetectable | Undetectable | undetectable |
| Lineage/differ entiation | GATA4/Actin | | n/a | n/a | n/a |
| | GATA4/MyHC | | 18561.2 | 9809.7 | 7098.8 |
| | Nkx2.5/Actin | | n/a | n/a | n/a |
| | Nkx2.5/MyHC | | 93.70 | 72.00 | 11.31 |

**Table 11: Gene expression in expanded hCTC**

| **Category** | **Sub-category** | **Gene** | **hCTC** | **huHeart** | **Fibroblast** |
|---|---|---|---|---|---|
| House-keeping | | GAPDH | 16.51 | 18.85 | 17.79 |
| Cardiac specific | lineage commitment | G4TA4 | 24.64 | 23.54 | 37.71 |
| | | Nkx2.5 | 34.78 | 23.81 | Undetectable |
| | Differentiation | actin 1 | n/a | n/a | n/a |
| | | MyHC | 33.67 | 16.89 | 34.69 |
| Stem cell marker | Mutipotent | c-kit | 25.86 | 29.77 | 27.95 |
| | Cell division | Telomerase | Undetectable | Undetectable | 36.34 |
| | embryonic | Islet-1 | Undetectable | Undetectable | Undetectable |
| Lineage/differentia tion | C-ATA4/Actin | | n/a | n/a | n/a |
| | GATA4/MyHC | | 522.76 | 0.01 | 0.12 |
| | Nkx2.5)Actin | | n/a | n/a | n/a |
| | Nkx2.5/MyHC | | 0.46 | 0.01 | 0.00 |

| | | | | | |
|---|---|---|---|---|---|
| Fibroblast: NHDF | | | | | |

**Table 12: rCTC gene expression**

| **Category** | **Sub-category** | **Gene** | **rCTC** | **rHeart** |
|---|---|---|---|---|
| House-keeping | | GAPDH | 18.89 | 16.2 |
| Cardiac specific | lineage commitment | GATA4 | 28.32 | |
| | | Nkx2.5 | 27.93 | 27.61 |
| | Differentiation | actin 1 | 33.78 | 26.65 |
| | | MyHC | 37.4 | 20.36 |
| Stem cell marker | Multipotent | c-kit | 34.85 | 27.95 |
| | Cell division | Telomerase | 30.06 | 30.73 |
| | embryonic | Islet-1 | 26.93 | 30.87 |
| Lineage/differentia tion | GATA4/Actin | | 44.02 | |
| | GATA4/MyHC | | 541.19 | |
| | Nkx2.5/Actin | | 57.68 | 0.51 |
| | Nkx2.5/MyHC | | 709.18 | 0.01 |

| | | | | |
|---|---|---|---|---|
| *: rHeart: Rat heart | | | | |

**Table 13: Mouse GFP-CTC gene expression**

| **Category** | **Sub-category** | **Gene** | **mGFP-CTC** | **mHeart** |
|---|---|---|---|---|
| House-keeping | | GAPDH | 19.07 | 19.41 |
| Cardiac specific | lieage commitment | GATA4 | 24.64 | 25.38 |
| | | Nkx2.5 | 30.48 | 26.71 |
| | Differentiation | actin 1 | 33.78 | 18.01 |
| | | MyHC | 38.05 | 19.59 |
| Stem cell marker | Multipotent | c-kit | 34.74 | 32.31 |
| | Cell division | Telomerase | 26.58 | 30.04 |
| | embryonic | Islet-1 | 33.52 | 38.78 |
| Lineage/differ entiation | GATA4/Actin | | 564 | 0.006 |
| | GATA4/MyHC | | 10884 | 0.02 |
| | Nkx2.5/Actin | | 9.9 | 0.002 |
| | Nkx2.5/MyHC | | 190 | 0.007 |

**Table 14: Gene expression in pCTC**

| | pCTC | Pig heart |
|---|---|---|
| GATA-4 | 28.54 | 23.86 |
| MyHC | undetectable | 22.21 |
| Telomerase | undetectable | undetectable |
| internal control | 15.25 | 15.07 |

**Table 15: Regional Wall Motion Score Definition**

| **Score** | **Wall motion** | **Definition** |
|---|---|---|
| 1 | Normal | Normal inward motion and thickening |
| 2 | Hypokinesis | Reduced wall motion and thickening |
| 3 | Akinesis | Absence of motion or thickening |
| 4 | Dyskinesis | Outward motion "bulging" |

**Table 16: Summary of the observed mortality rate**

| **Group** | **Mortality** |
|---|---|
| Vehicle | 6.7% (1/15) |
| rCPC 1e6 | 13.3% (2/15) |
| hCPC 1e4 | 13.3% (2/15) |
| hCPC 1e5 | 26.7%(4/15) |
| hCPC 1e6 | 20% (3/15) |

**Table 17: Cardiac function Summary**

| **Function parameter** | | **Fractional shortening (FS, %)** | | **Left Ventricular End Systolic Dimention (LVESD, cm)** | | **Left ventricular End Diastolic Dimention (LVEDD, cm)** | | **Regional Wall Motion Score (RWMS)** | |
|---|---|---|---|---|---|---|---|---|---|
| | | **Mean** | **SD** | **Mean** | **SD** | **Mean** | **SD** | **Mean** | **SD** |
| **Vehicle** | **7 days** | 22.19 | 3.8 | 0.58 | 0.077 | 0.75 | 0.075 | 24.93 | 2.1 |
| | **28 days** | 20.82 | 3.1 | 0.65 | 0.063 | 0.83 | 0.072 | 26.14 | 1.4 |
| **rCT (A2)** | **7 days** | 18.99 | 4.2 | 0.56 | 0.081 | 0.70 | 0.083 | 25.29 | 1.9 |
| | **28 days** | 23.06 | 6.1 | 0.62 | 0.082 | 0.80 | 0.071 | 23.86 | 2.3 |
| **hCTC (A3) 10⁴** | **7 days** | 21.28 | 3.7 | 0.56 | 0.049 | 0.71 | 0.045 | 24.42 | 1.4 |
| | **28 days** | 30.51 | 3.7 | 0.54 | 0.085 | 0.78 | 0.079 | 21.08 | 1.7 |
| **hCTC (A3) 10⁵** | **7 days** | 21.33 | 2.3 | 0.58 | 0.037 | 0.73 | 0.045 | 25.58 | 1.4 |
| | **28 days** | 31.88 | 5.0 | 0.51 | 0.080 | 0.74 | 0.082 | 21.08 | 1.9 |
| **hCTC (A3) 106** | **7 days** | 18.38 | 3.2 | 0.62 | 0.047 | 0.76 | 0.038 | 25.91 | 1.6 |
| | **28 days** | 33.99 | 6.2 | 0.48 | 0.091 | 0.72 | 0.094 | 20 | 1.7 |

**Table 18: Statistic analysis of absolute change of fractional shortening**

| Stud y Day | Comparison (A vs. B) | Percent Differenc e Estimate | Standar d Error | 95% Confidence Interval (Lower Upper) | | P-value | Point Estimate of A | Standar d Error of A | Point Estimate of B | Standard Error of B |
|---|---|---|---|---|---|---|---|---|---|---|
| 5 | rCPC vs Vehicle | -15 | 6 | -26 | -3 | 0.016 | 18.6 | 0.9 | 21.9 | 1.0 |
| 5 | 1e4 vs. Vehicle | -4 | 7 | -17 | 10 | 0.538 | 21.0 | 1.1 | 21.9 | 1.0 |
| 5 | 1e5 vs. Vehicle | -3 | 7 | -16 | 11 | 0.650 | 21.2 | 1.1 | 21.9 | 1.0 |
| 5 | 1e6 vs. Vehicle | -17 | 6 | -28 | -4 | 0.010 | 18.1 | 1.0 | 21.9 | 1.0 |
| 28 | rCPC vs Vehicle | 8 | 7 | -5 | 24 | 0.242 | 22.3 | 1.1 | 20.6 | 1.0 |
| 28 | 1e4 vs. Vehicle | 47 | 10 | 28 | 69 | < 0.001 | 30.3 | 1.6 | 20.6 | 1.0 |
| 28 | 1e5 vs. Vehicle | 53 | 11 | 33 | 76 | < 0.001 | 31.5 | 1.6 | 20.6 | 1.0 |
| 28 | 1e6 vs. Vehicle | 62 | 12 | 41 | 87 | < 0.001 | 33.5 | 1.8 | 20.6 | 1.0 |

**Table 19: Statistic analysis of absolute change of regional wall motion score**

| Stud y Day | Comparison (A vs. B) | Percent Differenc e Estimate | Standar d Error | 95% Confidence Interval (Lower Upper) | | P-value | Point Estimate of A | Standard Error of A | Point Estimate of B | Standard Error of B |
|---|---|---|---|---|---|---|---|---|---|---|
| 5 | rCPC vs Vehicle | 1 | 3 | -4 | 7 | 0.616 | 25 | 1 | 25 | 1 |
| 5 | 1e4 vs. Vehicle | -2 | 3 | -8 | 4 | 0.530 | 24 | 1 | 25 | 1 |
| 5 | 1e5 vs. Vehicle | 3 | 3 | -3 | 9 | 0.363 | 26 | 1 | 25 | 1 |
| 5 | 1e6 vs. Vehicle | 4 | 3 | -2 | 11 | 0.199 | 26 | 1 | 25 | 1 |
| 28 | rCPC vs Vehicle | -9 | 3 | -14 | -4 | 0.001 | 24 | 0 | 26 | 1 |
| 28 | 1e4 vs. Vehicle | -19 | 2 | -24 | -15 | < 0.001 | 21 | 0 | 26 | 1 |
| 28 | 1e5 vs. Vehicle | -20 | 2 | -24 | -15 | < 0.001 | 21 | 0 | 26 | 1 |
| 28 | 1e6 vs. Vehicle | -24 | 2 | -28 | -19 | < 0.001 | 20 | 0 | 26 | 1 |

**Table 20: Statistic analysis of absolute change of LVEDD**

| Stud y Day | Comparison (A vs. B) | Percent Differenc e Estimate | Standar d Error | 95% Confidence Interval (Lower Upper) | | P-value | Point Estimate of A | Standard Error of A | Point Estimate of B | Standard Error of B |
|---|---|---|---|---|---|---|---|---|---|---|
| 5 | rCPC vs Vehicle | -7 | 3 | -14 | 0 | 0.047 | 0.690 | 0.018 | 0.743 | 0.019 |
| 5 | 1e4 vs. Vehicle | -5 | 4 | -12 | 3 | 0.199 | 0.707 | 0.020 | 0.743 | 0.019 |
| 5 | 1e5 vs. Vehicle | -1 | 4 | -9 | 6 | 0.716 | 0.733 | 0.020 | 0.743 | 0.019 |
| 5 | 1e6 vs. Vehicle | 2 | 4 | -5 | 10 | 0.565 | 0.760 | 0.022 | 0.743 | 0.019 |
| 28 | rCPC vs Vehicle | -4 | 4 | -11 | 3 | 0.294 | 0.796 | 0.021 | 0.828 | 0.021 |
| 28 | 1e4 vs. Vehicle | -7 | 4 | -14 | 1 | 0.069 | 0.772 | 0.022 | 0.828 | 0.021 |
| 28 | 1e5 vs. Vehicle | -11 | 3 | -17 | -4 | 0.004 | 0.740 | 0.021 | 0.828 | 0.021 |
| 28 | 1e6 vs. Vehicle | -14 | 3 | -20 | -7 | < 0.001 | 0.712 | 0.021 | 0.828 | 0.021 |

**Table 21: Statistic analysis of relative change of LVEDD**

| Comparison | Simple Difference Estimate | Standard Error | 95% Confidence Interval | | P-value | Group | Point Estimate | Standard Error | Individual 95% Confidence Intervals | |
|---|---|---|---|---|---|---|---|---|---|---|
| rCPC vs. Vehicle | -0.0018 | 0.0466 | -0.0952 | 0.0916 | 0.969 | rCPC | 0.1083 | 0.0330 | 0.0423 | 0.1743 |
| | | | | | | Vehicle | 0.1101 | 0.0330 | 0.0441 | 0.1761 |
| 1e4 vs. Vehicle | -0.0260 | 0.0485 | -0.1232 | 0.0712 | 0.594 | 1e4 | 0.0841 | 0.0356 | 0.0128 | 0.1554 |
| | | | | | | Vehicle | 0.1101 | 0.0330 | 0.0441 | 0.1761 |
| 1e5 vs. Vehicle | -0.1170 | 0.0485 | -0.2142 | -0.0199 | 0.019 | 1e5 | -0.0069 | 0.0356 | -0.0782 | 0.0644 |
| | | | | | | Vehicle | 0.1101 | 0.0330 | 0.0441 | 0.1761 |
| le6 vs. Vehicle | -0.1561 | 0.0497 | -0.2556 | -0.0565 | 0.003 | le6 | -0.0459 | 0.0372 | -0.1204 | 0.0285 |
| | | | | | | Vehicle | 0.1101 | 0.0330 | 0.0441 | 0.1761 |

**Table 22: Statistic analysis of absolute change of LVESD**

| Study Day | Comparison (A vs. B) | Percent Difference Estimate | Standard Error | 95% Confidence Interval (Lower Upper) | | P-value | Point Estimate of A | Standard Error of A | Point Estimate of B | Standard Error of B |
|---|---|---|---|---|---|---|---|---|---|---|
| 5 | rCPC vs Vehicle | -3 | 5 | -12 | 7 | 0.517 | 0.559 | 0.020 | 0.577 | 0.021 |
| 5 | 1e4 vs. Vehicle | -4 | 5 | -13 | 7 | 0.483 | 0.556 | 0.021 | 0.577 | 0.021 |
| 5 | 1e5 vs. Vehicle | 0 | 5 | -10 | 11 | 0.982 | 0.576 | 0.022 | 0.577 | 0.021 |
| 5 | 1e6 vs. Vehicle | 7 | 6 | -4 | 19 | 0.222 | 0.616 | 0.025 | 0.577 | 0.021 |
| 28 | rCPC vs Vehicle | -6 | 5 | -15 | 4 | 0.201 | 0.611 | 0.022 | 0.652 | 0.023 |
| 28 | 1e4 vs. Vehicle | -17 | 4 | -26 | -8 | < 0.001 | 0.538 | 0.021 | 0.652 | 0.023 |
| 28 | 1e5 vs. Vehicle | -23 | 4 | -30 | -14 | < 0.001 | 0.503 | 0.019 | 0.652 | 0.023 |
| 28 | 1e6 vs. Vehicle | -28 | 4 | -35 | -20 | < 0.001 | 0.468 | 0.019 | 0.652 | 0.023 |

**Table 23: Rat CTC isolation from different part of heart**

| **Date of isolation** | **Atria** | | **Apex** | | **Remainnig Ventricle** | |
|---|---|---|---|---|---|---|
| | Yield | Viability | Yield | Viability | Yield | Viability |
| 71706 | 2.70E+06 | 81.10% | 4.90E+05 | 72.70% | 5.20E+06 | 77.90% |
| 72406 | 6.00E+06 | 68.70% | 1.20E+06 | 55.90% | 5.60E+06 | 55.70% |
| 80206 | 4.00E+06 | 89% | 1.02E+06 | 73.50% | 4.80E+06 | 78.60% |
| **Average** | **4.23E+06** | **79.6%** | **9.03E+05** | **67.4%** | **5.20E+06** | **70.7%** |
| **S.D.** | **1.66E+06** | **10.2%** | **3.69E+05** | **9.9%** | **4.00E+05** | **13.0%** |

**Table 24: Cytokine secretion from hCTC**

| **Cytokine** | **Unit** | **hCTC conditioned medium** | **Blank medium** |
|---|---|---|---|
| **Pierce Searchlight** | | | |
| **hTIMP1** | pg/ml | 120000.0 | 225.6 |
| **hKGF** | pg/ml | 118.8 | 21.2 |
| **hL-Selectin** | pg/ml | 1086.8 | 329.0 |
| **hHGF** | pg/ml | 787.4 | 259.6 |
| **hVCAM** | pg/ml | 1458.6 | 548.0 |
| **hHB-EGF** | pg/ml | 169.6 | 63.8 |
| **hICAM1** | pg/ml | 116.6 | 44.8 |
| **hVEGF-RI** | pg/ml | 95.8 | 41.2 |
| **hANG2** | pg/ml | 549.0 | 240.6 |
| **hE-Selectin** | pg/ml | 238.4 | 108.2 |
| **hPDGF-BB** | pg/ml | 41.2 | 23.2 |
| **hVEGF** | pg/ml | 1170.0 | 700.8 |
| **hE Cadherin** | pg/ml | 175.4 | 116.8 |
| **hP-selectin** | pg/ml | 4433.8 | 3040.0 |
| **hFGFb** | pg/ml | 8.6 | 6.2 |
| **hTPO** | pg/ml | 1119.8 | 892.6 |
| **hVEGF-RII** | pg/ml | 11.6 15.6 | 15.6 |

| **Rules-Based Medicine** | | | |
|---|---|---|---|
| **PAI-1** | **ng/mL** | 68 | <LOW> |
| **TIMP-1** | **ng/mL** | 58 | <LOW> |
| **IL-6** | **pg/mL** | 736 | <LOW> |
| **VEGF** | **pg/mL** | 346 | <LOW> |
| **MCP-1** | **pg/mL** | 623 | <LOW> |
| **IL-8** | **pg/mL** | 54 | 1.5 |
| **MIP-1alpha** | **pg/mL** | 8.1 | 0.73 |
| **Cancer Antigen 1** | **U/mL** | 0.35 | <LOW> |
| **FGF basic** | **pg/mL** | 107 | <LOW> |
| **Endothelin-1** | **pg/mL** | 7.1 | <LOW> |
| **Eotaxin** | **pg/mL** | 26 | <LOW> |
| **ICAM-1** | **ng/mL** | 0.13 | 0.044 |
| **Alpha-Fetoproteir** | **ng/mL** | 0.12 | 0.065 |
| **IL-1 beta** | **pg/mL** | 0.16 | 0.098 |
| **IL-10** | **pg/mL** | 0.36 | 0.32 |
| **IL-12p70** | **pg/mL** | 7.4 | 7.1 |
| **Cancer Antigen 1** | **U/mL** | 0.86 | <LOW> |
| **IL-7** | **pg/mL** | 13 | 13 |
| **Glutathione S-Trr** | **ng/mL** | 0.14 | 0.14 |
| **IL-13** | **pg/mL** | 7.0 | 7.6 |
| **SGOT** | **ug/mL** | 1.5 | 1.7 |
| **MMP-2** | **ng/mL** | 20 | <LOW> |
| **IFN-gamma** | **pg/mL** | 0.59 | <LOW> |
| **EGF** | **pg/mL** | 0.86 | <LOW> |

**Table 25: Ratio of proliferating myocytes in total myocytes at border zone**

| **Group** | **Ki-67/total myocyte** | **std dev** | **SEM** |
|---|---|---|---|
| **vehicle** | 2.34% | 1.36% | 0.55% |
| **hCTC 10000** | 3.85% | 1.79% | 0.73% |
| **hCTC 100000** | 3.69% | 2.49% | 1.02% |
| **hCTC 1000000** | 1.16% | 1.33% | 0.54% |

**Table 26: Myocyte density (mm²)**

| **group** | **vehicle** | **hCTC 10000** | **hCTC 100000** | **hCTC 1000000** |
|---|---|---|---|---|
| **mean** | 1813 | 2210 | 2220 | 2113 |
| **std** | 205 | 555 | 455 | 456 |
| **SEM** | 84 | 227 | 186 | 186 |

**Table 27: Differentially Expressed Genes In Treatment Groups Over Vehicle Group**

| **Accession ID** | **Gene Symbol** | **Regulation** |
|---|---|---|
| 1368674_at | Pygl | ↓ |
| 1368858_at | Ugt8 | ↑ |
| 1369015_at | Nos1 | ↓ |
| 1369653_at | Tgfbr2 | ↓ |
| 1370225_at | Cited4 | ↑ |
| 1370297_at | Plk1 | ↑ |
| 1370597_at | Stx17 | ↑ |
| 1371457_at | | ↑ |
| 1373799_at | | ↑ |
| 1373896_at | Syt1 | ↑ |
| 1374356_at | | ↑ |
| 1376856_at | RGD1310414 | ↑ |
| 1377059_at | Mapk10 | ↓ |
| 1377073_at | | ↑ |
| 1378206_a_at | | ↑ |
| 1378647_at | | ↑ |
| 1378834_at | Xrcc5 | ↑ |
| 1378867_at | | ↑ |
| 1379930_at | | ↑ |
| 1380586_at | Ggps1 | ↓ |
| 1380727_at | | ↑ |
| 1381588_at | RGD1310623 | ↑ |
| 1382433_at | Sorcs1_predicted | ↓ |
| 1383656_at | | ↑ |
| 1383736_at | Elavl2 | ↑ |
| 1387415_a_at | Stxbp5 | ↑ |
| 1387445_at | Phkg1 | ↑ |
| 1387492_at | Slco2a1 | ↑ |
| 1389240_at | | ↑ |
| 1389948_at | | ↑ |
| 1391144_at | | ↑ |
| 1391331_at | | ↑ |
| 1392688_at | Rassf1 | ↑ |
| 1392727_at | RGD1307365 | ↑ |
| 1393261_at | | ↑ |
| 1393526_at | | ↑ |
| 1393812_at | Plac9_predicted | ↑ |
| 1394401_at | Elovl6 | ↑ |
| 1394603_at | | ↓ |
| 1394641_at | | ↓ |
| 1395227_at | | ↓ |
| 1395415_at | | ↑ |
| 1397577_at | | ↑ |
| 1397911_at | | ↑ |
| 1398686_at | | ↑ |

**Table 28: hCTC Donor information**

| **Donor (lot)** | **Age** | **Gender** | **Cause of Death** | **Cardiac related diagnosis** | **Medical history** | **Others** |
|---|---|---|---|---|---|---|
| 1 (lot 1) | 49 | Female | ICH | None | Hypertension | Transplant-grade |
| 2 (lot 2) | 65 | Female | ICH | None | Hypertension | |
| 3 (lot 3) | 65 | Male | ICH | Dilated CardioMyopathy; Coronary Artery Disease | Hypertension | |

**Table 29: Mortality in multiple-lot and long-term efficacy study**

| **Group** | **Mortality** |
|---|---|
| Vehicle | 40% (4/10) |
| hCTC lot 1 | 20% (2/10) |
| hCTC lot 2 | 20% (2/10) |
| hCTC lot 3 | 40% (4/10) |
| hFibroblast | 40% (4/10) |
| pCTC | 10% (1/10) |

**Table 30: Cardiac function measured by echocardiography (absolute value)**

| **Function parameter** | | **Fractional shortening (FS, %)** | | **Left Ventricular End Systolic Dimention (LVESD, cm)** | | **Left ventricular End Diastolic Dimention (LVEDD, cm)** | | **Regional Wall Motion Score (RWMS)** | |
|---|---|---|---|---|---|---|---|---|---|
| | | **Mean** | **SD** | **Mean** | **SD** | **Mean** | **SD** | **Mean** | **SD** |
| **Vehicle** | **7 days** | 21.25 | 3.478 | 0.5872 | 0.02992 | 0.7458 | 0.02612 | 25.5 | 1.643 |
| | **28 days** | 18.58 | 3.265 | 0.6812 | 0.05998 | 0.8357 | 0.04769 | 26.17 | 1.602 |
| | **84 days** | 18.58 | 3.265 | 0.6833 | 0.06113 | 0.8572 | 0.05673 | 26.5 | 1.517 |
| **human Fibroblast** | **7 days** | 21.23 | 4.155 | 0.573 | 0.04188 | 0.7268 | 0.0365 | 21.38 | 0.9574 |
| | **28 days** | 26.85 | 1.848 | 0.6025 | 0.0298 | 0.824 | 0.02617 | 25.75 | 1.155 |
| | **84 days** | 25.38 | 3.453 | 0.6153 | 0.02916 | 0.8238 | 0.02848 | 23 | 1.708 |
| **hCTC lot 1** | **7 days** | 20.1 | 4.155 | 0.6341 | 0.1115 | 0.7896 | 0.09621 | 24.83 | 1.642 |
| | **28 days** | 28.54 | 4.371 | 0.5699 | 0.06522 | 0.7985 | 0.07923 | 25.88 | 1.246 |
| | **84 days** | 29.03 | 5.39 | 0.6001 | 0.07568 | 0.8435 | 0.0709 | 22.13 | 1.506 |
| **hCTC lot2** | **7 days** | 21.88 | 4.086 | 0.5804 | 0.05531 | 0.7425 | 0.06553 | 25.44 | 1.069 |
| | **28 days** | 26.19 | 1.485 | 0.575 | 0.0684 | 0.7784 | 0.06983 | 25.5 | 1.642 |
| | **84 days** | 31.36 | 4.205 | 0.5699 | 0.08822 | 0.8269 | 0.06365 | 23.13 | 2.204 |
| **HCTC lot3** | **7 days** | 22.15 | 6.682 | 0.5712 | 0.03337 | 0.7332 | 0.03658 | 21 | 1.751 |
| | **28 days** | 22.18 | 2.234 | 0.6235 | 0.05712 | 0.8005 | 0.05026 | 25.67 | 1.549 |
| | **84 days** | 21.75 | 3.291 | 0.6433 | 0.07528 | 0.8203 | 0.05551 | 25 | 1.835 |

## Claims

1. A purified population of human cardiac tissue-derived cells that do not express telomerase, and:
a. express CD90, CD105, CD117, GATA4 and Nkx2.5; and
b. do not express CD16, CD31, CD45 and myosin heavy chain; and preferably
c. are obtainable by the steps of:
i. dissociating heart tissue obtained from heart,
ii. digesting the heart tissue to release cells by treating the heart tissue with at least one enzyme, wherein the at least one enzyme comprises dispase,
iii. removing cardiomyocytes from the released cells, and
iv. culturing the remaining cells.

2. The purified population of human cardiac tissue-derived cells of claim 1, wherein the cardiac tissue-derived cells that do not express telomerase further express CD49e, CD59, and/or CD34.

3. The purified population of human cardiac tissue-derived cells of claim 1 or claim 2, wherein the cardiac tissue-derived cells that do not express telomerase further do not express MDR and/or Isl-1.

4. A method to produce cardiac tissue-derived cells that do not express telomerase, comprising the steps of:
i. dissociating heart tissue obtained from heart,
ii. digesting the heart tissue to release cells by treating the heart tissue with at least one enzyme, wherein the at least one enzyme comprises dispase,
iii. removing cardiomyocytes from the released cells, and
iv. culturing the remaining cells.

5. The method of claim 4, wherein the heart tissue is dissociated manually.

6. The method of claim 4, wherein the heart tissue is dissociated mechanically.

7. The method of claim 4, wherein the at least one enzyme further comprises collagenase.

8. The method of claim 4, wherein an entire heart is used as the source for the heart tissue.

9. A population of cardiac tissue-derived cells according to any one of claims 1 to 3, for use in treating or repairing damaged myocardium in a patient, wherein the treating or repairing comprises administering the population of cardiac tissue-derived cells to the patient in an amount sufficient to treat the damaged myocardium.

10. The population of cells for use according to claim 9, wherein the administration of the cardiac tissue-derived cells is via direct injection into the damaged myocardium.

11. The population of cells for use according to claim 9, wherein the administration of the cardiac tissue-derived cells is via direct injection into the area of the heart immediately surrounding the damaged myocardium.

12. The population of cells for use according to claim 9, wherein the cells are administered systemically, optionally using a catheter.

13. The population of cells for use according to claim 9, wherein the cells are administered along with another agent selected from the group consisting of: stem cell factor (SCF), granulocyte-colony stimulating factor (G-CSF), granulocyte-macrophage colony stimulating factor (GM-CSF), stromal cell-derived factor-1, steel factor, vascular endothelial growth factor, macrophage colony stimulating factor, granulocyte-macrophage stimulating factor, and Interleukin-3.

14. The population of cells for use according to claim 9, wherein the cells are further differentiated into cardiomyocytes prior to, or after, administration of the cells to a patient.

## Patentansprüche

1. Aufgereinigte Population aus menschlichem Herzgewebe stammender Zellen, die keine Telomerase exprimieren und:
a. CD90, CD105, CD117, GATA4 und Nkx2.5 exprimieren; und
b. kein CD16, CD31, CD45 und keine Myosinschwere-Kette exprimieren; und vorzugsweise
c. über die folgenden Schritte erhältlich sind:
i. Dissoziieren von aus Herz erhaltenem Herzgewebe,
ii. Verdauen des Herzgewebes zur Freisetzung von Zellen durch Behandeln des Herzgewebes mit wenigstens einem Enzym, wobei das wenigstens eine Enzym Dispase umfasst,
iii. Abtrennen von Kardiomyozyten von den freigesetzten Zellen und
iv. Kultivieren der verbliebenen Zellen.

2. Aufgereinigte Population aus menschlichem Herzgewebe stammender Zellen nach Anspruch 1, wobei die aus Herzgewebe stammenden Zellen, die keine Telomerase exprimieren, ferner CD49e, CD59 und/oder CD34 exprimieren.

3. Aufgereinigte Population aus menschlichem Herzgewebe stammender Zellen nach Anspruch 1 oder Anspruch 2, wobei die aus Herzgewebe stammenden Zellen, die keine Telomerase exprimieren, ferner kein MDR und/oder Isl-1 exprimieren.

4. Verfahren zur Herstellung aus Herzgewebe stammender Zellen, die keine Telomerase exprimieren, umfassend die Schritte:
i. Dissoziieren von aus Herz erhaltenem Herzgewebe,
ii. Verdauen des Herzgewebes zur Freisetzung von Zellen durch Behandeln des Herzgewebes mit wenigstens einem Enzym, wobei das wenigstens eine Enzym Dispase umfasst,
iii. Abtrennen von Kardiomyozyten von den freigesetzten Zellen und
iv. Kultivieren der verbliebenen Zellen.

5. Verfahren nach Anspruch 4, wobei das Herzgewebe von Hand dissoziiert wird.

6. Verfahren nach Anspruch 4, wobei das Herzgewebe mechanisch dissoziiert wird.

7. Verfahren nach Anspruch 4, wobei das wenigstens eine Enzym ferner Kollagenase umfasst.

8. Verfahren nach Anspruch 4, wobei ein ganzes Herz als Quelle für das Herzgewebe verwendet wird.

9. Population aus Herzgewebe stammender Zellen gemäß einem der Ansprüche 1 bis 3, zur Verwendung beim Behandeln oder Reparieren von geschädigtem Myokard bei einem Patienten, wobei das Behandeln bzw. Reparieren Verabreichen der Population aus Herzgewebe stammender Zellen an den Patienten in einer Menge, die zur Behandlung des geschädigten Myokards ausreicht, umfasst.

10. Population von Zellen zur Verwendung gemäß Anspruch 9, wobei die Verabreichung der aus Herzgewebe stammenden Zellen über direkte Injektion in das geschädigte Myokard erfolgt.

11. Population von Zellen zur Verwendung gemäß Anspruch 9, wobei die Verabreichung der aus Herzgewebe stammenden Zellen über direkte Injektion in den das geschädigte Myokard unmittelbar umgebenden Bereich des Herzens erfolgt.

12. Population von Zellen zur Verwendung gemäß Anspruch 9, wobei die Zellen systemisch, gegebenenfalls unter Verwendung eines Katheters, verabreicht werden.

13. Population von Zellen zur Verwendung gemäß Anspruch 9, wobei die Zellen zusammen mit einem anderen Agens, das aus der Gruppe bestehend aus SCF (Stern Cell Factor), G-CSF (Granulocyte-Colony Stimulating Factor), GM-CSF (Granulocyte-Macrophage Colony Stimulating Factor), aus Stromazellen stammendem Faktor 1, Steel Factor, VEGF (Vascular Endothelial Growth Factor, M-CSF (Macrophage Colony Stimulating Factor), Granulozyten-Monozyten-stimulierendem Faktor und Interleukin-3 ausgewählt ist, verabreicht werden.

14. Population von Zellen zur Verwendung gemäß Anspruch 9, wobei die Zellen weiter zu Kardiomyozyten vor oder nach Verabreichung der Zellen an einen Patienten differenziert werden.

## Revendications

1. Population purifiée de cellules humaines dérivées de tissu cardiaque qui n'expriment pas la télomérase, et :
a. expriment CD90, CD105, CD117, GATA4 et Nkx2.5 ; et
b. n'expriment pas CD16, CD31, CD45 et la chaîne lourde de myosine ; et de préférence
c. peuvent être obtenues par les étapes de :
i. dissociation de tissu cardiaque obtenu à partir d'un coeur,
ii. digestion du tissu cardiaque pour libérer les cellules par traitement du tissu cardiaque avec au moins une enzyme, dans laquelle l'au moins une enzyme comprend une dispase,
iii. retrait des cardiomyocytes à partir des cellules libérées, et
iv. culture des cellules restantes.

2. Population purifiée de cellules humaines dérivées de tissu cardiaque de la revendication 1, dans laquelle les cellules dérivées de tissu cardiaque qui n'expriment pas la télomérase expriment en outre CD49e, CD59, et/ou CD34.

3. Population purifiée de cellules humaines dérivées de tissu cardiaque de la revendication 1 ou la revendication 2, dans laquelle les cellules dérivées de tissu cardiaque qui n'expriment pas la télomérase, en outre, n'expriment pas MDR et/ou Isl-1.

4. Procédé de production de cellules dérivées de tissu cardiaque qui n'expriment pas la télomérase, comprenant les étapes de :
i. dissociation de tissu cardiaque obtenu à partir du coeur,
ii. digestion du tissu cardiaque pour libérer les cellules par traitement du tissu cardiaque avec au moins une enzyme, l'au moins une enzyme comprenant une dispase,
iii. retrait des cardiomyocytes à partir des cellules libérées, et
iv. culture des cellules restantes.

5. Procédé de la revendication 4, dans lequel le tissu cardiaque est dissocié manuellement.

6. Procédé de la revendication 4, dans lequel le tissu cardiaque est dissocié mécaniquement.

7. Procédé de la revendication 4, dans lequel l'au moins une enzyme comprend en outre une collagénase.

8. Procédé de la revendication 4, dans lequel un coeur entier est utilisé en tant que source de tissu cardiaque.

9. Population de cellules dérivées de tissu cardiaque selon l'une quelconque des revendications 1 à 3, pour utilisation dans le traitement ou la réparation de myocarde endommagé chez un patient, le traitement ou la réparation comprenant l'administration de la population de cellules dérivées de tissu cardiaque au patient dans une quantité suffisante pour traiter le myocarde endommagé.

10. Population de cellules pour utilisation selon la revendication 9, l'administration des cellules dérivées de tissu cardiaque étant effectuée par injection directe dans le myocarde endommagé.

11. Population de cellules pour utilisation selon la revendication 9, l'administration des cellules dérivées de tissu cardiaque étant effectuée par injection directe dans la région du coeur entourant immédiatement le myocarde endommagé.

12. Population de cellules pour utilisation selon la revendication 9, les cellules étant administrées de façon systémique, facultativement au moyen d'un cathéter.

13. Population de cellules pour utilisation selon la revendication 9, les cellules étant administrées avec un autre agent choisi dans le groupe constitué des : facteur de cellule souche (SCF), facteur stimulant les colonies de granulocytes (G-CSF), facteur stimulant les colonies de granulocytes-macrophages (GM-CSF), facteur dérivé de cellules stromales 1, facteur Steel, facteur de croissance endothéliale vasculaire, facteur stimulant les colonies de macrophages, facteur stimulant les granulocytes-macrophages et interleukine 3.

14. Population de cellules pour utilisation selon la revendication 9, les cellules étant en outre différenciées en cardiomyocytes avant, ou après, l'administration des cellules à un patient.
